(19) Europäisches Patentamt
European Patent Office
Office européen des brevets

(11) Numéro de publication : **0 375 560 B1**

# (12) FASCICULE DE BREVET EUROPEEN

(45) Date de publication du fascicule du brevet : **12.05.93 Bulletin 93/19**

(21) Numéro de dépôt : **89403622.7**

(22) Date de dépôt : **22.12.89**

(51) Int. Cl.$^5$ : **C07C 217/30,** C07D 285/10, C07D 209/08, C07C 217/32, C07C 217/34, C07C 255/54, C07C 233/25, A61K 31/40, A61K 31/16, A61K 31/135, A61K 31/275

## (54) Aryloxypropanolaminotétralines, un procédé pour leur préparation et composés pharmaceutiques les contenant.

(30) Priorité : **23.12.88 FR 8817139**

(43) Date de publication de la demande : **27.06.90 Bulletin 90/26**

(45) Mention de la délivrance du brevet : **12.05.93 Bulletin 93/19**

(84) Etats contractants désignés : **AT BE CH DE ES FR GB GR IT LI LU NL SE**

(56) Documents cités :
**EP-A- 0 045 911**
**WO-A-80/00152**
**DE-A- 2 558 285**
**US-A- 4 562 184**

(73) Titulaire : **ELF SANOFI**
**32-34, rue Marbeuf**
**F-75008 Paris (FR)**
(84) **BE CH DE ES FR GB GR LI LU NL SE AT**
Titulaire : **MIDY S.p.A.**
**Via Piranesi 38**
**I-20137 Milano (IT)**
(84) **IT**

(72) Inventeur : **Guzzi, Umberto**
**Via Don Gnocchi, 28**
**I-20010 Milano (IT)**
Inventeur : **Baroni, Marco**
**Via Carducci 11**
**I-20010 Vanzago Milano (IT)**
Inventeur : **Boveri, Sergio**
**Corso Repubblica 48**
**I-15057 Tortona Alessandria (IT)**
Inventeur : **Manara, Luciano**
**Via Novella, 2/2**
**I-15040 Pietra Marazzi Alessandria (IT)**
Inventeur : **Bianchetti, Alberto**
**Via Corridoni 11**
**I-20122 Milano (IT)**

(74) Mandataire : **Gillard, Marie-Louise et al**
**Cabinet Beau de Loménie 158, rue de l'Université**
**F-75340 Paris Cédex 07 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

La présente invention concerne des aryloxypropanolaminotétralines bêta-antagonistes, leurs sels, un procédé pour leur préparation et des compositions pharmaceutiques contenant lesdites aryloxypropanolaminotétralines ou leurs sels pharmaceutiquement acceptables en tant que principes actifs.

On sait que les récepteurs bêta sont ubiquitaires dans l'organisme et que leur blocage peut conditionner de nombreux organes et systèmes métaboliques.

Les produits bêta-antagonistes connus sont des inhibiteurs des cathécolamines sur les récepteurs bêta des tissus de l'appareil cardiovasculaire et sont utilisés comme médicaments dans le traitement des troubles cardiovasculaires, notamment en tant qu'anti-hypertenseurs ou anti-arythmiques.

Toutefois, les récepteurs bêta sont nombreux également dans la trachée et dans les bronches et, par conséquent, les bêta-antagonistes peuvent provoquer une constriction des voies aériennes susdites. Pour cette raison, les recherches antérieures ont été dirigées vers le développement de bêta-antagonistes sélectifs cardiaques sans effets secondaires respiratoires.

Les produits antagonistes des recepteurs bêta-adrénergiques ont été également proposés pour le traitement de maladies autres que cardiaques, par exemple la thyrotoxicose, l'hyperparatyroidisme, le glaucome, la migraine et l'anxiété. Dans ces cas, non seulement l'activité sur la trachée, mais aussi celle sur le coeur représente un effet secondaire.

Il est également connu que des recepteurs bêta-adrénergiques sont présents dans l'intestin et qu'aussi bien les bêta-agonistes que les bêta-antagonistes ont une activité sur la motricité intestinale.

Il est enfin connu (EP 211721 et EP 255415) que certaines phényléthanolaminotétralines ont une activité bêta-agoniste sélective intestinale qui n'affecte pas le coeur ou la trachée.

On a maintenant trouvé qu'en remplaçant le substituant alkyle ou aralkyle sur l'amine des aryloxypropanolamines bêta-bloquantes classiques par une tétraline, dans sa position 1- ou 2-, on obtient des produits qui, tout en conservant leur activité bêta-antagoniste, ont une activité cardiaque réduite.

On a également trouvé que les aryloxypropanolaminotétralines ainsi obtenues ont une activité bêta-antagoniste plus élevée sur le tractus gastro-intestinal et un effet réduit ou pas d'effet sur le tissu cardiaque ou de la trachée.

Ainsi, la présente invention concerne, selon un de ses aspects, des aryloxypropanolaminotétralines de formule:

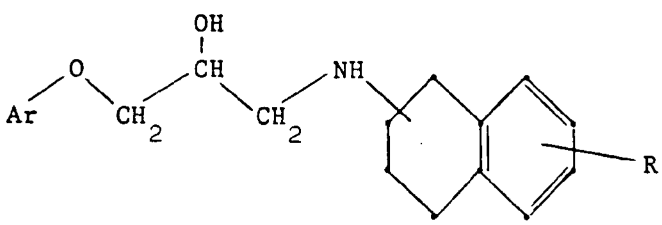

(i)

dans laquelle R représente l'hydrogène, un groupe hydroxy ou un groupe méthoxy et Ar représente un groupe aromatique ou hétéroaromatique éventuellement substitué, et leurs sels avec des acides minéraux ou organiques, pharmaceutiquement acceptables ou non.

De façon particulière, dans la formule (i), Ar est un groupe aromatique ou hétéroaromatique mono-,di- ou tri-cyclique, éventuellement substitué, dont un atome de carbone du carbocycle aromatique ou de l'hétérocycle aromatique est directement lié à l'oxygène. Plus particulièrement, dans la formule (i) Ar représente un groupe aromatique ou hétéroaromatique présent dans une des molécules d'aryloxypropanolamines ayant une activité bêta-antagoniste. Notamment, Ar est le résidu éthérifiant en position 3 d'un 1-amino-2,3-propandiol N-substitué, ledit propandiol N-substitué ayant une activité bêta-bloquante adrénergique.

Les groupes aromatiques mono-,di- et tri-cycliques comprennent, entre autres, les radicaux phényle, naphtyle, 5,6-, 5,8- et 7,8-dihydronaphtyle, 5,6,7,8-tétrahydronaphtyle, 5,8-éthano-5,6,7,8-tétrahydronaphtyle, 5,8-méthano-5,6,7,8-tétrahydronaphtyle, 9,10-dihydro-9,10-éthano (ou éthéno) anthryle, et fluorényle, lesdits radicaux pouvant être substitués.

Les groupes hétéroaromatiques mono-,di- et tri-cycliques comprennent soit le radical phényle condensé sur un noyau hétérocyclique à 5 ou 6 membres contenant 1, 2 ou 3 hétéroatomes choisis parmi les atomes d'oxygène, d'azote et de soufre, à son tour éventuellement condensé sur un autre noyau benzène, soit le radical phényle condensé à la fois sur un noyau furanne et sur un noyau pyranne ou condensé sur le pyrrole d'un tétrahydropyrido(3,4-c)pyrrole; soit des radicaux hétérocycliques à 5 ou 6 membres contenant 1, 2, 3 ou 4 hétéroatomes choisis parmi les atomes d'oxygène, d'azote et de soufre, éventuellement condensés sur un noyau phényle, lesdits radicaux et noyau condensés pouvant être substitués.

Plus particulièrement, le radical Ar peut être un groupe phényle, qui peut être non substitué ou éventuellement contenir un ou plusieurs susbtituants. Comme substituants on peut citer: halogène, cyano, hydroxy, amino, formyle, nitro, carboxyle, carbamoyle, trifluorométhyle, alkyle, alkényle, alkynyle, cycloalkyle, bicycloalkyle, alcanoyle, alcoxy, hydroxyalkyle, alcoxyalkyle, alcoxyalcoxyalkyle, alcoxyalcoxy, alkényloxy, alkényloxyalkyle, alkynyloxy, alkynyloxyalkyle, cycloalcoxy, alkylthio, alkylthioalkyle, morpholino, acylamino, acylaminoalkyle, acyloxy, alcoxycarbonyle, cycloalkyloxycarbonyle, aminocarbonylamino, aminocarbonylaminoalkyle, dialkylaminocarbonylalkyle, dialkylaminocarbonylalcoxy, dans les 2 derniers groupes, les groupes alkyle terminaux pouvant former, avec l'atome d'azote aussi un groupe cyclique contenant 4 ou 5 atomes de carbone, cycloalkylaminocarbonylamino, alkylaminocarbonylaminoalkyle, cycloalkylaminocarbonylaminoalkyle, alcoxycarbonylaminoalkyle, cycloalcoxycarbonylaminoalkyle, carbamoylalkyle, alkylaminocarbonylalkyle, cycloalkylaminocarbonylalkyle ou un groupe alkylaminocarbonylalcoxy.

Le radical Ar peut être aussi un groupe thiadiazolyle, naphtyle, indényle, indolyle, indazolyle, imidazolyle, benzimidazolyle, benzotriazolyle, benzofurannyle, benzodioxolyle, benzothényle, benzothiazolyle, benzisothiazolyle, quinolyle, isoquinolyle, benzodiazinyle, benzothiinnyle, benzothiazinyle, benzothiadiazinyle, benzoxathiinnyle, benzoxazolyle, benzisoxazolyle, benzoxazinyle, benzodioxinyle ou carbazolyle dont un ou plusieurs doubles liaisons peuvent être hydrogénées, lesdits groupes pouvant être non substitués ou contenir éventuellement un ou plusieurs substituants tels que: le groupe alkyle, cyano, hydroxyalkyle, hydroxy, oxo, formyle, alcanoyle, alkylcarbonylamino et alcoxycarbonyle ainsi que le groupe morpholino.

Le groupe phényle peut être de préference mono- ou disubstitué, notamment en position 2,5, mais aussi par exemple en position 2,3, 2,4, 3,4 ou 3,5; il peut cependant être trisubstitué, notamment en position 3,4,5, mais aussi par exemple en 2,3,4, 2,3,5 ou 2,4,5; tétrasubstitué, par exemple en 2,3,4,5; ou pentasubstitué. Les substituants dans le groupe phényle peuvent être: F; Cl; Br; I; CN; OH; $NH_2$; $NH-CO-NH_2$; $NO_2$; COOH; $CONH_2$; $CF_3$; alkyle en C1-C10, de préférence en $C_1$-$C_4$, méthyle ou éthyle étant préférés, ainsi que par exemple n-propyle, isopropyle, n-butyle, isobutyle, sec-butyle, tert.butyle, pentyle ou n-pentyle, hexyle ou n-hexyle, heptyle ou n-heptyle, octyle ou n-octyle, nonyle ou n-nonyle ainsi que décyle ou n-décyle; alkényle contenant 2 à 10, de préférence 2-4 atomes de carbone, par exemple vinyle, allyle, 1-propényle, isopropényle, butényle ou 1-butén-1-, -2-, -3- ou -4-yle, 2-butén-1-yle, 2-butén-2-yle, pentényle, hexényle ou décényle; alkynyle contenant 2 à 10, de préférence 2-4 atomes de carbone, par exemple éthynyle, 1-propin-1-yle, propargyle, butynyle ou 2-butyn-1-yle, pentinyle, décinyle; cycloalkyle contenant 3 à 8, de préférence 5 ou 6 atomes de carbone, cyclopentyle ou cyclohexyle étant préférés, ainsi que par exemple cyclopropyle, cyclobutyle, 1-, 2- ou 3-méthylcyclopentyle, 1-, 2-, 3- ou 4-méthylcyclohexyle, cycloheptyle ou cyclooctyle; bicycloalkyle contenant 4 à 11, de préférence 7 atomes de carbone, exo ou endo 2-norbornyle étant préférés, ainsi que par exemple 2-isobornyle ou 5-camphyle; hydroxyalkyle contenant 1 à 5, de préférence 1-2 atomes de carbone, hydroxyméthyle et 1- ou 2-hydroxyéthyle étant préférés, ainsi que par exemple 1-hydroxyprop-1-yle, 2-hydroxyprop-1-yle, 3-hydroxyprop-1-yle, 1-hydroxyprop-2-yle, 1-hydroxybut-1-yle, 1-hydroxypent-1-yle; alcanoyle contenant 1 à 7, de préférence 1-4 atomes de carbone, formyle, acétyle ou propionyle étant préférés, ainsi que par exemple butyryle, isobutyryle, valéroyle, caproyle, heptanoyle; alcoxy contenant 1 à 10, de préférence 1-4 atomes de carbone, méthoxy ou éthoxy étant préférés, ainsi que par exemple n-propoxy, isopropoxy, n-butoxy, isobutoxy, sec-butoxy, tert-butoxy, pentyloxy, hexyloxy, heptyloxy, octyloxy, nonyloxy ou décyloxy; alcoxyalkyle contenant 2 à 10, de préférence de 2 à 6 atomes de carbone, par exemple alcoxyméthyle ou méthoxyméthyle, alcoxyéthyle ou 1- ou 2-méthoxyéthyle, 1- ou 2-n-butoxyéthyle, 1- ou 2-n-octyloxyéthyle; alcoxyalcoxyalkyle contenant jusqu'à 10, de préférence de 4 à 7 atomes de carbone, par exemple alcoxyalcoxyméthyle, par exemple 2-méthoxyéthoxyméthyle, 2-éthoxyéthoxyméthyle ou 2-isopropoxyéthoxyméthyle, alcoxyalcoxyéthyle par exemple 2-(2-méthoxy- éthoxy)éthyle ou 2-(2-éthoxyéthoxy)éthyle; alcoxyalcoxy contenant de 2 à 10, de préférence de 3 à 6 atomes de carbone, par exemple 2-méthoxyéthoxy, 2-éthoxyéthoxy ou 2-n-butoxyéthoxy; alkényloxy contenant 2 à 10, de préférence 2 à 4 atomes de carbone, allyloxy étant préféré, ainsi que par exemple vinyloxy, propényloxy, isopropényloxy, buténoyloxy ou 1-butén-1-, -2-, -3- ou -4-yloxy, 2-butén-1-yloxy, 2-butén-2-yloxy, penténoyloxy, hexényloxy ou décényloxy; alkényloxyalkyle avec jusqu'à 10, de préférence 3-6 atomes de carbone, par exemple allyloxyméthyle; alkynyloxy contenant de 2 à 10, de préférence 2 à 4 atomes de carbone, propargyloxy étant préféré, ainsi que par exemple éthynyloxy, 1-propyn-1-yloxy, butynyloxy ou 2-butyn-1-yloxy, pentynyloxy ou décynyloxy; alkynyloxyalkyle contenant de 3 à 10, de préférence 3 à 6 atomes de carbone, par exemple éthynyloxyméthyle, propargyloxyméthyle ou 2-(2-butyn-1-yloxy)éthyle; cycloalcoxy contenant 3 à 8, de préférence 5 ou 6 atomes de carbone, cyclopentyloxy ou cyclohexyloxy étant préférés, ainsi que par exemple cyclopropyloxy, cyclobutyloxy, 1-, 2- ou 3-méthylcyclopentyloxy, 1-, 2-, 3- ou 4-méthylcyclohexyloxy, cycloheptyloxy ou cyclooctyloxy; alkylthio contenant de 1 à 10, de préférence 1 à 4 atomes de carbone, méthylthio ou éthylthio étant préférés, ainsi que par exemple n-propylthio, isopropylthio,

n-butylthio, isobutylthio, sec-butylthio, tert-butylthio, pentylthio, hexylthio, octylthio, nonylthio ou décylthio; alkylthioalkyl contenant de 2 à 10, de préférence 2 à 6 atomes de carbone, par exemple méthylthiométhyl, 2-méthylthioéthyl ou 2-n-butylthioéthyl; acylamino, à savoir alcanoylamino contenant de 1 à 7, de préférence 1 à 4 atomes de carbone, formylamino et acétylamino étant préférés, ainsi que propionylamino, butyrylamino, isobutyrylamino, valéroylamino, caproylamino, heptanoylamino, ainsi que aroylamino ou benzoylamino; acylaminoalkyle, de préférence alcanoylaminoalkyle contenant de 2 à 8, de préférence 3 à 6 atomes de carbone, tel que formylaminoéthyle, acétylaminoéthyle, propionylaminoéthyle, n-butyrylaminoéthyle, formylaminopropyle, acétylaminopropyle, propionylaminopropyle, formylaminobutyle acétylaminobutyle, ainsi que propionylaminobutyle, butyrylaminobutyle; acyloxy contenant de 1 à 6, de préférence 2 à 4 atomes de carbone, acétyloxy, propionyloxy ou butyryloxy étant préférés, ainsi que par exemple formyloxy, valéroyloxy, caproyloxy; alcoxycarbonyle contenant de 2 à 5, de préférence 2 et 3 atomes de carbone, méthoxycarbonyle et éthoxycarbonyle étant préférés, ainsi que par exemple n-propoxycarbonyle, isopropoxycarbonyle, n-butoxycarbonyle, isobutoxycarbonyle, sec-butoxycarbonyle ou tert-butoxycarbonyle; cycloalcoxycarbonyle contenant de 4 à 8, de préférence 6 ou 7 atomes de carbone, cyclopentyl- oxycarbonyle, cyclohexyloxycarbonyle étant préférés, ainsi que cyclopropyloxycarbonyle, cyclobutyloxycarbonyle ou cycloheptyloxycarbonyle; alkylaminocarbonylamino contenant de 2 à 4 atomes de carbone, tel que méthylaminocarbonylamino, éthylaminocarbonylamino, propylaminocarbonylamino; dialkylaminocarbonylamino contenant de 3 à 7, de préférence 3 à 5 atomes de carbone, de préférence diméthylaminocarbonylamino, ainsi que di-n-propylaminocarbonylamino, diisopropylaminocarbonylamino; (1-pyrrolidino)-carbonylamino; (1-pipéridino)carbonylamino; cycloalkylaminocarbonylamino contenant de 4 à 8, de préférence 6 ou 7 atomes de carbone, cyclopentylaminocarbonylamino, cyclohexylaminocarbonylamino étant préférés, ainsi que cyclopropylaminocarbonylamino, cyclobutylaminocarbonylamino, cylcoheptylaminocarbonylamino; alkylaminocarbonylaminoalkyle contenant de 3 à 9, de préférence 4 à 7 atomes de carbone, méthylaminocarbonylaminoéthyle, éthylaminocarbonylaminoéthyle, éthylaminocarbonylaminopropyle, éthylaminocarbonylaminobutyle étant préférés, ainsi que par exemple méthylaminocarbonylaminométhyle, n-propylaminocarbonylaminobutyle, n-butylaminocarbonylaminobutyle; dialkylaminocarbonylaminoalkyle contenant de 4 à 11 atomes de carbone, par exemple diméthylaminocarbonylaminométhyle, diéthylaminocarbonylaminoéthyle, diéthylaminocarbonylaminopropyle, diéthylaminocarbonylaminobutyle; (1-pirrolidino)carbonylaminoéthyle; (1-pipéridino)carbonylaminoéthyle; cycloalkylaminocarbonylaminoalkyle contenant de 5 à 12, de préférence 8 à 11 atomes de carbone, cyclopentylaminocarbonylaminoéthyle, cyclopentylaminocarbonylaminopropyle, cyclopentylaminocarbonylaminobutyle, cyclohexylaminocarbonylaminoéthyle, cyclohexylaminocarbonylaminopropyle et cyclohexylaminocarbonylamino butyle étant préférés, ainsi que par exemple cyclopropylaminocarbonylaminométhyle, cycloheptylaminocarbonylaminoéthyle; alcoxycarbonylaminoalkyle contenant de 3 à 12, de préférence 4 à 9 atomes de carbone, méthoxycarbonylaminoéthyle, éthoxycarbonylaminoéthyle, n-propoxycarbonylaminoéthyle, isopropoxycarbonylaminoéthyle, n-butoxycarbonylaminoéthyle, isobutoxycarbonylaminoéthyle, sec-butoxycarbonylaminoéthyle, tert-butoxycarbonylaminoéthyle, éthoxycarbonylamino- propyle, n-butoxycarbonylaminopropyle, éthoxycarbonylaminobutyle, n-butoxycarbonylaminobutyle étant préférés, ainsi que par exemple n-propoxycarbonylaminopropyle, n-propoxycarbonylaminobutyle, isopro- poxycarbonylaminobutyle; cycloalcoxycarbonylaminoalkyle contenant de 5 à 12, de préférence 8 à 11 atomes de carbone, cyclopentyloxy- carbonylaminoéthyle, cyclopentyloxycarbonylaminopropyle, cyclopentyloxycarbonylaminobutyle, cyclohexyloxycarbonylaminoéthyle, cyclohexyloxycarbonylaminopropyle, cyclohexyloxycarbonylaminobutyle étant préférés, ainsi que par exemple cyclopropyloxycarbonylaminométhyle, cycloheptyloxycarbonylaminoéthyle; carbamoylalkyle contenant de 2 à 5, de préférence 2 atomes de carbone, de préférence carbamoylméthyle, ainsi que carbamoyléthyle, carbamoylpropyle, carbamoylbutyle; alkylaminocarbonylalkyle contenant de 3 à 9, de préférence 3 à 6 atomes de carbone, méthylaminocarbonyléthyle, éthylaminocarbonylméthyle, n-propylaminocarbonylméthyle, isopropylaminocarbonylméthyle, n-butylaminocarbonylméthyle, isobutylaminocarbonylméthyle, sec-butylaminoque par exemple éthylaminocarbonyléthyle, éthylaminocarbonylpropyle, éthylaminocarbonylbutyle, n-propylaminocarbonylbutyle, n-butylaminocarbonylbutyle; dialkylaminocarbonylalkyle contenant de 4 à 11, de préférence 4 à 8 atomes de carbone, diméthylaminocarbonylméthyle, diéthylaminocarbonylméthyle, di-n-propylaminocarbonylméthyle, (1-pirrolidino)carbonylméthyle, (1-pipéridino)carbonylméthyle étant préférés, ainsi que par exemple diéthylaminocarbonyléthyle, (1-pipéridino)carbonyléthyle, diéthylaminocarbonylpropyle, diéthylaminocarbonylbutyle; cycloalkylaminocarbonylalkyle contenant de 5 à 12, de préférence 7 ou 8 atomes de carbone, cyclopentylaminocarbonylméthyle et cyclohexylaminocarbonylméthyle étant préférés, ainsi que par exemple cyclopropylaminocarbonylméthyle, cyclobutylaminocarbonylméthyle, cycloheptylaminocarbonylméthyle, cyclohexylaminocarbonyléthyle, cyclohexylaminocarbonylpropyle, cyclohexylaminocarbonylbutyle; alkylaminocarbonylalcoxy contenant de 3 à 10, de préférence 3 à 5 atomes de carbone, méthylaminocarbonylméthoxy étant préféré, ainsi que par exemple méthylaminocarbonyléthoxy, méthylaminocarbonylpropoxy; dialkylaminocarbonylalkoxy contenant de 4 à 10, de préférence 4 à 7 atomes de carbone, tel que diméthylaminocarbonylméthoxy, diéthylaminocarbonyléthoxy, (1-pipéridino)carbonylméthoxy; cycloalkylaminocarbonylalcoxy contenant de 5 à 11, de préférence 7 ou 8 atomes de carbone, tel que cyclopentylaminocarbonylméthoxy, cyclohexylaminocarbonylméthoxy.

Le radical Ar peut également représenter, par exemple: 1,2,5-thiadiazol-3-yle, 4-(N-morpholino)-1,2,5-thiadiazol-3-yle; 1-ou 2-naphtyle; 1-, 2-, 3-, (de préférence) 4-, 5-, 6- ou 7-indanyle; 1-oxo-4-, -5-, -6-, ou (de préférence) -7-indanyle; alkyl-1-oxo-indanyle, de préférence 1-oxo-5-méthyl-7-indanyle; 1-hydroxy-4-, -5-, -6- ou (de préférence) -7-indanyle; 1-, 2-, 3-, (de préférence) 4-, 5-, 6- ou 7-indényle; 1-, 2-, 3-, 4-, (de préférence) 5-, 6-, 7- ou 8-tétralyle; oxo-tétralyle, de préférence 1-oxo-5-tétralyle, ainsi que 2-, 3- ou 4-oxo-5-tétralyle ou 1-, 2-, 3- ou 4-oxo-6-tétralyle; hydroxytétralyle, (de préférence) 1-hydroxy-5-tétralyle, ainsi que 2-, 3- ou 4-hydroxy-5-tétralyle; (de préférence) 4-, 5-, 6- ou 7-indolyle; alkylindolyle, de préférence méthylindolyle, par exemple 2-méthyl-4-indolyle, 3-méthyl-4-indolyle ou 6-méthyl-4-indolyle, ainsi que par exemple 2-éthyl-4-indolyle ou 6-éthyl-4-indolyle; dialkylindolyle, de préférence diméthylindolyle, par exemple 2,3-diméthyl-4-indolyle, 2,6-diméthyl-4-indolyle, ainsi que par exemple 2-méthyl-3-éthyl-4-indolyle, 2-éthyl-3-méthyl-4-indolyle, 2,3-diéthyl-4-indolyle; cyanoindolyle, par exemple 2-cyano-4-indolyle, 3-cyano-4-indolyle; alkyl-cyano-indolyle, de préférence 2-cyano-6-méthyl-4-indolyle, ainsi que par exemple 3-cyano-6-méthyl-4-indolyle; carbamoylindolyle, de préférence 2-carbamoyl-4-indolyle, 3-carbamoyl-4-indolyle, ansi que par exemple 6-carbamoyl-4-indolyle, alkyl-carbamoylindolyle, de préférence méthyl-carbamoylindolyle, par exemple 2-carbamoyl-6-méthyl-4-indolyle; hydroxyalkylindolyle, de préférence 2-hydroxyméthyl-4-indolyle, ainsi que par exemple 2-hydroxyméthyl-5- indolyle, 3-hydroxyméthyl-4-indolyle, 2-(2-hydroxyéthyl)-4-indolyle; 2-oxo-indolinyle, de préférence 2-oxo-indolin-4-yle, ainsi que 2-oxo- indolin-5-yle; alkyl-2-oxo-indolinyle, de préférence méthyl-2-oxo- indolin-4-yle, par exemple 3-méthyl-2-oxo-indolin-4-yle, ainsi que par exemple 3-éthyl-2-oxo-indolin-4-yle, 3-isopropyl-2-oxo-indolin- 4-yle; dialkyl-2-oxo-indolinyle, par exemple 3,3-diméthyl-2-oxo-indolin-4-yle, 3,3,-diéthyl-2-oxo-indolin-4-yle; indazol- (de préférence) -4-, -5-, -6- ou -7-yle; benzimidazol-4-yle; alkylbenzimidazol-4-yle, de préférence méthyl-benzimidazol-4-yle, par exemple 3-méthyl-benzimidazol-4-yle, 1-méthylbenzimidazol-4-yle, 2-méthyl-benzimidazol-4-yle, 6-méthyl-benzimidazol-4-yle, 7-méthyl-benzimidazol-4-yle; benzimidazolin-2-on-4-yle (de préférence), benzimidazolin-2-on-5-yle; alkylbenzimidazolin-2-on-4-yle, de préférence méthyl-benzimidazolin-2-on-4-yle, par exemple 6-méthyl-benzimidazolin-2-on-4-yle, 7-méthylbenzimidazolin-2-on-4-yle; benzotriazol- (de préférence) 4- ou 5-yle; benzofuran- (de préférence) 4-, 5-, 6- ou 7-yle; alkylbenzofuran-4-yle, par exemple 2-méthylbenzofuran-4-yle, 3-méthylbenzofuran-4-yle, 6-méthylbenzofuran-4-yle; alcanoylbenzofuran-4-yle; par exemple 2-acétylbenzofuran-4-yle, 6-acétylbenzofuran-4-yle; bis-alcanoylbenzofuranyle, par exemple 2,4-diacétylbenzofuran-5-yle, 2,6-diacétylbenzofuran-4-yle; 1,3-benzodioxolyle, de préférence 1,3-benzodioxol-4-yle; alkyl-1,3-benzodioxolyle, de préférence 2-méthyl-1,3-benzodioxol-4-yle, ainsi que par exemple 6-méthyl-1,3-benzodioxol-4-yle; dialkyl-1,3-benzodioxolyle, de préférence 2,2-diméthyl-1,3-benzodioxol-4-yle, ainsi que par exemple 2,2-diéthyl-1,3-benzodioxol-4-yle, 2,6-diméthyl-1,3-benzodioxol-4-yle; 1,2-benzisoxazol- (de préférence) 4-, 5-, 6- ou 7-yle; alkyl-1,2-benzisoxazolyle, de préférence 3-méthyl-1,2-benzisoxazol-4-yle, ainsi que par exemple 3-éthyl-1,2-benzisoxazol-4-yle, 3-propyl-1,2-benzisoxazol-4-yle, 3-isopropyl-1,2-benzisoxazol-4-yle, 6-méthyl-1,2-benzisoxazol-4-yle; 1,3-benzoxazol- (de préférence) 4-, 5-, 6- ou 7-yle; alkyl-1,3-benzoxazolyle, de préférence 2-méthyl-1,3-benzoxazol-4-yle, ainsi que par exemple 2-éthyl-1,3-benzoxazol-4-yle, 6-méthyl-1,3-benzoxazol-4-yle, 6- éthyl-1,3-benzoxazol-4-yle; aryl-1,3-benzoxazolyle, de préférence 2-phényl-1,3-benzoxazol-4-yle, 2-(4-pyridyl)-1,3-benzoxazol-4-yle; benzothien- (de préférence) 4-, 5-, 6- ou 7-yle; 1,2-benzisothiazol- (de préférence) 4-, 5-, 6- ou 7-yle; alkyl-1,2-benzisothiazolyle, par exemple 6-méthyl-1,2-benzisothiazol-4-yle; 1,3-benzothiazol-4-, -5-, -6- ou (de préférence) -7-yle; alkyl-1,3-benzothiazol-7-yle, par exemple 2-méthyl-1,3-benzothiazol-7-yle; 4-méthyl-1,3-benzothiazol-7-yle, 2-éthyl-1,3-benzothiazol-7-yle; 2-aryl-1,3-benzothiazol-7-yle, par exemple 2-phényl-1,3-benzothiazol-7-yle; 2-(4-chlorophenyl)-1,3-benzothiazol-7-yle; 2(4-pyridyl)-1,2-benzothiazol-7-yle; 1,2-dihydro-2-oxo-3-, -4-, (de préférence) -5-, -6-, -7- ou 8-quinolinyle; 1,2,3,4-tétrahydro- (de préférence) -5-, -6-, -7- ou -8-quinolinyle; 1,2,3,4-tétrahydro-2-oxo (de préférence) -5-, -6-, -7- ou -8-quinolinyle; 1,2 -dihydro-8-hydroxy-2-oxo- (de préférence) -5-, -6- ou -7-quinolinyle 1,2-dihydro-8-alcoxy-2-oxo- (de préférence) -5-, -6- ou -7-quinolinyle, par exemple 1,2-dihydro-8-méthoxy-2-oxo-5-quinolinyle; 1,2,3,4-tétrahydro-8-hydroxy-2-oxo- (de préférence) -5-, -6-, ou -7-quinolinyle; 1,2,3,4-tétrahydro-8-alcoxy-2-oxo- (de préférence) -5-, -6- ou -7-quinolinyle, par exemple 1,2,3,4-tétrahydro-8-méthoxy-2-oxo-5-quinolinyle; 1,2,3,4-tétrahydro-8-alcanoylamino-2-oxo- (de préférence) -5-, -6- ou -7-quinolinyle, par exemple 1,2,3,4-tétrahydro-8-acétylamino-2-oxo-5-quinolinyle; 1,2-dihydro-3-cyano-2-oxo- (de préférence) -5-, -6-, -7- ou -8-quinolinyle; 1,2-dihydro-3-cyano-2-oxo-7-méthyl-5-quinolinyle; 1,2-dihydro-1-oxo- (de préférence) -4-, -5-, -6- -7 ou -8-isoquinolinyle, 1,2-dihydro-2-alkyl-1-oxo- (de préférence) -4-, -5-, -6-, -7- ou -8-isoquinolinyle, par exemple 1,2-dihydro-2-méthyl-1-oxo-4-isoquinolinyle; 1,2,3,4-tétrahydro-2-alcanoyl- (de préférence) -5-, -6-, -7- ou -8-isoquinolinyle, de préférence 1,2,3,4-tétrahydro-2-formyl-5-isoquinolinyle, ainsi que par exemple 1,2,3,4-tétrahydro-2-acétyl-5-isoquinolinyle; 1,2-dihydro-2-oxo-1,3-benzodiazin- (de préférence) -5-, -6-, -7-

ou -8-yle; 2H-3,4-dihydro-5-, -6-, -7- ou (de préférence) -8-benzopyranyle; 2H-5-, -6-, -7- ou (de préférence) -8-benzopyranyle; 2H-2-oxo-5-alkyl-7- ou (de préférence) -8-benzopyranyle, par exemple 2H-2-oxo-5-méthyl-8-benzopyranyle; 2H-3-cyano-5-, -6-, -7- ou (de préférence) 8-benzopyranyle; 2H-3,4-dihydro-5-, -6-, -7- ou (de préférence) -8-benzothiinyle; 3,4-dihydro-1H-2,2-dioxo-2,1-benzothiazin- (de préférence) 5-, 6-, 7- ou 8-yle; 3,4-dihydro-1H-1-alkyl-2,2-dioxo-2,1-benzothiazin- (de préférence) 5-, 6-, 7- ou 8-yle, par exemple 3,4-dihydro- 1H-1-méthyl-2,2-dioxo-2,1-benzothiazin-5-yle; 3,4-dihydro-2H-3-oxo-1,4-benzothiazin-5-, -6-, -7- ou (de préférence) -8-yle; 5- ou 6-alkyl-3,4-dihydro-3-oxo-1,4-benzothiazin-8-yle, par exemple 6-méthyl-3,4-dihydro-3-oxo-1,4-benzothiazin-8-yle; 1,1-dioxo-1,2,4-benzothiadiazin-5-, -6-, -7- ou -8-yle; 1,1-dioxo-3-alkyl-1,2,4-benzothiadiazin-5-, -6-, -7- ou -8-yle, par exemple 1,1-dioxo-3-méthyl-1,2,4-benzothiadiazin-5-, -6-, -7- ou -8-yle; 1,1-dioxo-3-alcanoyl-1,2,4-benzothiadiazin-5-, -6-, -7- ou -8-yle, par exemple 1,1-dioxo-3-formyl-1,2,4-benzothiadiazin-5-, -6-, -7- ou -8-yle; 1,1-dioxo-3-acétyl-1,2,4-benzothiadiazin-5-, -6-, -7-ou -8-yle; 1,1-dioxo-3-aroyl-1,2,4-benzothiadiazin-5-, -6-, -7- ou -8-yle, par exemple 1,1-dioxo-3-benzoyl-1,2,4-benzothiadiazin-5-, -6-, -7- ou -8-yle; 1,1-dioxo-3-(4-pyridyl-carbonyl) -1,2,4-benzothiadiazin-5-, -6-, -7- ou -8-yle; 3,4-dihydro-2,2-dioxo-1,2-benzoxathiin- (de préférence) -5-, -6-, -7- ou (de préférence) -8-yle; 1-, 2-, 3- ou (de préférence) 4-carbazolyle.

Notamment, lorsque dans la formule (i) ci-dessus Ar est un groupe aromatique ou hétéroaromatique tel que défini dans le paragraphe précédent, ledit groupe aromatique ou hétéroaromatique peut être représenté par un radical ayant une des structures 1 à 67 ci-dessous.

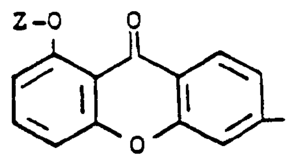

1

(Z = H ou alkylaminoalkyle)

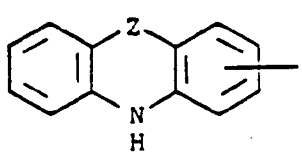

2

(Z = une liaison directe ou un atome de soufre)

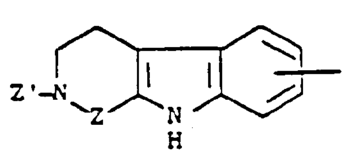

3

(Z = CO ou $CH_2$; Z' = H, $CH_3$)

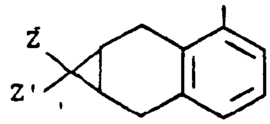

4

(Z et Z' = H où halogène ou Z = H, Z' = alkyle)

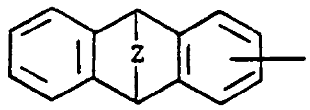

5

(Z = $CH_2CH_2$, CH=CH)

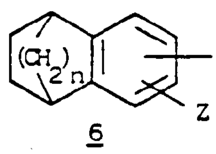

6

(Z = H, halogène, alkylsulfonylamino)
n = 1 ou 2

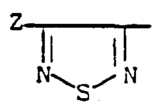

7

(Z = Cl, alkyle, alkoxy, phényle, benzyle, morpholino, pipéridino)

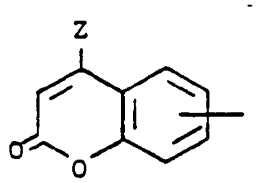

8

(Z = H, alkyle)

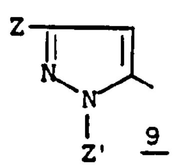

9

(Z = H, alkyle, alcoxy, phényle, benzyle)
(Z' = cycloalkyle, alkyle, aralkyle, aralkyle éventuellement substitué, phényle éventuellement substitué)

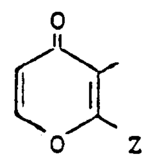

10

(Z = alkyle, aralkyle)

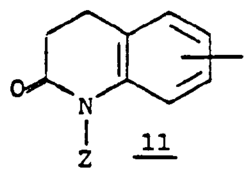

11

(Z = H, alkyle, aralkyle, alkényle)

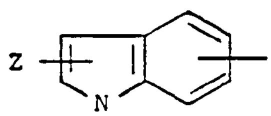

12

(Z = H, $CH_3$)

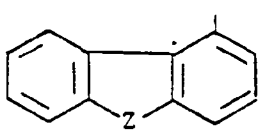

13

(Z = CH(OH), CO)

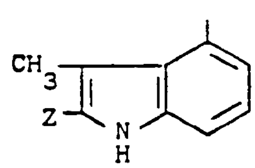

14

(Z = $CH_3$, $CH_2OH$, $CH_2OCH_3$, COOH, COOAlkyle)

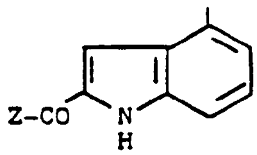

15

(Z = hydroxy, alcoxy, amino, méthylamino, pyrrolidino, pipéridino, morpholino)

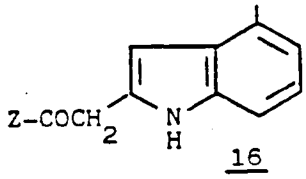

16

(Z = amino, alcoxy, alkylamino, dialkylamino, pyrrolidino, pipéridino, morpholino)

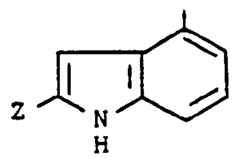

17

(Z = éthyle ou propyle, hydroxyméthyle)

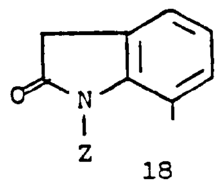

18

(Z = H, alkyle)

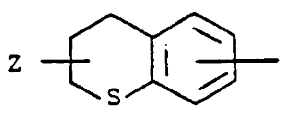

19

(Z = H, alkyle)

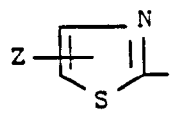

20

(Z = hydrogène, alkyle, hydroxyalkyle, halogène, $CF_3$, alcanoyle, cyano)

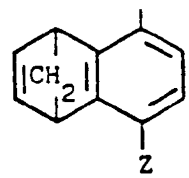

21

(Z = H, OH, alkyle, alcoxy, halogène, CN, NH-CHO, NH-CO-alkyle, alkényloxy, alcanoyloxy)

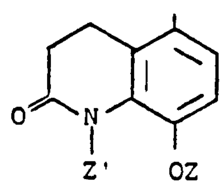

22

(Z = hydrogène, alkyle, alcanolalkyle, carboxyalkyle, alcoxycarbonylalkyle)
(Z' = hydrogène, alkyle, phénylalkyle)

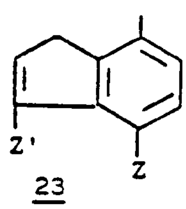

23

(Z = H, halogène)
(Z' = alkyle)

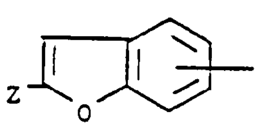

24

(Z = alcanoyle, alcoxycarbonyle, benzoyle)

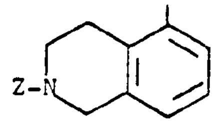

25

(Z = alcanoyle)

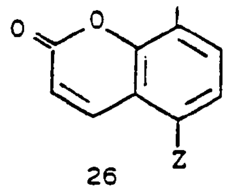

26

(Z = alkyle)

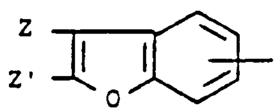

27

(un des Z et Z' est l'hydrogène et l'autre est l'hydrogène ou un groupe méthyle)

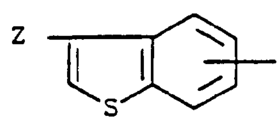

28

(Z = hydrogène ou méthyle)

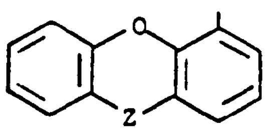

29

(Z = $CH_2$, CO)

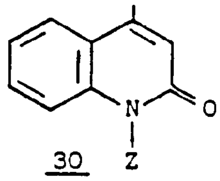

30

(Z = méthyle, éthyle, allyle, phényle)

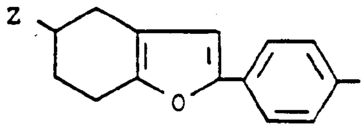

31

(Z = H ou alkyle)

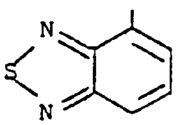

32

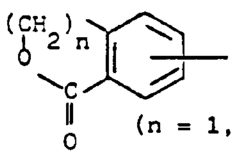

(n = 1,2)

33

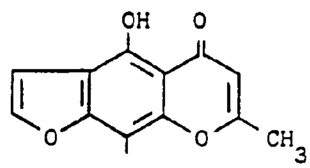

34

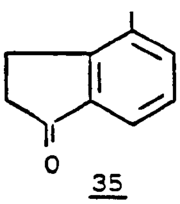

35

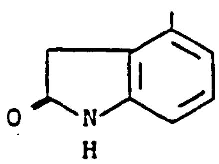

36

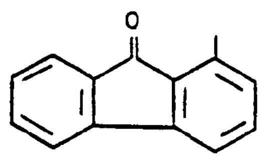

37

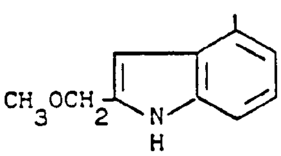

38

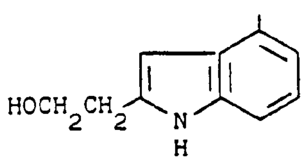

39

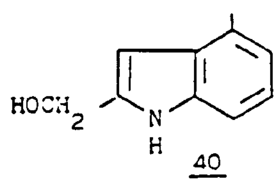

40

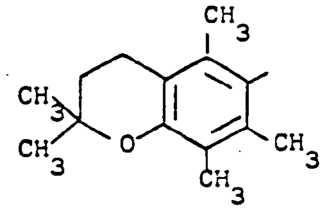

41

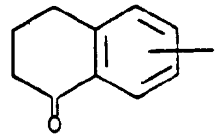

42

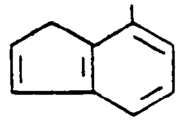

43

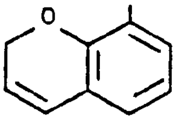

44

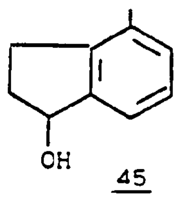

45

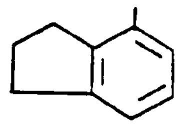

46

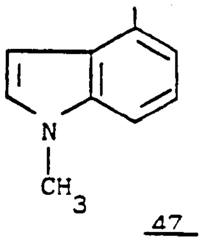

47

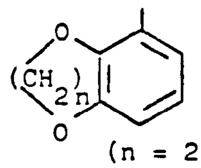

(n = 2,3,4)

48

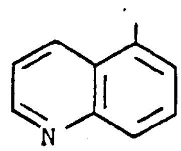

49

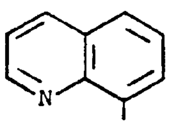

50

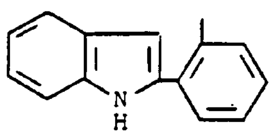

51

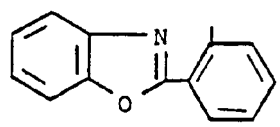

52

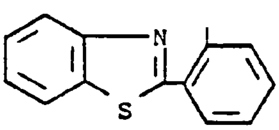

53

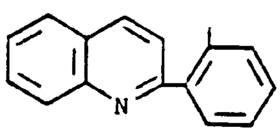

54

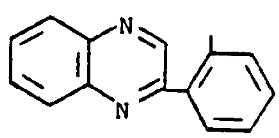

55

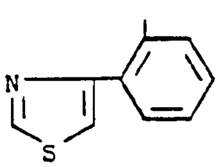

56

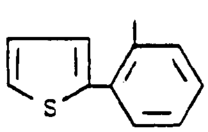

57

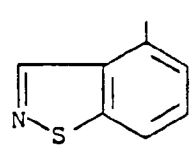

58

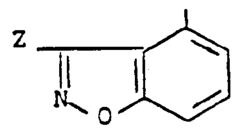

59

(Z = H, alkyle, phényle)

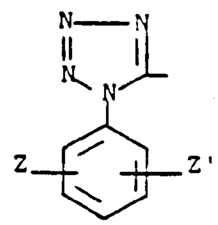

60

(Z = H, halogène, $NO_2$, acétamido)
(Z' = H, $CH_3$)

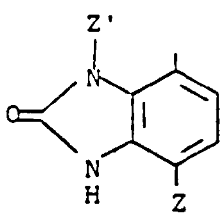

61

(Z et Z' = H, $CH_3$)

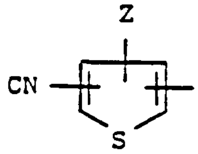

62

(Z = H, alkyle, phénylalkyle, phényle non substitué ou substitué par halogène, alkyle, alcoxy, alkylthio, $CF_3$, OH, carboxy, méthylènedioxy)

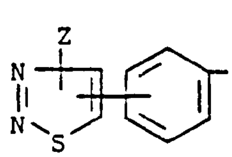

63

(Z = alkyle, H, phényle, CN, $CF_3$, COOH, COOAlkyle, aminocarbonyle, alkylaminocarbonyle, dialkylaminocarbonyle)

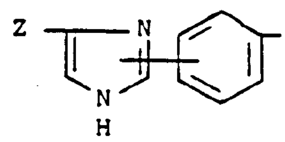

64

(Z = H, alkyle, phényle, $CF_3$)

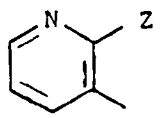

65

(Z = H, $NH_2$)

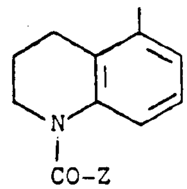

66

(Z = H, alkyle)

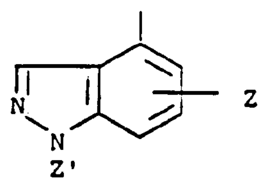

67

(Z = H, alkyle, Z' = H, acétyle)

Selon un de ses aspects avantageux, la présente invention concerne les composés de formule (i), dans laquelle R est tel que défini ci-dessus et Ar représente

(a) un groupe phényle, non substitué ou contenant un ou plusieurs substituants, éventuellement condensé, par les positions 2 et 3, sur un carbocycle à 5 ou 6 atomes de carbone qui

- peut contenir une ou deux doubles liaisons additionnelles,
- peut porter 1 ou 2 substituants,
- peut être à son tour condensé sur un noyau benzénique, ou,
- lorsque ledit carbocycle est à 6 atomes de carbone, peut être ponté sur les positions 3 et 6 par un groupe éthylène ou méthylène;

(b) un groupe phényle condensé, par les position 3 et 4, sur un hétérocycle à 5 membres dont un est un atome d'azote et l'autre un atome de soufre ou un groupe NH, ou bien un groupe phényle, éventuellement substitué, condensé, par les positions 2 et 3, sur

- un hétérocycle à 5 ou 6 membres, aromatique ou non, pouvant contenir une double liaison ou un substituant tel qu'un groupe alkyle inférieur, notamment méthyle, un groupe alcanoyle, notamment acétyle, ou un groupe oxo, un desdits membres étant un atome d'azote, d'oxygène ou de soufre ou un groupe NH ou N-alcanoyle;
- un hétérocycle à 5 membres, aromatique, dont un des membres est un atome d'azote et un autre est un atome de soufre ou un groupe NH;
- un hétérocycle à 6, 7 ou 8 membres, saturé dans la partie non condensée, dont deux sont des atomes d'oxygène directement liés au groupe phényle;

(c) un groupe phényle condensé sur les position 2,3 et 5,6 sur un noyau (a)-furano et sur un noyau (b)-4-oxopyrano et éventuellement substitué sur la position 4;
(d) un groupe 4-carbazolyle;
(e) un groupe 2-thiazolyle éventuellement substitué;
(f) un groupe 3-(1,2,5)-thiadiazolyle éventuellement substitué dans la position 4;

et leurs sels avec des acides minéraux ou organiques.

Dans le groupement (a) ci-dessus, le groupe phényle peut être non substitué ou peut contenir des substituants. Comme substituants additionnels appropriés éventuellement présents sur le noyau phényle, on peut mentionner, par exemple, un ou plusieurs substituants qui sont des atomes d'halogène, par exemple des atomes de fluor, de chlore, de brome ou d'iode, et des radicaux alkyle, alcoxy ou alkylthio, par exemple les radicaux méthyle, éthyle, isopropyle, t-butyle, méthoxy, éthoxy, n-butoxy ou méthylthio, des radicaux alcanoyle, par exemple acétyle, phénylalcanoyle ou benzoyle, et le radical hydroxy, et des radicaux halogénoalkyle, de préférence le radical trifluorométhyle, et des radicaux phényle, phénoxy, 4-tolyloxy, phénylthio, phénylsulfonyle, anilino, morpholino, benzyle, alpha, alpha-diméthylbenzyle ou benzyloxy, et le groupe nitro et des radicaux alkényle, ou encore le groupe cyano.

Dans le groupement (a) ci-dessus, le phényle peut contenir 1 seul substituant, ledit substituant étant dans la position 2. Dans ce cas, ledit substituant est de préférence un groupe alkyle ou alkényle, éventuellement substitué par des halogènes, alkynyle, ou alcoxy, éventuellement substitué sur le groupe alkyle; alkylthio, alkényloxy, alkynyloxy, tétrahydrofurfuryloxy ou phénoxy, un halogène, un groupe cyano, un groupe cycloalkyle, cycloalkényle ou 2,5-méthanocyclohéxyle, ou un groupe alcanoyle, phénylalcanoyle ou benzoyle, ou encore un groupe 2-indolyle, 2-benzoxazolyle, 2-benzothiazolyle, 2-quinolinyle, 2-quinoxalinyle, 2-thiényle, 4-thiazolyle, 4-morpholinyle.

Lorsque dans le groupement (a) Ar est un groupe phényle contenant un substituant en position 3, celui-ci est de préférence un halogène, un alkyle éventuellement substitué par des halogènes, notamment le trifluorométhyle, ou un alkényle, notamment l'allyle, ou le cyano.

Lorsque dans le groupement (a) Ar est un phényle contenant un seul substituant en position 4, celui-ci est de préférence un alkyle éventuellement substitué par un ou plusieurs halogènes, ou par un alkoxy, par un alkoxycarbonylamino, par un carbamoyle ou par un cycloalkylalkoxy; un groupe alkényle éventuellement substitué par un atome d'halogène ou un groupe cyano; un groupe acylamido ou haloacylamido ou un groupe 3-cycloalkylureido.

Lorsque dans le groupement (a) Ar est un phényle contenant deux substituants, ceux-ci peuvent être deux atomes d'halogène, deux groupes alkyle, l'un un atome d'halogène et l'autre un alkyle, ou l'un un groupe 2-acyle, par exemple acétyle ou benzoyle, et l'autre un halogène, un groupe acylamido, par exemple acétylamino, butyrylamino, ou benzylamino, ou un groupe ureido 3-mono ou disubstitué, ou l'un un groupe OH et l'autre un groupe alkyle, hydroxyméthyle, nitro ou carbamoyle, ou l'un est un groupe alkyle et l'autre est un groupe alkylthio, ou encore l'un peut être un groupe 2-acétamido ou 4-acétamido et l'autre un groupe 3-nitro.

Le phényle du groupement (a) peut également contenir 3, 4 ou 5 substituants.

Comme substituants additionnels spécifiques fixés sur le groupe phényle (a) on peut citer les suivants: 2-méthyle, 3-méthyle, 4-méthyle, 4-isopropyle, 2,3-diméthyle, 2,4-diméthyle, 2,5-diméthyle, 3,4-diméthyle, 3,5-diméthyle, 3-méthyl-5-éthyle, 2-t-butyl-5-méthyle, 2-allyle, 2-chloro, 3-chloro, 4-chloro, 2,3-dichloro, 2,5-dichloro, 3,4-dichloro, 3,5-dichloro, 2,4,5-trichloro, 2-iodo, 3-bromo, 3-fluoro, 4-chloro-3-méthyle, 2-chloro-4-méthyle, 4-chloro-3,5-diméthyle, 2,4,6-tribromo-3,5-diméthyle, 2-méthoxy, 3-méthoxy, 2-allyloxy, 2-propargyloxy, 3,5-diméthoxy, 2,3-diméthoxy, 3-t-butyl-4-méthoxy, 3-éthoxy, 2-hydroxy, 4-hydroxy, 2-nitro, 3-nitro, 2-acétamido-3-nitro, 4-acétamido-3-nitro, 3-trifluorométhyle, 4-acétyle, 2-phényle, 2-phénoxy, 3-phénoxy, 3-(4-tolyloxy), 2-benzyle, 2-benzoyl-5-méthoxy, 2-phénylthio, 2-cyano, 3-cyano, 4-cyano.

Lorsque, dans le groupement (a), le groupe phényle est condensé sur un carbocycle à 5 ou 6 atomes de carbone, celui-ci forme de préférence, avec ledit groupe phényle, un radical 4-indényle, 4-indanyle, 1-naphtyle, 5,6-dihydro-1-naphtyle, 5,6,7,8-tétrahydro-1-naphtyle, 5,8-dihydro-1-naphtyle, 6,7-dihydroxy-5,6,7,8-tétrahydro-1-naphtyle, 5,8-éthano-5,6,7,8-tétrahydro-1-naphtyle, éventuellement substitué en position 4 par exemple par un groupe hydroxy, acylamido ou alkylsulfonylamino, 5-oxo-5,6,7,8-tétrahydronaphtyle ou 8-oxo-5,6,7,8-tétrahydronaphtyle, 9-oxo-4-fluorényle.

Parmi les composés de la présente invention caractérisés par la formule (i) ci-dessus, où Ar représente le groupement (a), une sous-classe avantageuse comprend les aryloxypropanolaminotétralines de formule (iA)

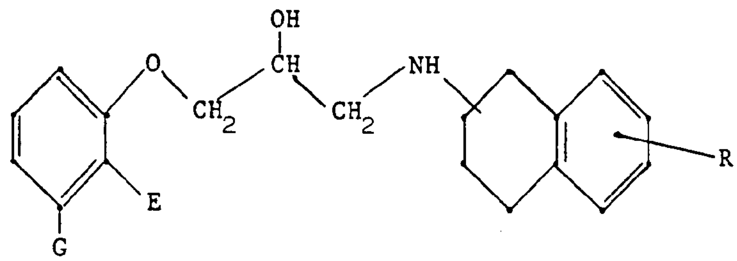

(iA)

dans laquelle R est tel que défini ci-dessus, E représente l'hydrogène, un halogène, un groupe alkyle, alkényle, alkynyle, alcoxy, alkényloxy, alkynyloxy, tétrahydrofurfuryloxy, phénoxy, cycloalkyle de 3 à 6 atomes de carbone, 1-cyclohexényle, 2-cyclohexényle, 2,5-méthanocyclohexyle, alkylthio, alcanoyle, phénylalcanoyle, cyano, 3-chloroallyle, 2-indolyle, 2-benzoxazolyle, 2-benzothiazolyle, 2-quinolinyle, 2-quinoxalinyle, 2-thiényle, 4-thiazolyle, 4-morpholinyle, G est l'hydrogène, un halogène, un alkyle, un alkényle, un groupe trifluorométhyle, un groupe cyano ou un groupe acétamido, au moins un des substituants E et G étant l'hydrogène, ou bien E et G représentent, respectivement, un groupe acétamido et un groupe nitro ou, ensemble, forment un groupe $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, $-CH=CH-CH=CH-$, $-CO-CH_2-CH_2-CH_2-$, $-CH_2-CH_2-CH_2-CO-$ ou $-CH_2-CH(OH)-CH(OH)-CH_2-$, ainsi que leurs sels d'acides minéraux ou organiques.

Parmi les composés de formule (iA), une sous-classe particulièrement avantageuse comprend les composés où R est tel que défini ci-dessus, E représente l'hydrogène, un halogène, un groupe alkyle, alkényle, alkynyle, alcoxy, alkényloxy ou alkynyloxy, G est l'hydrogène ou bien E et G, ensemble, forment un groupe $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, $-CH=CH-CH=CH-$, $-CO-CH_2-CH_2-CH_2-$, $-CH_2-CH_2-CH_2-CO-$ ou $-CH_2-CH(OH)-CH(OH)-CH_2-$, ainsi que leurs sels d'acides minéraux ou organiques.

Une sous-classe encore plus avantageuse comprend les composés de formule (iA) où R est tel que défini ci-dessus, E représente un atome d'halogène, un groupe alkyle, alkényle, alcoxy, alkényloxy ou alkynyloxy et G est l'hydrogène ou bien E et G, ensemble, forment un groupe $-CH=CH-CH=CH-$ ou $-CH_2-CH(OH)-CH(OH)-CH_2-$, ou un de ses sels d'acides minéraux ou organiques.

Les composés de formule (iA) dans laquelle R est l'hydrogène ou un groupe hydroxy, E représente un groupe allyloxy et G est l'hydrogène, leurs formes optiquement actives (SR), (SS), (RS) et (RR) et leurs sels sont particulièrement intéressants.

Le composé de formule (iA) dans laquelle R est l'hydrogène, l'amine est liée à la position 1 de la tétraline et E et G ensemble forment un groupe $-CH=CH-CH=CH-$, ses formes optiquement actives et leurs sels sont aussi particulièrement intéressants.

Parmi les composés de la presente invention caractérisés par la formule (i) ci-dessus, où Ar représente le groupement (a), une autre sous-classe avantageuse comprend les aryloxypropanolaminotétralines de formule (iB)

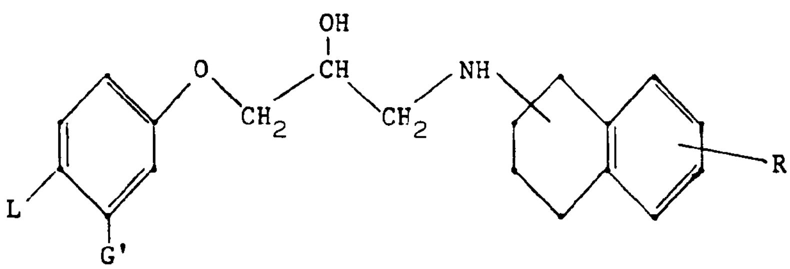

(iB)

dans laquelle R est tel que défini ci-dessus, G' est l'hydrogène, L représente un halogène; un groupe alkyle, non substitué ou substitué par un groupe alcoxy, alcoxycarbonylamino, aminocarbonyle, cycloalkylalcoxy; un groupe alkényle, non substitué ou substitué par un groupe cyano; un groupe alcanoylamino ou haloalcanoylamino; un groupe ureido N'-mono ou disubstitué par des groupe alkyle ou cyclo- alkyle; un groupe alcoxy éventuellement substitué sur l'alkyle par un groupe alcoxy; un groupe cyano; ou un groupe hydroxyméthyle éthérifié par un groupe alcoxyalkyle; ou bien G' et L représentent, respectivement, un groupe nitro et un groupe acétamido; ou bien G' et L, ensemble forment un groupe -CH=CH-CH=CH-; ainsi que leurs sels d'acides minéraux ou organiques.

Une sous-classe encore plus avantageuse comprend les composés de formule (iB) où R est tel que défini ci-dessus, G' représente l'hydrogène, L représente un atome de chlore, un groupe cyano ou un groupe 2-méthoxyéthyle ou bien G' et L ensemble forment un groupe -CH=CH-CH=CH-.

Parmi les composés de la presente invention caractérisés par la formule (i) ci-dessus, où Ar représente le groupement (a), une autre sous-classe avantageuse comprend les aryloxypropanolaminotétralines de formule (iC)

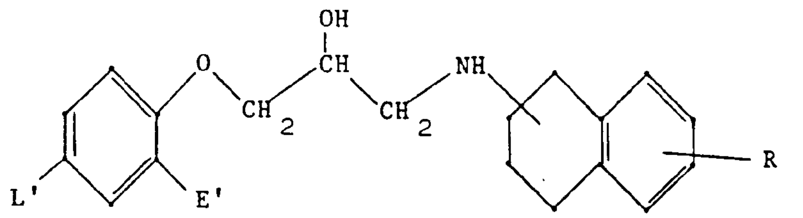

(iC)

dans laquelle R est tel que défini ci-dessus, E' représente un groupe alcanoyle et L' représente un halogène, un groupe alcanoylamino, ou un groupe ureido N'-mono ou disubstitué par un groupe alkyle, ainsi que leurs sels d'acides minéraux ou organiques.

Parmi les composés de la presente invention caractérisés par la formule (i), où Ar représente le groupement (a), une ultérieure sous-classe avantageuse comprend les aryloxypropanolaminotétralines de formule (iD)

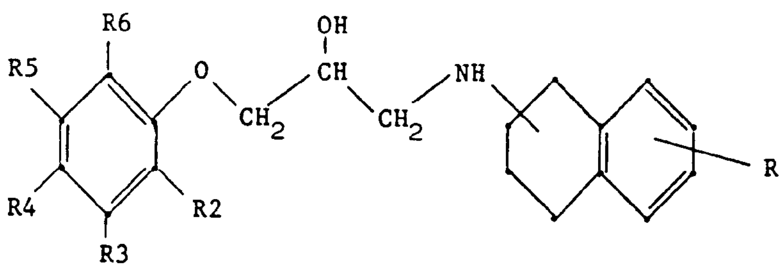

(iD)

dans laquelle R est tel que défini ci-dessus et:

- soit les substituants R2 à R6 peuvent être indépendemment l'hydrogène, un halogène, un groupe alcoxy ou un groupe alkylthio, au moins deux d'entre eux étant autres que l'hydrogène;
- soit un des susbstituants R4 et R5 représente un groupe hydroxy et l'autre l'hydrogène et R3 représente un groupe $CONH_2$ ou $CH_2OH$, R2 et R6 étant l'hydrogène;
- soit R4 représente un groupe alcanoyloxy et R2, R3 et R5 repré sentent un groupe méthyle, R6 étant l'hydrogène,

ainsi que leurs sels d'acides minéraux ou organiques.

Parmi les composés de la presente invention caractérisés par la formule (i), où Ar représente le groupement (a), une autre sous-classe avantageuse comprend les aryloxypropanolaminotétralines de formule (iE)

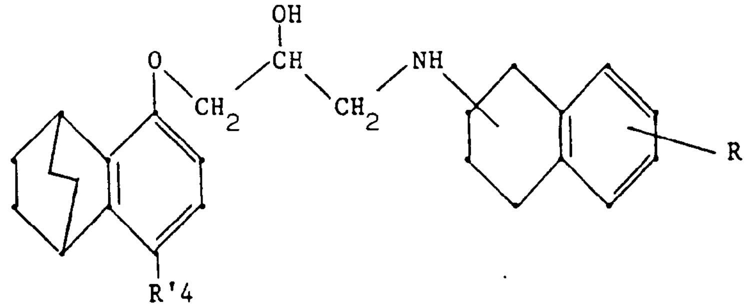

(iE)

dans laquelle R'4 est l'hydrogène, un groupe hydroxy, un groupe alcanoyloxy ou un groupe alkylsulfonylamino et R est tel que défini ci-dessus, ainsi que leurs sels d'acides minéraux ou organiques.

Parmi les composés de la presente invention caractérisés par la formule (i), où Ar représente le groupement (b), une sous-classe avantageuse comprend les aryloxypropanolaminotétralines de formule (iF)

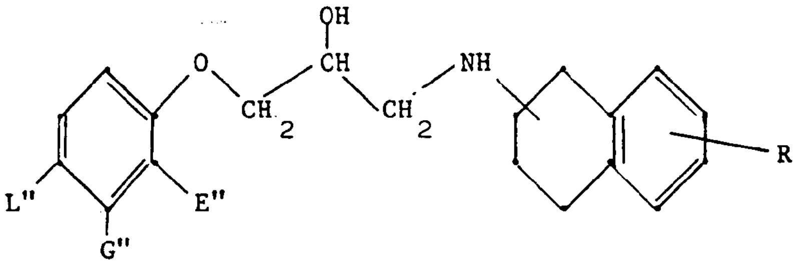

(iF)

dans laquelle R est tel que défini ci-dessus, et

- soit E" représente l'hydrogène et G" et L", ensemble, forment un groupe -N=CH-NH- ou -S-CH=N-;
- soit L" représente l'hydrogène et E" et G", ensemble, forment un groupe -CH=CH-NH-; $-CH=C(CH_3)-NH-$, -CH=CH-O-, $-C(CH_3)=CH-O-$, $-CH=C(CH_3)-O-$, $-CH=C(COCH_3)-O-$, $-CO-O-CH_2-$, -N=CH-NH-, $-S-CH_2-CH_2-CH_2-$, $-O-CH_2-CH_2-O-$ ou $-CH_2-CH_2-N(CHO)-CH_2-$;
- soit L" représente l'hydrogène, un groupe méthyle ou un groupe acétonyloxy et E" et G", ensemble, forment un groupe $-CH_2-CH_2-CO-NH-$;
- soit L" représente l'hydrogène ou un groupe méthyle et E" et G", ensemble, forment un groupe -O-CO-CH=CH-;

ainsi que leurs sels d'acides minéraux ou organiques.

Parmi les composés de formule (iF) sont particulièrement préférés les composés où R est tel que défini ci-dessus, L" représente l'hydrogène et E" et G", ensemble, forment un groupe -CH=CH-NH-, ainsi que leurs sels d'acides minéraux ou organiques.

Parmi les composés de la presente invention caractérisés par la formule (i), où Ar représente le groupement (c), une sous-classe avantageuse comprend les aryloxypropanolaminotétralines de formule (iG)

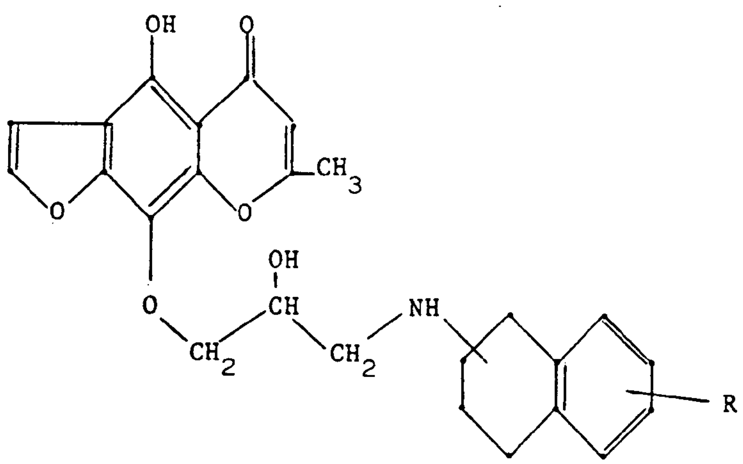

(iG)

dans laquelle R est tel que défini ci-dessus, ainsi que leurs sels d'acides minéraux ou organiques.

Parmi les composés de la présente invention caractérisés par la formule (i), où Ar représente le groupement (d), une sous-classe avantageuse comprend les aryloxypropanolaminotétralines de formule (iH)

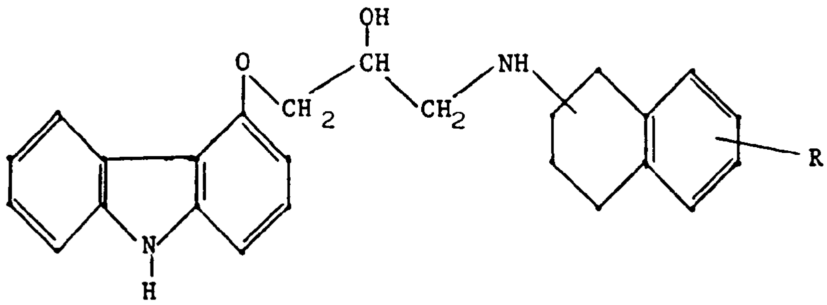

(iH)

dans laquelle R est tel que défini ci-dessus, ainsi que leurs sels d'acides minéraux ou organiques.

Parmi les composés de la présente invention caractérisés par la formule (i), où Ar représente le groupement (e), une sous-classe avantageuse comprend les aryloxypropanolaminotétralines de formule (iI)

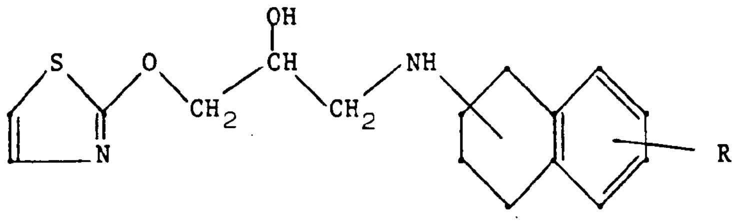

(iI)

dans laquelle R est tel que défini ci-dessus, ainsi que leurs sels d'acide minéraux ou organiques.

Parmi les composés de la présente invention caractérisés par la formule (i), où Ar représente le groupement (f), une sous-classe avantageuse comprend les aryloxypropanolaminotétralines de formule (iJ)

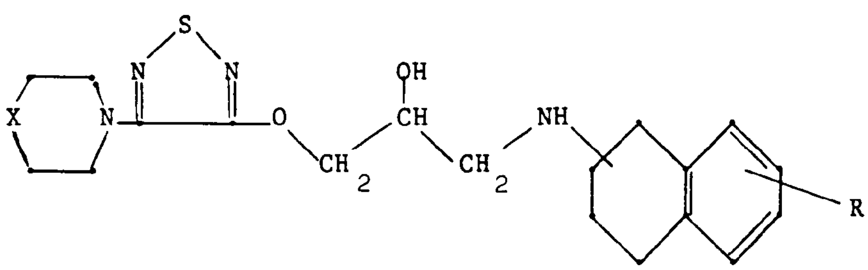

(iJ)

dans laquelle R est tel que défini ci-dessus et X représente un atome d'oxygène ou un groupe méthylène, ainsi que leurs sels d'acides minéraux ou organiques.

Parmi les composés de formule (i), en particulier, sont compris ceux dont Ar représente un des groupements préférés I à LXXVI suivants

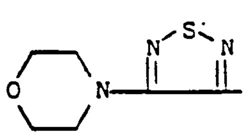

I

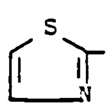

II

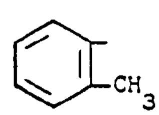

III

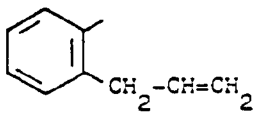

IV

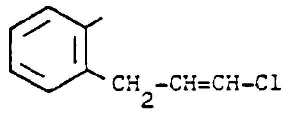

V

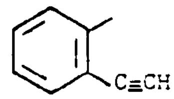

VI

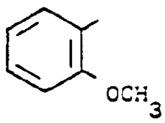

VII

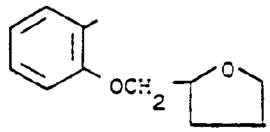

VIII

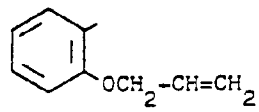

IX

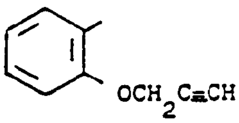

X

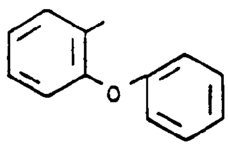

XI

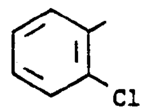

XII

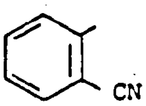

XIII

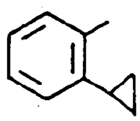

XIV

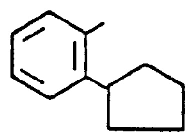

XV

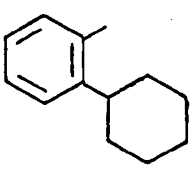

XVI

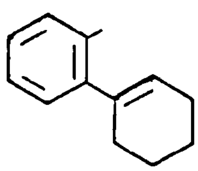

XVII

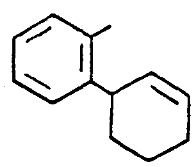

XVIII

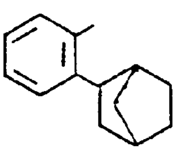

XIX

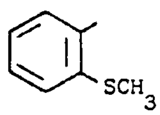

XX

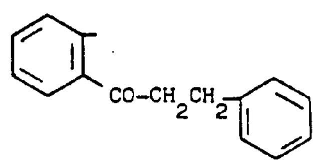

XXI

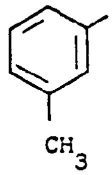

XXII

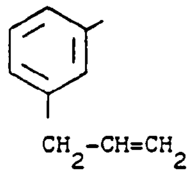

XXIII

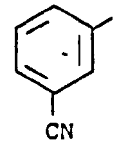

XXIV

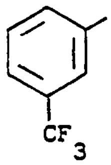

XXV

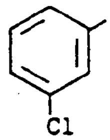

XXVI

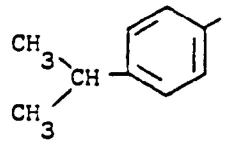

XXVII

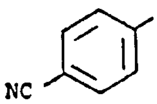

XXVIII

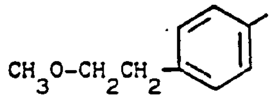

XXIX

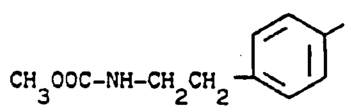

XXX

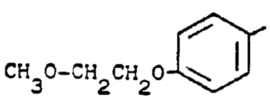

XXXI

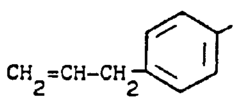

XXXII

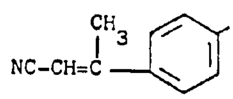

XXXIII

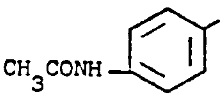

XXXIV

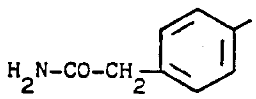

XXXV

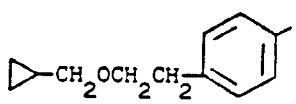

XXXVI

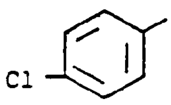

XXXVII

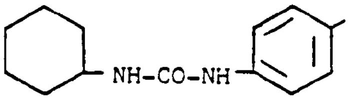

XXXVIII

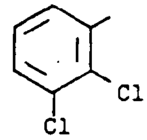

XXXIX

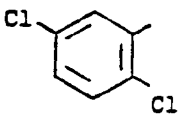

XL

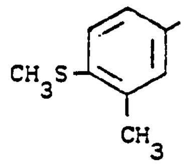

XLI

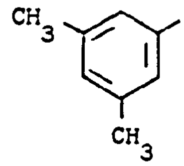

XLII

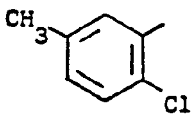

XLIII

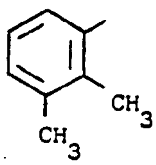

XLIV

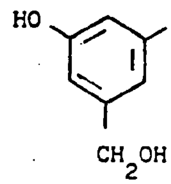

XLV

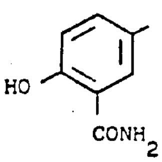

XLVI

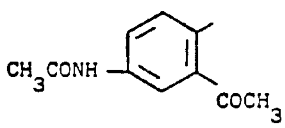

XLVII

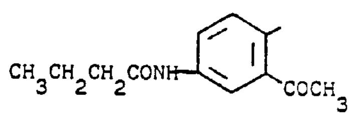

XLVIII

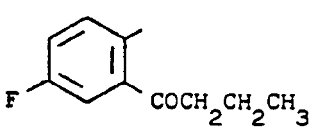

IL

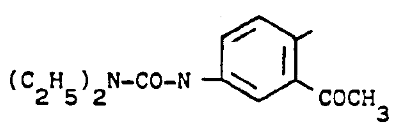

L

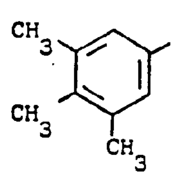

LI

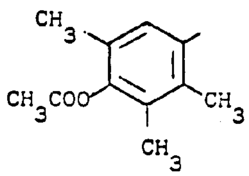

LII

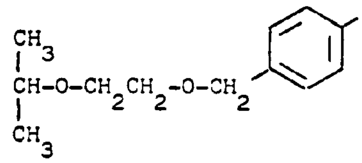

LIII

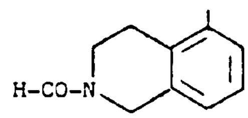

LIV

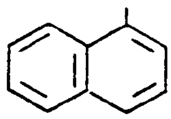

LV

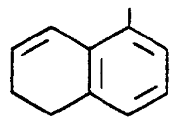

LVI

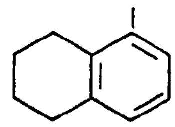

LVII

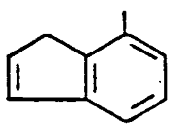

LVIII

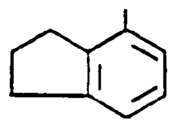

LIX

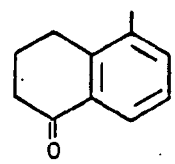

LX

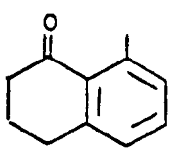

LXI

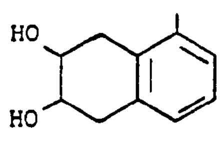

LXII

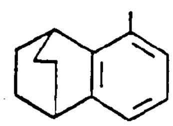

LXIII

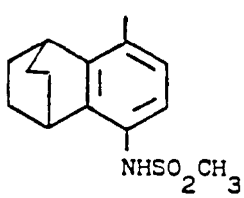

LXIV

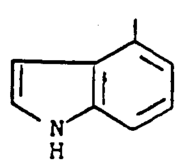

LXV

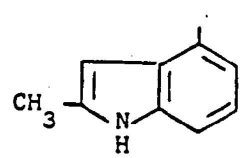

LXVI

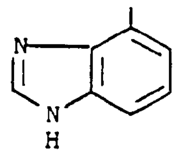

LXVII

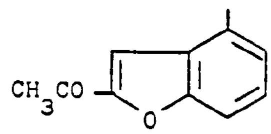

LXVIII ,

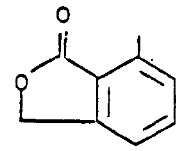

LXIX

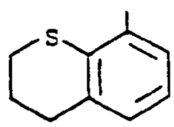

LXX

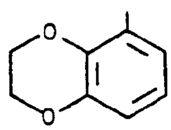

LXXI

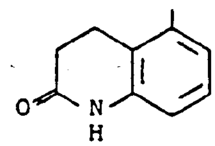

LXXII

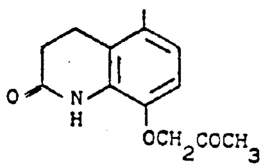

LXXIII

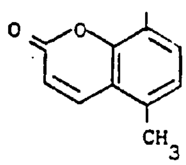

LXXIV

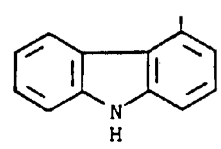

LXXV

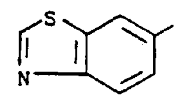

LXXVI

Les composés de formule (i) ci-dessus peuvent être sous une forme optiquement inactive ou sous une forme optiquement active choisie parmi celles des énantiomères, des diastéréoisomères et de leur mélanges. Tous ces composés et leurs sels peuvent être utilisés dans le cadre de la présente invention.

Dans la présente description, les termes "alkyle", "alkényle" et "alkynyle", à moins qu'ils ne soient expressément définis autrement, désignent des radicaux monovalents d'hydrocarbures contenant jusqu'à 4 atomes de carbone, saturés ou insaturés avec une double ou triple liaison, tels que méthyle, éthyle, propyle, isopropyle, allyle, éthynyle ou propargyle.

Les termes "alkoxy", "alkényloxy" ou "alkynyloxy" désignent un groupe hydroxyle substitué par un groupe alkyle, alkényle ou alkynyle tel que défini ci-dessus.

Les termes "alcanoyle" et "alcanoyloxy" désignent, respectivement, le groupe alkylcarbonyle et le groupe hydroxyle substitué par un groupe alkylcarbonyle, alkyle étant comme défini ci-dessus.

Les termes "tétraline" et "tétralone" se rapportent au 1,2,3,4-tétrahydronaphtalène.

Le terme "aryloxypropanolaminotétraline" désigne de façon générale un composé de formule (i) dont la nomenclature appropriée est N-tétrahydronaphtyl-3-aryloxy-2-hydroxypropanamine.

Pour éviter des ambiguités dans la nomenclature des composés de formule (i), le groupe amino des aryloxypropanolaminotétralines est considéré, dans la présente description, comme lié à la position 1 ou 2 de la tétraline et, par conséquent, le groupe R est considéré comme lié dans une des positions 5, 6, 7 et 8.

Par "bêta-antagoniste" ou "bêta-bloquant" on entend "antagoniste des récepteurs bêta-adrénergiques".

Les acides minéraux ou organiques qui forment les sels d'addition selon la présente invention comprennent aussi bien ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le naphtalènesulfonate que ceux qui ne sont pas pharmaceutiquement acceptables mais qui permettent une séparation ou une cristallisation convenable des composés de formule (i), tels que l'acide picrique, l'acide oxalique ou un acide optiquement actif, par exemple un acide mandélique ou un acide camphosulfonique.

Selon un autre de ses aspects, la présente invention concerne un procédé pour la préparation des aryloxypropanolaminotétralines de formule (i) et de leurs sels, caractérisé en ce qu'on traite un 1-aryloxypropane de formule

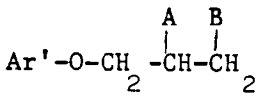

(ii)

dans laquelle Ar' a la signification donnée ci-dessus à Ar, mais peut contenir des groupes N-protecteurs fixés sur des groupes $NH_2$ ou NH, A est un groupe hydroxy, B est un halogène choisi parmi le chlore, le brome ou l'iode ou bien A et B, ensemble, forment un groupe époxy, avec une aminotétraline de formule

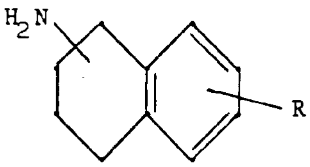

(iii)

dans laquelle R est tel que défini ci-dessus, éventuellement en présence d'une amine tertiaire; on déprotège éventuellement le produit ainsi obtenu par élimination du groupe N-protecteur; lorsque dans le composé de départ de formule (ii) Ar' est un groupe 2-acétamido-3-nitrophényle ou 4-acétamido-3-nitrophényle, on déacétyle éventuellement le produit obtenu par chauffage dans l'acide chlorhydrique et on soumet éventuellement le produit obtenu à un hydrogénation en présence de palladium sur charbon à 10% dans de l'acide formique à 97%; et on transforme éventuellement le produit ainsi obtenu dans un de ses sels d'acides minéraux ou organiques.

Dans le présent contexte, le terme "groupe N-protecteur" désigne un groupement protecteur d'amines facilement éliminable, comme un groupe formyle, un groupement alkylcarbonyle tel qu'acétyle ou propionyle, un groupement arylcarbonyle tel que benzoyle, un groupement alkylsulfonyle tel que méthanesulfonyle ou un groupement arylsulfonyle tel que benzènesulfonyle ou p-toluènesulfonyle; le groupement arylsulfonyle et en particulier les groupements benzènesulfonyle et p-toluènesulfonyle sont les groupes N-protecteurs préférés.

La réaction entre le 1-aryloxypropane (ii) et l'aminotétraline (iii) est mise en oeuvre en chauffant au reflux les réactifs dans un solvant approprié tel qu'un solvant polaire aprotique comme le diméthylsulfoxyde ou le diméthylacétamide ou un alcool inférieur, par exemple l'éthanol, le n-propanol ou l'isopropanol, éventuellement en présence d'une amine tertiaire comme la triéthylamine ou la 1-méthylpipéridine.

La N-déprotection éventuelle du produit ainsi obtenu est effectuée par des méthodes classiques, par exemple par chauffage au reflux dans un solvant approprié tel qu'un alcool inférieur comme l'éthanol, le n-propanol ou l'isopropanol, en présence d'un hydroxyde de métal alcalin tel que l'hydroxyde de sodium.

Lorsque le composé de départ a la formule (ii) ci-dessus où Ar' représente un groupe 2-acétamido-3-nitrophényle ou 4-acétamido-3-nitrophényle, le produit ainsi obtenu peut être déacétylé par chauffage dans l'acide chlorhydrique, de préférence 4N, de façon à obtenir un composé de formule (i) où Ar représente un groupe 2-amino-3-nitrophényle ou 4-amino-3-nitrophényle. Le groupe nitro de ces produits peut être réduit par hydrogénation catalytique (Pd/C à 10%) dans de l'acide formique à 97% de façon à réduire le groupe nitro et, en même temps, à cycliser pour obtenir un composé de formule (i) où Ar est 4-benzimidazolyle ou 5-benzimidazolyle.

Le produit de formule (i) ainsi obtenu est isolé, sous forme de base libre ou de sel, selon les techniques conventionnelles.

Lorsque le composé de formule (i) est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques conventionnelles. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2-sulfonate.

A la fin de la réaction du composé (ii) avec le composé (iii), le composé de formule (i) peut être isolé sous

forme d'un de ses sels, par exemple le chlorhydrate ou l'oxalate; dans ce cas, s'il est nécessaire, la base libre peut être préparée par neutralisation du dit sel avec une base minérale ou organique telle que l'hydroxyde de sodium ou la triéthylamine ou avec un carbonate ou bicarbonate alcalin, tel que le carbonate ou bicarbonate de sodium ou de potassium.

Les réactions qui forment l'ensemble de ce procédé ne modifient pas la stéréochimie des composés concernés. Ainsi, le procédé de la présente invention permet d'opérer tant sur des mélanges racémiques que sur des isomères optiquement purs.

Ainsi, en partant d'un 1-aryloxypropane (ii) et d'une aminotétraline (iii) racémiques, on obtient un mélange de stéréoisomères RR, SS et RS, SR. De même, en partant, par exemple, d'un 1-aryloxy-2,3-époxypropane optiquement actif et d'une aminotétraline racémique, on obtient une aryloxypropanolaminotétraline de formule (i) sous forme de couple de diastéréoisomères (RR+RS) ou (SS+SR) qui, par séparation, donne les énantiomères purs (RR) et (RS) ou (SS) et (SR). Si le 1-aryloxy-2,3-époxypropane est sous forme optiquement active, l'aminotétraline de formule (iii) sera de préférence utilisée sous forme optiquement active, de façon à préparer un isomère optiquement pur.

Les produits de départ de formule (ii) ci-dessus sont décrits généralement en littérature, ou peuvent être préparés selon des méthodes bien connues.

Notamment, les méthodes générales de préparation des composés de formule (ii) sont illustrées dans US 3,501,769.

Ainsi, les 1-aryloxypropanes de formule (ii), dans laquelle A est un groupe hydroxy et B est un halogène, sont préparés à partir des phénols correspondants Ar'OH avec une épihalogénhydrine, de préférence l'épichlorhydrine, en présence d'une amine tertiaire.

Les 1-aryloxypropanes de formule (ii) dans laquelle A et B, ensemble, forment un groupe époxy sont également préparés à partir des phénols correspondant Ar'OH, mais sous forme de leurs sels alcalins (en opérant donc dans un milieu rendu basique par la présence d'un hydroxyde alcalin, par exemple l'hydroxyde de sodium) avec l'épichlorhydrine.

Les composés de formule (ii), dans laquelle A et B, ensemble, forment un époxy, sont préparés sous forme optiquement active par des méthodes connues, plus particulièrement en faisant réagir le phénol correspondant avec l'épichlorhydrine sous forme optiquement active (R) ou (S) (J. Org. Chem. 1978, 43, 4876-4878), et en opérant dans les même conditions que ci-dessus. Les époxydes de configuration (S) peuvent également être obtenus selon DE 2 453 324.

Par exemple, les composés de formule (ii) dans laquelle Ar' est un des groupement 1 à 67, sont décrits dans les documents suivants: 1: US 3,912,733; 2: DE 2 240 599; 3: DE 2 353 996; 4: DE 2 418 776; 5: BE 813.751; 6: BE 753.917; 7: BE 733.390;8: FR 1 588 855, DE 2 021 958; 9: US 3,920,691; 10: NL 73/05478; 11: BE 794.669; 12: FR 1 466 164; 13: BE 724.929; 14: BE 754.860; 15: BE 764.659, BE 739.545; 16: CH 526 542, CH 526 544; 17: CH 527 188; 18: BE 793.073; 19: BE 765.313; 20: NL 72/14438; 21: DE 2 362 877; 22: BE 852.556; 23: JP 73-01070; 24: BE 783.440; 25: DE 2 620 179; 26: FR 2 042 378, DE 2 021 958; 27, 28, 29, 30, 47, 48, 49, 50, 51, 52, 53, 54, 55, 56, 57: J. Med. Chem., 1972, 15, 260-266; 31: US 3,894,058; 32: DE 2 404 858; 33: BE 815.745; 34: FR 2 077 694; 35: JP 49-048649; 36: JP 49-094666; 37: NL 70/07503; 38: BE 753.840; 39: BE 773.205; 40: BE 739.545; 41: NL 69/011816; 42: BE 739.195; 43: DE 1 955 229; 44: JP 72-00055; 45: JP 49-051258; 46: NL 86/05887; 58: BE 863.622; 59: DE 2 711 382, BE 846.010; 60: BE 866.278; 61: BE 866.596; 62: DE 2 720 613; 63: US 3,940,407; 64: DE 2 608 448; 65: BE 845.049; 66: JP 52-053868; 67: BE 853.949.

Plus particulièrement, des composés de formule (ii) dans laquelle Ar' est un des groupements préférés I à LXXVII ci-dessus sont décrit dans les documents suivants: I: FR 2 009 110; II: FR 2 199 463; III: FR 2 023 556 ou US 3,501,769; IV, V, XXIII, XXXII: FR 1 479 614; VI: FR 1 510 271; VII: US 3,501,769; VIII: FR 2 051 536; IX: FR 1 463 034; X: FR 1 583 559; XI, XII, XXII, XXVII: US 3,501,769; XIII, XIV, XXVIII: FR 1 583 559, NL 67/17837; XIV: GB 1 294 159; XV: FR 1 575 615; XVI, XVII, XVIII: DE 2 636 725; XIX: FR 2 267 095 ou FR 2 361 106; XX: FR 1 552 786; XXI: FR 2 144 601; XXII: FR 1 555 463 ou US 3,501,769; XXV, XXVI: US 3,501,769; XXIX, XXX: FR 2 081 523; XXXI: FR 2 044 806; XXXIII: FR 2 353 520; XXXIV: FR 1 458 635; XXXV: FR 2 034 561; XXXVI: FR 2 330 383; XXXVII: US 3,501,769; XXXVIII: FR 2 113 982; XXXIX: FR 2 261 001 ou US 3,501,769; XL: FR 2 130 284 ou US 3,501,769; XLI: FR 2 070 102; XLII: US 3 501 769; XLIII: BE 691.159 ou US 3,309,406; XLIV: FR 2 073 434 ou FR 2 215 419 ou US 3,501,769; XLV: US 3,857,873; XLVI: US 3 883 560; XLVII, XLVIII: GB 1 247 384; IL: FR 2 276 032; L: FR 2 255 058; LI: US 3,501,769; LII: FR 1 567 149; LIII: BE 859.425, LIV: DE 2 620 179; LV: GB 994 918; LVI: FR 1 601 338; LVII: BE 641.417; LVIII: FR 2 035 816; LIX: FR 8298 M, BE 641.417; LX: FR 2 018 626 ou BE 755.071; LXI: EP 67 106; LXII: FR 2 100 811, DE 2 258 995; LXIII: FR 2 059 580; LXIV: FR 2 157 897; LXV, LXVI: FR 1 466 164; LXVII: J. Med. Chem., 1979, 22, 210-214, qui décrit le précurseur 1-(2-acétamido-3-nitrophénoxy)-2,3-époxypropane de formule (ii) où Ar est 2-acétamido-3-nitrophényle; LXVIII: FR 2 137 901; LXIX: FR 2 231 387; LXX: FR 2 092 004; LXXI: J. Med. Chem.,

1972, 15, 260-266; LXXII: FR 2 179 715; LXXIII: FR 2 344 538; LXXIV: FR 2 042 378; LXXV: FR 2 196 165; LXXVI: FR 2 225 153.

Les aminotétralines de départ de formule (iii) sont décrites en littérature ou peuvent être aisément préparées par réaction de 1-tétralones ou de 2-tétralones de formule

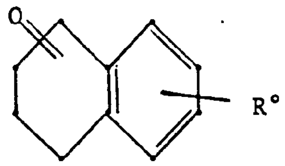

(iv)

dans laquelle R° représente l'hydrogène ou un groupe méthoxy, avec la benzylamine, réduction, par du borohydrure de sodium, de la benzylimine ainsi obtenue, débenzylation par hydrogénation catalytique et déméthylation éventuelle par l'acide bromhydrique à 48%.

Les deux formes optiquement actives des aminotétralines de formule (iii) sont préparées par résolution des racémates selon des méthodes connues, par exemple par salification avec un acide optiquement actif, l'acide mandélique de préférence.

Les composés de formule (i) sont des bêta-antagonistes agissant sur les récepteurs bêta de l'intestin, tout en ayant une activité réduite sur les récepteurs cardiaques (oreillette droite de cobaye) et respiratoires (trachée de cobaye).

L'effet antagoniste des récepteurs bêta-adrénergiques sur le côlon isolé de rat a été mis en évidence selon la méthode décrite dans la demande de brevet européen 255415 et cet effet s'exerce tant vis-à-vis des bêta-agonistes classiques, par exemple l'isoprénaline, que des bêta-agonistes sélectifs, par exemple le SR 58375 A (EP 211721).

L'effet sur les récepteurs cardiaques et respiratoires a été évalué par les tests classiques in vitro sur l'oreillette droite de cobaye et sur la trachée isolée de cobaye (Pharmacol. Res. Comm., 1988, 20, 147-151). Dans ces tests les composés de formule (i) se sont demontrés peu actifs.

Plus particulièrement, on a constaté que les composés de formule (i) sont beaucoup plus actifs que les bêta-antagonistes connus sur le côlon isolé de rat et beaucoup moins actifs des composés connus sur l'oreillette et sur la trachée.

Ces propriétés surprenantes des composés de formule (i) ci-dessus permettent d'envisager leur utilisation comme bêta-antagonistes dans des domaines autres que cardiovasculaire et respiratoire.

De plus, les composés de formule (i) sont peu toxiques; notamment, leur toxicité aigue est compatible avec leur utilisation comme médicaments, notamment gastro-intestinaux, psychotropes, antiglaucomateux, antimigraneux ou en général pour le traitement de maladies dans lesquelles les bêta-antagonistes trouvent leur application, par exemple la thyrotoxicose ou l'hyperparathyroidisme, chez les mammifères.

Pour une telle utilisation, on administre auxdits mammifères, qui nécessitent dudit traitement, une quantité efficace d'un composé de formule (i) ou d'un de ses sels pharmaceutiquement acceptables.

Les composés de formule (i) ci-dessus et leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 mg à 5000 mg par jour, plus particulièrement de 2,5 à 2500 mg selon l'age du sujet à traiter, le type de traitement, prophylactique ou curatif, et la gravité de l'affection. Les composés de formule (i) sont généralement administrés en unité de dosage de 0,1 à 500 mg, de préférence de 0,5 à 250 mg de principe actif, une à cinq fois par jour.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (i) ci-dessus ou un de ses sels pharmaceutiquement acceptables.

Dans les compositions pharmaceutiques de la présente invention pour l'administration orale, sublinguale, sous-cutanée, intramusculaire, intraveineuse, topique, transdermique ou rectale, les ingrédients actifs de formule (i) ci-dessus peuvent être administrés sous formes unitaires d'administration, en mélange avec des supports pharmaceutiques classiques, aux animaux et aux êtres humains pour le traitement des susdites affections. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire ou intraveineuse,

les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration locale, on mélange le principe actif dans un excipient pour la préparation de crèmes ou ounguents ou on le dissout dans une véhicule pour l'administration intraoculaire, par exemple sous forme de collyre.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les composés de formule (i), notamment les composés (i) marquées par un isotope, peuvent aussi être utilisés comme outils de laboratoire dans des essais biochimiques.

Les composés de formule (i) se lient sélectivement à un récepteur bêta-adrénergique présent dans l'intestin des mammifères qui est différent des récepteurs bêta-1 et bêta-2 connus. On peut donc utiliser ces composés dans un essai ordinaire de "binding", dans lequel on emploie le côlon de rat où ce récepteur est particulièrement abondant, et on mesure la quantité de composé (i) déplacé par le composé en examen, pour évaluer l'affinité du composé vis-à-vis des sites de liaison de ce récepteur particulier.

En associant cet essai biochimique à d'autres essais conventionnels de binding pour les récepteurs bêta-adrénergiques classiques (bêta-1 et bêta-2), on peut reconnaître parmi les composés organiques examinés, ceux qui sont capables de se lier sélectivement à ce récepteur et peuvent donc exercer une action spécifique sur l'intestin sans effects secondaires cardiaques ou respiratoires.

Un autre objet spécifique de la présente invention est donc un réactif utilisable dans les essais biochimiques pour la différentiation des récepteurs bêta-adrénergiques, qui comprend au moins un composé de formule (i) convenablement marqué.

Les exemples suivants illustrent l'invention sans toutefois la limiter. Le symbole du pouvoir rotatoire est indiqué comme /alpha/, mais on doit l'entendre comme $[alpha]_D^{20}$. Les chromatographies ont été effectuées sur des colonnes de gel de silice MERCK 70-230 mesh.

PREPARATION I

Bromhydrate de 2-amino-7-hydroxytétraline.

(a) On chauffe au reflux pendant 3 heures un mélange de 8 g de 7-méthoxy-2-tétralone, 4,8 g de benzylamine, 150 ml de toluène anhydre et 100 mg d'acide p.toluènesulfonique. On évapore à sec l'huile résiduelle, on reprend par 100 ml de méthanol et à la solution obtenue on ajoute, avec précaution et à 0-5°C, 8,5 g de borohydrure de sodium. On laisse le mélange sous agitation et à la température ambiante pendant une nuit, puis on y ajoute 50 ml d'eau, on laisse sous agitation pendant 30 minutes, on évapore le solvant, on reprend avec 30 ml d'eau et 10 ml d'une solution concentrée d'hydroxyde d'ammonium. On extrait avec 200 ml d'acétate d'éthyle, on sèche la phase organique sur du sulfate de sodium, filtre et évapore à sec. On obtient une huile de couleur foncée qui est purifiée par flash chromatographie en utilisant comme éluant le mélange acétate d'éthyle/méthanol 95/5. On transforme la base obtenue en son chlorhydrate par dissolution dans 40 ml d'isopropanol et addition d'isopropanol saturé d'acide chlorhydrique gazeux. On obtient ainsi 11,4 g de chlorhydrate de 7-méthoxy-2-benzylamino-1,2,3,4-tétrahydronaphtalène; p.f. 265-267°C (déc.).

(b) On soumet le produit ci-dessus, dissous dans 200 ml de méthanol et 100 ml d'eau, à une hydrogénation en présence de 1,2 g de palladium sur charbon à 10%, à la pression ambiante et à la température de 45-50°C. Après 4 heures on filtre, évapore à sec, reprend deux fois avec de l'éthanol absolu et l'on évapore à sec. On obtient un solide blanc qui est repris avec 70 ml d'isopropanol à chaud. Par refroidissement, la suspension obtenue précipite et donne 7,8 g de chlorhydrate de 2-amino-7-méthoxytétraline; p.f. 214-216°C.

(c) On met en suspension 6,6 g du produit ci-dessus dans 80 ml d'acide bromhydrique à 48% et on chauffe le mélange au reflux pendant 2 heures. On évapore à sec la solution obtenue, on reprend par de l'éthanol absolu et on évapore à sec deux fois. On obtient ainsi une huile qu'on dissout dans 20 ml d'isopropanol à chaud. Par addition de 30 ml d'éther éthylique à la solution on obtient 6,8 g de bromhydrate de 2-amino-7-hydroxytétraline cristallin; p.f. 171-173°C.

PREPARATION II

Monohydrate de R(+)-2-amino-7-hydroxytétraline.

A une solution dans 550 ml d'éthanol absolu de 50 g de 2-amino-7-méthoxytétraline base brute, obtenue du chlorhydrate correspondant (PREPARATION I b) par neutralisation avec de l'hydroxyde de sodium à 10%, extraction avec de l'acétate d'éthyle et évaporation du solvant, on ajoute une solution de 43 g d'acide (+) mandélique dans 550 ml d'éthanol absolu. Après une nuit à la température ambiante, on filtre le précipité obtenu et on le cristallise deux fois dans l'éthanol absolu en recupérant chaque fois le produit cristallisé après repos d'une nuit à la température ambiante. On obtient ainsi 34,2 g (74%) de sel pur de l'acide (+) mandélique avec la (+)-2-amino-7-méthoxytétraline; p.f. 190-192°C. (Les eaux mères de cette première cristallisation sont séparées et utilisées pour la PREPARATION III ci-dessous). On suspend 34 g du sel ainsi obtenu dans 300 ml d'eau et on rend basique le mélange réactionnel avec de l'hydroxyde de sodium N. On extrait la base avec de l'acétate d'éthyle, on évapore à sec et on reprend le residu par 260 ml d'acide bromhydrique à 48%. On chauffe au reflux le mélange réactionnel pendant trois heures, on l'évapore à sec sous vide et on reprend le résidu obtenu avec 70 ml d'eau. On rend basique la solution aqueuse avec de l'hydroxyde d'ammonium concentré, on la refroidit pendant une nuit et on la filtre. On obtient 17 g de R(+)-2-amino-7-hydroxytétraline sous forme monohydratée; p.f. 143-144°C, /alpha/ = + 85,1° (méthanol, c = 0,5 %).

Le chlorhydrate de ce produit a un pouvoir rotatoire qui correspond à celui de la littérature (Molecular Pharmacology, 1982, 22, 281-289).

PREPARATION III

Monohydrate de S(-)-2-amino-7-hydroxytétraline.

On évapore à sec les eaux mères de la première cristallisation du produit de la PREPARATION II, on suspend le résidu ainsi obtenu dans 300 ml d'eau et on rend la solution basique par de l'hydroxyde de sodium N. On extrait la base avec de l'acétate d'éthyle. Suivant le mode opératoire décrit dans la PREPARATION II et en utilisant comme produits de départ la base ainsi obtenue et l'acide (-) mandélique, on obtient le sel de l'acide (-) mandélique avec la (-)-2-amino-7-méthoxytétraline (p.f. 189-191°C) qui, par neutralisation et déméthylation par l'acide bromhydrique, donne 17 g de S(-)-2-amino-7-hydroxytétraline, sous forme de monohydrate; p.f. 143-144°C, /alpha/ = - 86,9° (méthanol, c = 0,5 %).

Le chlorhydrate de ce produit a un pouvoir rotatoire qui correspond à celui de la littérature (Molecular Pharmacology 1982, 22, 281-289).

EXEMPLE 1.

On chauffe au reflux pendant 5 heures une solution de 3 g de 2-amino-7-hydroxytétraline base, obtenue du bromhydrate de la PREPARATION I par neutralisation avec de l'hydroxyde d'ammonium et extraction par un mélange acétate d'éthyle/éthanol en rapport 9/1 v/v, et de 3,68 g de 1-naphtyloxy-2,3-époxypropane dans 80 ml d'éthanol. On laisse refroidir le mélange réactionnel jusqu'à la température ambiante. On évapore le solvant sous pression réduite et on soumet l'huile ainsi obtenue à une chromatographie sur une colonne de silice en éluant d'abord par de l'acétate d'éthyle jusqu'à la complète élution de la première tache et ensuite par un mélange acétate d'éthyle/méthanol 9/1 v/v jusqu'à la complète élution des fractions du produit. On réunit les fractions ainsi obtenues et on les concentre sous pression réduite. On dissout à chaud le résidu dans 25 ml

d'isopropanol, on rend la solution acide par de l'acide chlorhydrique dans l'isopropanol et on filtre le précipité cristallin. On obtient ainsi 3,6 g de chlorhydrate de N-(7-hydroxy-1,2,3,4-tétrahydronapht-2-yl)-2-hydroxy-3-(1-naphtyloxy)propanamine; p.f. 184-187°C.

EXEMPLE 2.

On chauffe au reflux pendant 6 heures une solution de 3,2 g de 2-aminotétraline base, obtenue par neutralisation de son chlorhydrate par de l'hydroxyde de sodium et extraction par de l'acétate d'éthyle, et de 4,34 g de 1-naphtyloxy-2,3-époxypropane dans 90 ml d'éthanol absolu. Après évaporation à sec, on dissout le résidu dans 50 ml d'isopropanol et on rend acide la solution par de l'acide chlorhydrique dans l'isopropanol. On filtre le précipité ainsi obtenu et on obtient 5,6 g d'un produit qui après cristallisation dans 150 ml d'éthanol 95%, donne 3 g du chlorhydrate de N-(1,2,3,4-tétrahydronapht-2-yl)-2-hydroxy-3-(1-naphtyloxy)propanamine; p.f. 209-212°C.

EXEMPLE 3.

On chauffe au reflux pendant 6 heures une solution de 2,65 g de 1-aminotétraline et 3,6 g de 1-naphtyloxy-2,3-époxypropane dans 80 ml d'éthanol absolu. Après évaporation sous pression réduite, on obtient une huile qu'on chromatographie sur colonne de gel de silice en éluant par de l'acétate d'éthyle jusqu'à la complète élution des fractions du produit, qui sont ensuite réunies et évaporées sous pression réduite. On dissout le produit dans 40 ml d'isopropanol et on rend acide la solution par de l'acide chlorhydrique dans de l'isopropanol. On filtre le précipité ainsi obtenu et, après cristallisation dans 70 ml d'éthanol 95%, on obtient 2,5 g de chlorhydrate de N-(1,2,3,4-tétrahydronapht-1-yl)-2-hydroxy-3-(1-naphtyloxy)propanamine; p.f. 179-183°C.

EXEMPLE 4.

On chauffe au reflux pendant 5 heures une solution de 4,7 g de 2-amino-7-hydroxytétraline base et 5,48 g de 1-(2-allyl)-phénoxy-2,3-époxypropane dans 120 ml d'éthanol absolu. On évapore à sec le solvant, on reprend le résidu par de l'isopropanol et on acidifie avec un mélange d'acide chlorhydrique dans de l'isopropanol. Après 30 minutes à 0-5°C, on filtre le précipité ainsi obtenu, on lave à l'éther isopropylique et on sèche à l'étuve. Après cristallisation dans 250 ml d'éthanol absolu, on obtient 3,1 g de chlorhydrate de N-(7-hydroxy-1,2,3,4-tétrahydronapht-2-yl)-2-hydroxy-3-(2-allylphénoxy)propanamine; p.f. 232-235°C.

EXEMPLE 5.

En opérant comme décrit dans l'Exemple 2 et en remplaçant le 1-naphtyloxy-2,3-époxypropane par le 1-(2-allyl)phénoxy-2,3-époxypropane, on obtient un produit qui, après cristallisation dans de l'éthanol absolu, donne le chlorhydrate de N-(1,2,3,4-tétrahydronapht-2-yl)-2-hydroxy-3-(2-allylphénoxy)propanamine; p.f. 175-177°C; rendement 57%.

EXEMPLE 6.

On chauffe au reflux pendant 5 heures une solution de 2,5 g de 1-aminotétraline et 3,23 g de 1-(2-allyl)phénoxy-2,3-époxypropane dans 70 ml d'éthanol. Après évaporation sous pression réduite, on obtient une huile qu'on chromatographie sur colonne de gel de silice en éluant par de l'acétate d'éthyle jusqu'à la complète élution des fractions du produit. On évapore sous pression réduite les fractions réunies et l'on obtient une huile qu'on dissout dans 50 ml d'éther éthylique. Par addition d'une solution de 1,1 g d'acide oxalique dans 10 ml d'acétone, on obtient un précipité qui est filtré et cristallisé dans de l'éthanol 95%. On obtient ainsi 3,0 g d'oxalate de N-(1,2,3,4-tétrahydronapht-1-yl)-2-hydroxy-3-(2-allylphénoxy)propanamine; p.f. 163-165°C.

EXEMPLE 7.

On chauffe au reflux pendant 4 heures un mélange de 20 g de 1-[1-(p-toluènesulfonyl)indol-4-yloxy]-2,3-époxypropane (EP 228 356) et 10,5 g de 2-amino-7-hydroxytétraline base dans 300 ml d'isopropanol. Après évaporation du solvant sous pression réduite, on dissout le résidu dans de l'acétate d'éthyle et on le lave à l'eau. On sèche sur du sulfate de sodium et on évapore sous pression réduite. On reprend le résidu d'abord par 200 ml d'éthanol et, ensuite, par une solution de 15 g d'hydroxyde de sodium dans 90 ml d'eau. On chauffe au reflux le mélange réactionnel pendant 4 heures, on le rend légèrement acide par de l'acide chlorhydrique

N et ensuite on le rend basique par de l'hydroxyde d'ammonium concentré. On concentre sous pression réduite, on reprend par de l'éthanol absolu et on concentre à sec, en répétant l'opération deux fois. On reprend le résidu par de l'éthanol, on filtre le chlorure de sodium et on le lave sur le filtre avec de l'éthanol en récuperant tout le produit. On rend la solution basique par de l'hydroxyde d'ammonium concentré et ensuite on évapore à sec. On chromatographie le résidu sur une colonne de gel de silice en éluant d'abord par de l'acétate d'éthyle et ensuite par un mélange acétate d'éthyle/méthanol 9/1 v/v. On récupère la tache du produit, on l'évapore à sec et l'on ajoute de l'acide acétique dans l'isopropanol. On obtient un précipité qui, après cristallisation dans 60 ml d'éthanol à 95%, donne 2,6 g d'acétate de N-(7-hydroxy-1,2,3,4-tétrahydronapht-2-yl)-2-hydroxy-3-indol-4yloxypropanamine; p.f. 167-170°C.

EXEMPLE 8.

On chauffe au reflux pendant 4 heures 12,45 g de 1-[1-(p-toluène-sulfonyl)indol-4-yloxy]-2,3-époxypropane et 6,5 g de monohydrate de R(+)-2-amino-7-hydroxytétraline, PREPARATION II, dans 200 ml d'isopropanol, puis on évapore le solvant sous pression réduite, on dissout le résidu dans de l'acétate d'éthyle et on lave la solution à l'eau. On sèche sur du sulfate de sodium et on évapore sous pression réduite. On reprend le résidu par 150 ml d'éthanol et une solution de 10 g d'hydroxyde de sodium dans 60 ml d'eau. On chauffe au reflux le mélange réactionnel pendant 4 heures, on le rend légèrement acide par de l'acide chlorhydrique N et ensuite on le rend basique par de l'hydroxyde d'ammonium concentré. On concentré le mélange réactionnel sous pression réduite, on reprend le résidu par de l'éthanol absolu et on concentre à sec en répétant l'opération deux fois. On reprend le résidu par de l'éthanol. On filtre le chlorure de sodium et on le lave à l'éthanol pour récupérer tout le produit. On rend la solution basique par de l'hydroxyde d'ammonium concentré et ensuite on évapore à sec. On chromatographie le résidu sur une colonne de gel de silice en éluant par un mélange chlorure de méthylène/éthanol en rapport 9/1 v/v. On récupère la tache du produit et, après évaporation à sec, on obtient 1 g de (2RS)-N-[(2R)-7-hydroxy-1,2,3,4-tétrahydronapht-2-yl]-2-hydroxy-3-indol-4-yloxypropanamine, solide vitreux. NMR (DMSO-d6): 7,1 (1H, dd, J1≃J2=2Hz, CH alpha), 10,9 (OH + NH)$\delta$

EXEMPLE 9.

On chauffe au reflux pendant 5 heures une solution de 2,6 g de 2-amino-6-hydroxytétraline base et de 3,2 g de 1-naphtyloxy-2,3-époxypropane dans 80 ml d'éthanol. On laisse refroidir le mélange reactionnel jusqu'à la température ambiante. On évapore le solvant sous pression réduite et on soumet l'huile ainsi obtenue à une chromatographie sur une colonne de silice (MERCK 70-230 mesh) en éluant d'abord par de l'acétate d'éthyle, jusqu'à la complète élution de la première tache, et ensuite par un mélange acétate d'éthyle/méthanol 9/1 v/v jusqu'à la complète élution des fractions du produit. On réunit les fractions ainsi obtenues et on les concentre sous pression réduite. On dissout le résidu dans 20 ml d'isopropanol et on ajoute une solution de 0,95 g d'acide fumarique dans 10 ml d'isopropanol. On filtre, on lave à l'éther éthylique et on dissout le résidu dans de l'eau. Après addition d'hydroxyde de sodium concentré jusqu'à réaction basique, on extrait par de l'acétate d'éthyle. On sèche la phase organique sur du sulfate de sodium, on évapore à sec et on reprend le résidu avec une très petite quantité d'acétate d'éthyle. On filtre et l'on obtient 1,9 g de N-(6-hydroxy-1,2,3,4-tétrahydronapht-2-yl)-2-hydroxy-3-(1-naphtyloxy)-propanamine; p.f. 118-120°C.

EXEMPLE 10.

On chauffe au reflux pendant 5 heures une solution de 2,45 g de 2-amino-7-hydroxytétraline base et de 2,5 g de 1-(2-méthylphénoxy)-2,3-époxypropane dans 75 ml d'éthanol. Après évaporation de l'éthanol, on reprend le résidu par 20 ml d'acétone et on rend acide la solution par de l'acide chlorhydrique dans l'isopropanol. On filtre, on lave à l'èther éthylique et on recristallise le produit ainsi obtenu dans 30 ml d'éthanol à 95%. On obtient 1,7 g de chlorhydrate de N-(7-hydroxy-1,2,3,4-tétrahydronapht-2-yl)-2-hydroxy-3-(2-méthylphénoxy)propanamine; p.f. 224-226°C.

EXEMPLE 11.

En opérant comme décrit dans l'Exemple 2, en remplaçant le 1-naphtyloxy-2,3-époxypropane par le 1-(2-méthylphénoxy)-2,3-époxypropane, on obtient un produit qui, après cristallisation dans de l'éthanol absolu, donne le chlorhydrate de N-(1,2,3,4-tétrahydronapht-2-yl)-2-hydroxy-3-(2-méthylphénoxy)-propanamine; p.f. 180-182°C.

EXEMPLE 12.

On chauffe au reflux pendant 5 heures une solution de 2,95 g de 1-aminotétraline et 3,3 g de 1-(2-méthylphénoxy)-2,3-époxypropane dans 80 ml d'éthanol. Après évaporation de l'éthanol, on chromatographie sur une colonne de gel de silice en éluant par un mélange acétate d'éthyle/cyclohéxane 7/3 v/v. On évapore à sec les fractions réunies et on reprend le résidu dans 40 ml d'isopropanol. On rend la solution acide par de l'acide chlorhydrique dans l'isopropanol et, après addition de 40 ml d'éther isopropylique, on laisse le mélange réactionnel pendant 2 heures à 0-5°C. On filtre et, après cristallisation dans 20 ml d'isopropanol, on obtient 2,5 g de chlorhydrate de N-(1,2,3,4-tétrahydronapht-1-yl)-2-hydroxy-3-(2-méthylphénoxy)propanamine; p.f. 123-124°C.

EXEMPLE 13.

(a) On chauffe au reflux pendant 3 heures une solution de 3,9 g de 1-[1-(p-toluènesulfonyl)indol-4-yloxy]-2,3-époxypropane et 2,2 g de 2-amino-7-méthoxytétraline dans 40 ml d'isopropanol. Après évaporation à sec, on reprend le résidu par de l'acétate d'éthyle, on lave la solution ainsi obtenue à l'eau, on la sèche sur du sulfate de sodium et on l'évapore à sec. On reprend le résidu par de l'acétate d'éthyle et de l'isopropanol et on rend la solution acide par de l'acide chlorhydrique dans l'isopropanol. Après précipitation, on filtre et l'on suspend le produit ainsi obtenu dans l'éthanol à chaud. On refroidit, on filtre et l'on essore. On obtient 3,1 g de chlorhydrate de N-(7-méthoxy-1,2,3,4-tétrahydronapht-2-yl)-2-hydroxy-3-[1-(p-toluènesulfonyl)indol-4-yloxy]propanamine; p.f. 228-231°C.

(b) On chauffe au reflux pendant 4 heures 12,5 g de N-(7-méthoxy-1,2,3,4-tétrahydronapht-2-yl)-2-hydroxy-3-/1-(p-toluènesulfonyl) indol-4-yloxy/propanamine (a) dans une solution de 5,4 g d'hydroxyde de sodium dans 34 ml d'eau et 76 ml d'éthanol à 95%. On refroidit le mélange réactionnel jusqu'à la température ambiante et l'on ajoute 400 ml d'acétate d'éthyle. Après séparation des deux phases ainsi obtenues, on lave la phase organique à l'eau, on la sèche sur du sulfate de sodium et on l'évapore à sec. On chromatographie le résidu sur une colonne de gel de silice (MERCK 70-230 mesh) en éluant par un mélange éthyle acétate/méthanol 95/5 v/v. On recupère la tache pure et, après évaporation du solvant, on obtient 6 g de N-(7-méthoxy-1,2,3,4-tétrahydronapht-2-yl)-2-hydroxy-3-(indol-4-yloxy)propanamine; solide amorphe. NMR (DMSO-d6): 3,68 (3H, s, $OCH_3$), 7,2 (1H, dd, J1≃J2=2Hz, CH alpha)δ

EXEMPLE 14.

On chauffe au reflux pendant 5 heures un mélange de 2,94 g de 1-aminotétraline et 3,6 g de 1-(2-méthoxy)phénoxy-2,3-époxypropane dans 100 ml d'éthanol absolu. Après évaporation du solvant sous pression réduite, on chromatographie le résidu sur une colonne de silice en éluant par de l'acétate d'éthyle. On évapore à sec les fractions réunies ainsi obtenues et on reprend le résidu par un mélange hexane/éther isopropylique 1/1 v/v. Après filtration et cristallisation dans de l'éthanol acétate, on obtient 2 g de N-(1,2,3,4-tétrahydronapht-1-yl)-2-hydroxy-3-(2-méthoxyphénoxy)propanamine; p.f. 99-102°C.

EXEMPLE 15.

On chauffe au reflux pendant 5 heures une solution de 2,2 g de 2-aminotétraline base et 2,7 g de 1-(2-méthoxy)phénoxy-2,3-époxypropane dans 70 ml d'éthanol. On évapore le solvant sous pression réduite, on reprend le résidu par de l'isopropanol et l'on acidifie, à chaud et sous agitation, par de l'acide chlorhydrique dans de l'isopropanol. Après filtration du précipité, on obtient 2,1 g de chlorhydrate de N-(1,2,3,4-tétrahydronapht-2-yl)-2-hydroxy-3-(2-méthoxyphénoxy)propanamine; p.f. 140-143°C.

EXEMPLE 16.

On chauffe au reflux pendant 5 heures un mélange de 0,9 g de 2-amino-6-hydroxytétraline et 1 g de 1-(2-méthoxy)phénoxy-2,3-époxypropane dans 30 ml d'éthanol absolu. Après évaporation du solvant sous pression réduite, on chromatographie le résidu sur une colonne de gel de silice en éluant par de l'acétate d'éthyle et ensuite par un mélange éthyle acétate/méthanol 9/1 v/v. Après évaporation à sec, on dissout le résidu dans de l'acétone et on acidifie par de l'acide chlorhydrique dans de l'isopropanol, à chaud et sous agitation. Après filtration du précipité, on obtient 0,25 g de chlorhydrate de N-(6-hydroxy-1,2,3,4-tétrahydronapht-2-yl)-2-hydroxy-3-(2-méthoxyphénoxy)propanamine; p.f. 146-148°C.

EXEMPLE 17.

On chauffe au reflux pendant 5 heures un mélange de 3,15 g de 2-amino-7-hydroxytétraline et 3,45 g de 1-(2-méthoxy)phénoxy-2,3-époxypropane dans 100 ml d'éthanol absolu. Après évaporation du solvant sous pression réduite, on chromatographie le résidu sur une colonne de gel de silice en éluant par de l'acétate d'éthyle et ensuite par un mélange acétate d'éthyle/méthanol 85/15 v/v. Après évaporation à sec des fractions réunies, on dissout le résidu huileux dans de l'éthanol absolu et on acidifie par de l'acide chlorhydrique dans de l'isopropanol. Après filtration du précipité, on obtient 2 g de chlorhydrate de N-(7-hydroxy-1,2,3,4-tétrahydronapht-2-yl)-2-hydroxy-3-(2-méthoxyphénoxy)propanamine; p.f. 173-176°C.

EXEMPLE 18.

On chauffe au reflux pendant 5 heures un mélange de 5,15 g de 1-aminotétraline et 7,22 g de 1-(2-allyloxy)phénoxy-2,3-époxypropane dans 80 ml d'éthanol absolu. Après évaporation du solvant sous pression réduite, on chromatographie le résidu sur une colonne de gel de silice en éluant par un mélange acétate d'éthyle/cyclohexane 7/3 v/v. Après évaporation à sec sous pression réduite des fractions réunies, on reprend le résidu par une quantité minimale d'éther éthylique. On filtre et on cristallise dans de l'éther isopropylique. On obtient ainsi 2,1 g de N-(1,2,3,4-tétrahydronapht-1-yl)-2-hydroxy-3-(2-allyloxyphénoxy)propanamine; p.f. 72-75°C.

EXEMPLE 19.

On chauffe au reflux pendant 5 heures un mélange de 2,2 g de 2-aminotétraline et 3,1 g de 1-(2-allyloxy)phénoxy-2,3-époxypropane dans 80 ml d'éthanol absolu. Après évaporation du solvant, on chromatographie le résidu sur une colonne de gel de silice en éluant d'abord par de l'acétate d'éthyle et ensuite par un mélange acétate d'éthyle/méthanol 9/1 v/v. On évapore à sec les fractions réunies, on reprend le résidu par de l'éther éthylique et on filtre. On obtient 2,6 g de N-(1,2,3,4-tétrahydronapht-2-yl)-2-hydroxy-3-(2-allyloxyphénoxy)propanamine; p.f. 98-100°C.

EXEMPLE 20.

On chauffe au reflux pendant 5 heures un mélange de 3,4 g de 2-amino-7-hydroxytétraline dans 80 ml d'éthanol absolu et 4,12 g de 1-(2-allyloxy)phénoxy-2,3-époxypropane dans 50 ml d'éthanol absolu. Après évaporation à sec, on chromatographie sur une colonne de gel de silice en éluant d'abord par de l'acétate d'éthyle et ensuite par un mélange acétate d'éthyle/méthanol 9/1 v/v. On évapore à sec les fractions réunies, on dissout l'huile ainsi obtenue dans de l'isopropanol et on acidifie par de l'acide chlorhydrique dans de l'isopropanol. Après filtraton du précipité et cristallisation dans de l'éthanol absolu, on obtient 3,1 g de chlorhydrate de N-(7-hydroxy-1,2,3,4-tétrahydronapht-2-yl)-2-hydroxy-3-(2-allyloxyphénoxy)propanamine; p.f. 183-185°C.

EXEMPLE 21.

On chauffe au reflux pendant 5 heures un mélange de 2,6 g de 2-amino-6-hydroxytétraline et 3,3 g de 1-(2-allyloxy)phénoxy-2,3-époxypropane dans 80 ml d'éthanol absolu. Après évaporation du solvant sous pression réduite, on chromatographie sur une colonne de gel de silice en éluant par de l'acétate d'éthyle et ensuite par un mélange acétate d'éthyle/méthanol 9/1 v/v. On évapore à sec les fractions réunies et on dissout le résidu dans de l'isopropanol. On acidifie par de l'acide chlorhydrique dans de l'isopropanol et, après filtraton du précipité, on cristallise dans de l'isopropanol. On obtient 2,1 g de chlorhydrate de N-(6-hydroxy-1,2,3,4-tétrahydronapht-2-yl)-2-hydroxy-3-(2-allyloxyphénoxy)propanamine; p.f. 145-147°C.

EXEMPLE 22.

On chauffe au reflux pendant 6 heures 12 g de 1-[1-(p-toluènesulfonyl)indol-4-yloxy]-2,3-époxypropane et 5,15 g de 1-aminotétraline dans 150 ml d'éthanol absolu. On évapore le solvant et l'on dissout l'huile ainsi obtenue dans 100 ml d'éthanol absolu. On ajoute une solution de 7,5 g d'hydroxyde de sodium dans 30 ml d'eau et on chauffe au reflux le mélange réactionnel pendant 6 heures. Après évaporation sous pression réduite, on dissout le résidu dans 150 ml d'acétate d'éthyle. On lave à l'eau à fond et on sèche sur du sulfate de sodium. Après évaporation de l'acétate d'éthyle, on obtient un résidu huileux qui est chromatographié sur une colonne de gel de silice en éluant par un mélange acétate d'éthyle/cyclohexane 7/3 v/v. On évapore à sec les

fractions réunies, on reprend l'huile ainsi obtenue par une quantité minimale d'éther éthylique et l'on élimine l'éther à la pompe mécanique; on obtient 1,5 g de N-(1,2,3,4-tétrahydronapht-1-yl)-2-hydroxy-3-(4-indolyloxy)propanamine, solide vitreux.
IR (KBr): 3404, 2926, 1361, 1244, 1092, 740 cm-1

EXEMPLE 23.

On chauffe au reflux pendant 5 heures 3,26 g de 2-amino-6-hydroxytétraline et 6,8 g de 1-[1-(p-toluènesulfonyl)indol-4-yloxy]-2,3-époxypropane dans 80 ml d'éthanol absolu. On évapore le solvant et on suspend le résidu dans 185 ml d'éthanol absolu. On ajoute une solution de 4,8 g d'hydroxyde de sodium dans 55 ml d'eau et on chauffe au reflux pendant 6 heures. Après évaporation sous pression réduite, on dissout le résidu dans 200 ml d'acétate d'éthyle et on lave deux fois à l'eau. On sèche la phase organique sur du sulfate de sodium et on l'évapore sous pression réduite. On chromatographie le résidu sur une colonne de gel de silice en éluant par de l'acétate d'éthyle et ensuite par un mélange acétate d'éthyle/méthanol 85/15 v/v. On évapore à sec les fractions réunies, on reprend le résidu par de l'éther éthylique et l'on filtre. On obtient 2,4 g de N-(6-hydroxy-1,2,3,4-tétrahydronapht-2-yl)-2-hydroxy-3-(4-indolyloxy)propanamine; solide vitreux.
NMR (DMSO-d6): 7,15 (1H, dd, J1≃J2=2Hz, CH alpha), 10,9 (OH + NH)δ
IR (KBr): 3408, 2923, 1501, 1240, 742 cm-1

EXEMPLE 24.

On chauffe au reflux pendant 6 heures 5 g de 2-aminotétraline et 11,7 g de 1-[1-(p-toluènesulfonyl)indol-4-yloxy]-2,3-époxypropane dans 150 ml d'éthanol absolu. On évapore à sec, on reprend le résidu à chaud dans 100 ml d'isopropanol et l'on acidifie sous agitation avec de l'acide chlorhydrique dans de l'isopropanol. Après filtration du précipité ainsi obtenu, on le dissout dans un mélange de 250 ml d'éthanol et d'une solution de 7,6 g d'hydroxyde de sodium dans 85 ml d'eau. On chauffe au reflux le mélange réactionnel pendant 6 heures, on l'évapore à sec et on le reprend par 200 ml d'acétate d'éthyle. On lave la solution deux fois à l'eau et on la sèche sur du sulfate de sodium. Après évaporation du solvant, on chromatographie le résidu sur une colonne de gel de silice en éluant par de l'acétate d'éthyle. On évapore à sec les fractions réunies et on dissout le résidu dans 20 ml d'éther éthylique, dans lequel le produit cristallise. Après filtration, on obtient 3,5 g de N-(1,2,3,4-tétrahydronapht-2-yl)-2-hydroxy-3-(4-indolyloxy)propanamine; p.f. 65-68°C.

EXEMPLE 25.

On chauffe au reflux pendant 5 heures une solution de 2,6 g de 2-amino-6-hydroxytétraline et de 3,05 g de 1-(2-allyl)phénoxy-2,3-époxypropane dans 80 ml d'éthanol absolu. On évapore le solvant sous pression réduite et on dissout le résidu dans un mélange isopropanol/éther éthylique 1/1 v/v. On acidifie par de l'acide chlorhydrique dans de l'isopropanol, on filtre le précipité cristallin et l'on obtient 2,8 g de chlorhydrate de N-(6-hydroxy-1,2,3,4-tétrahydronapht-2-yl)-2-hydroxy-3-(2-allylphénoxy)propanamine; p.f. 212-215°C.

EXEMPLE 26.

On chauffe au reflux pendant 5 heures 0,9 g de 2-amino-6-hydroxytétraline et 0,91 g de 1-(2-méthyl)phénoxy-2,3-époxypropane dans 30 ml d'éthanol absolu. On évapore le solvant, on dissout le résidu dans 120 ml d'isopropanol et on acidifie la solution par de l'acide chlorhydrique dans de l'isopropanol. Après filtration du précipité, on cristallise dans de l'éthanol à 95% et l'on obtient 0,55 g de chlorhydrate de N-(6-hydroxy-1,2,3,4-tétrahydronapht-2-yl)-2-hydroxy-3-(2-méthylphénoxy)propanamine; p.f. 232-234°C.

EXEMPLE 27.

A une solution de 2,71 g de 1-(2,3-époxypropoxy)phénothiazine dans 30 ml d'éthanol absolu, on ajoute 1,4 g de 2-aminotétraline et on chauffe 8 heures au reflux le mélange ainsi obtenu. On évapore le solvant sous pression réduite, on reprend le résidu par de l'isopropanol, on chauffe la solution ainsi obtenue et on y ajoute, sous agitation, une solution saturée d'acide chlorhydrique gazeux dans de l'isopropanol. On filtre le précipité et l'on obtient le chlorhydrate de N-(1,2,3,4-tétrahydronapht-2-yl)-2-hydroxy-3-(1-phénothiazinyloxy)propanamine, correspondant à la formule (i), R = H, Ar = radical 2 où Z est S, chaîne en position 2 de la tétraline.

EXEMPLE 28.

En opérant comme décrit dans l'Exemple 27, à partir de 2,46 g de 6-(2,3-époxypropoxy)-1-oxo-1,2,3,4-tétrahydro-bêta-carboline, on obtient le chlorhydrate de N-(1,2,3,4-tétrahydronapht-2-yl)-2-hydroxy-3-(1-oxo-1,2,3,4-tétrahydro-bêta-carboline-6-yloxy)propanamine correspondant à la formule (i), R = H, Ar = radical 3 où Z est CO et Z' est H, chaîne en position 2 de la tétraline.

EXEMPLE 29.

En opérant comme décrit dans l'Exemple 27, à partir de 2,78 g de 1-(2,3-époxypropoxy)-9,10-dihydro-9,10-éthanoanthracène dans 30 ml d'éthanol, on obtient le chlorhydrate de N-(1,2,3,4-tétrahydronapht-2-yl)-2-hydroxy-3-(9,10-dihydro-9,10-éthanoanthr-1-yloxy)propanamine, correspondant à la formule (i), R = H, Ar = radical 5 où Z est éthylène, chaîne en position 2 de la tétraline.

EXEMPLE 30.

En opérant comme décrit dans l'Exemple 27, à partir de 2,18 g de 5-(2,3-époxypropoxy)-coumarine dans 30 ml d'éthanol, on obtient le chlorhydrate de N-(1,2,3,4-tétrahydronapht-2-yl)-2-hydroxy-3-(coumarin-5-yloxy)propanamine, correspondant à la formule (i), R = H, Ar = radical 8 où Z est H, chaîne en position 2 de la tétraline.

EXEMPLE 31.

On chauffe 4 heures à 100°C, dans un flacon scellé, un mélange de 26,9 g de 3-chloro-2-hydroxy-1-(4-méthylcoumarin-7-yloxy)propane, de 35 ml d'éthanol absolu et 22,7 g de 2-aminotétraline. Après refroidissement, on concentre le mélange sous pression réduite, on reprend le résidu par 200 ml d'acide chlorhydrique N et l'on purifie par extraction avec du chloroforme. On sépare la phase aqueuse et on la neutralise avec du carbonate de sodium. On évapore l'eau, on reprend le résidu par de l'isobutylméthylcétone. On filtre et l'on traite la solution avec de l'acide chlorhydrique gazeux jusqu'à précipitation complète. Par cristallisation du précipité dans l'isopropanol on obtient le chlorhydrate de N-(1,2,3,4-tétrahydronapht-2-yl)-2-hydroxy-3-(4-méthylcoumarin-7-yloxy)propanamine, correspondant à la formule (i), R = H, Ar = radical 8 où Z est méthyle, chaîne en position 2 de la tétraline.

EXEMPLE 32.

On chauffe 90 minutes à 110°C un mélange de 5,22 g de 5-hydroxy-3-méthyl-1-phénylpyrazole (ou 3-méthyl-1-phényl-1,2-pyrazolin-5-one), 16,6 g d'épichlorhydrine et deux gouttes de pipéridine, puis on concentre le mélange réactionnel sous pression réduite et on reprend le résidu en agitant 30 minutes avec 45 ml d'hydroxyde de sodium à 33%. On extrait avec du chloroforme, on lave à l'eau, on sèche la phase organique et l'on évapore le solvant sous pression réduite. On dissout le résidu dans du chloroforme, on passe la solution sur une colonne de gel de silice et l'on évapore le solvant. On obtient ainsi 2,5 g de 5-(2,3-époxypropoxy)-3-méthyl-1-phénylpyrazole qui est dissout dans 30 ml d'éthanol absolu et traité avec 1,61 g de 2-aminotétraline. En opérant comme décrit dans l'Exemple 27, on obtient le dichlorhydrate de N-(1,2,3,4-tétrahydronapht-2-yl)-2-hydroxy-3-(3-méthyl-1-phénylpyrazol-5-yloxy)propanamine, correspondant à la formule (i), R = H, Ar = radical 9 où Z est méthyle et Z' est phényle, chaîne en position 2 de la tétraline.

EXEMPLE 33.

On agite 3 heures à la température ambiante et sous azote un mélange de 12,6 g de 3-hydroxy-2-méthyl-4-oxopyranne, 4 g d'hydroxyde de sodium, 9,25 g d'épichlorhydrine et 50 ml d'eau, puis on extrait 4 fois avec 120 ml de chloroforme. On lave la phase organique à l'eau, on la sèche et on l'évapore à sec On obtient ainsi 8,3 g de 3-époxypropoxy-2-méthyl-4-oxopyranne. Un mélange du produit ainsi isolé et 7,3 g de 2-aminotétraline dans 100 ml d'éthanol absolu est chauffé à 80°C pendant 5 heures dans un recipient fermé. Après refroidissement, on évapore sous pression réduite, on dissout le résidu dans de l'acétate d'éthyle et on ajoute une solution d'acide fumarique dans l'acétate d'éthyle. On cristallise le précipité dans l'éthanol et on obtient l'hydrogénofumarate de N-(1,2,3,4-tétrahydronapht-2-yl)-2-hydroxy-3-(2-méthyl-4- oxopyrann-3-yloxy)propanamine, correspondant à la formule (i), R = H, Ar = radical 10 où Z est méthyle, chaîne en position 2 de la tétraline.

EXEMPLE 34.

En opérant comme décrit dans l'Exemple 27, à partir de 2 g de 5-(2,3-époxypropoxy)-3,4-dihydrocarbostyrile et de 1,35 g de 2-aminotétraline dans 40 ml d'éthanol, on obtient le chlorhydrate de N-(1,2,3,4-tétrahydronapht-2-yl)-2-hydroxy-3-(3,4-dihydrocarbostyryl-5-oxy)propanamine, correspondant à la formule (i), R = H, Ar = radical 11 où Z est H et la chaîne est en position 4 de Ar et en position 2 de la tétraline.

EXEMPLE 35.

On chauffe 1 heure à l'ébullition sous azote un mélange de 10 g de 4-hydroxy-9-oxofluorène et 1,9 g d'hydroxyde de sodium, dans 100 ml d'eau. On laisse refroidir à 50°C, on ajoute 7 g d'épichlorhydrine et on laisse le mélange reactionnel 16 heures sous agitation à 50°C. On ajoute 40 ml d'acétone, on extrait trois fois avec de l'acétate d'éthyle, on lave à l'eau les extraits réunis, on les sèche sur du sulfate de magnésium et on les évapore à sec sous pression réduite. On reprend le résidu (13,5 g) dans 150 ml d'éthanol et on ajoute 8 g de 2-aminotétraline. On chauffe le mélange réactionnel 8 heures au reflux, puis l'on opère comme décrit dans l'Exemple 27. On obtient ainsi le chlorhydrate de N-(1,2,3,4-tétrahydronapht-2-yl)-2-hydroxy-3-(9-oxofluoren-4-yloxy)propanamine, correspondant à la formule (i), R = H, Ar = radical 13 où Z est >CO, chaîne en position 2 de la tétraline.

EXEMPLE 36.

En opérant comme décrit dans l'Exemple 27, à partir de 10 g de 2,3-diméthyl-4-(2,3-époxypropoxy)indole et 6,9 g de 2-aminotétraline dans 100 ml d'éthanol, on obtient le chlorhydrate de N-(1,2,3,4-tétrahydronapht-2-yl)-2-hydroxy-3-(2,3-diméthylindol-4-yloxy)propanamine, correspondant à la formule (i), R = H, Ar = radical 14 où Z est méthyle, chaîne en position 2 de la tétraline.

EXEMPLE 37.

En opérant comme décrit dans l'Exemple 27, à partir de 25,1 g de 4-(2,3-époxypropoxy)indole-2-carboxylate d'éthyle, obtenu du 4-hydroxyindole-2-carboxylate d'éthyle et de l'épichlorhydrine selon BE 739.545, et de 14,8 g de 2-aminotétraline dans 120 ml d'éthanol, on obtient le chlorhydrate de N-(1,2,3,4-tétrahydronapht-2-yl)-2-hydroxy-3-(2-éthoxycarbonylindol-4-yloxy)propanamine, cristallisé d'un mélange éthanol/isopropanol 2/1 et correspondant à la formule (i), R = H, Ar = radical 15 où Z est éthoxy, chaîne en position 2 de la tétraline.

EXEMPLE 38.

En opérant comme décrit dans l'Exemple 27, à partir de 27,5 g de 4-(2,3-époxypropoxy)indole-2-acétate d'éthyle, obtenu du 4-hydroxyindole-2-acétate d'éthyle et de l'épichlorhydrine selon CH 526 542, et de 14,8 g de 2-aminotétraline dans 120 ml d'éthanol, on obtient le chlorhydrate de N-(1,2,3,4-tétrahydronapht-2-yl)-2-hydroxy-3-(2-éthoxycarbonylméthylindol-4-yloxy)propanamine, cristallisé d'un mélange éthanol/isopropanol 2/1 et correspondant à la formule (i), R = H, Ar = radical 16 où Z est éthoxy, chaîne en position 2 de la tétraline.

EXEMPLE 39.

En opérant comme décrit dans l'Exemple 27, à partir de 10 g de 2-éthyl-4-(2,3-époxypropoxy)indole, obtenu de 2-éthyl-4-hydroxyindole et de l'épichlorhydrine selon CH 527 188, et de 7,95 g de 2-aminotétraline dans 90 ml d'éthanol, on obtient le chlorhydrate de N-(1,2,3,4-tétrahydronapht-2-yl)-2-hydroxy-3-(2-éthylindol-4-yloxy)propanamine, correspondant à la formule (i), R = H, Ar = radical 17 où Z est éthyle, chaîne en position 2 de la tétraline.

EXEMPLE 40.

En opérant comme décrit dans l'Exemple 27, à partir de 20,5 g de 4-(2,3-époxypropoxy)-2-oxoindoline et 14,81 g de 2-aminotétraline dans 150 ml d'éthanol, on obtient le chlorhydrate de N-(1,2,3,4-tétrahydronapht-2-yl)-2-hydroxy-3-(2-oxo-2, 3-dihydroindol-4-yloxy)propanamine, correspondant à la formule (i), R = H, Ar = radical 18 où Z est H, chaîne en position 2 de la tétraline.

EXEMPLE 41.

En opérant comme décrit dans l'Exemple 27, à partir de 22,2 g de 8-(2,3-époxypropoxy)thiochromanne, obtenu du 8-hydroxythiochromanne et de l'épichlorhydrine selon BE 765.313, et de 14,82 g de 2-aminotétraline, on obtient le chlorhydrate de N-(1,2,3,4-tétrahydronapht-2-yl)-2-hydroxy-3-(thiochromann-8-yloxy)propanamine, correspondant à la formule (i), R = H, Ar = radical 19 où Z est H et la chaîne est en position 8 de Ar et en position 2 de la tétraline.

EXEMPLE 42.

En opérant comme décrit dans l'Exemple 27, à partir de 19 g de 7-(2,3-époxypropoxy)-3-méthylindène , obtenu du 3-méthyl-7-indénol et de l'épichlorhydrine selon JP 73-01070, et 14,83 g de 2-amino-tétraline, on obtient le chlorhydrate de N-(1,2,3,4-tétrahydronapht-2-yl)-2-hydroxy-3-(3-méthylindén-7-yloxy)propanamine, correspondant à la formule (i), R = H, Ar = radical 23 où Z est H et Z' est méthyle, chaîne en position 2 de la tétraline.

EXEMPLE 43.

En opérant comme décrit dans l'Exemple 27, à partir de 23,2 g de 2-acétyl-7-(2,3-époxypropoxy)benzofuranne, obtenu du 2-acétyl-7-hydroxybenzofuranne et de l'épichlorhydrine selon BE 783.440, et de 14,81 g de 2-aminotétraline dans 120 ml d'éthanol, on obtient le chlorhydrate de N-(1,2,3,4-tétrahydronapht-2-yl)-2-hydroxy-3-(2-acétylbenzofuran-7-yloxy)propanamine, correspondant à la formule (i), R = H, Ar = radical 24 où Z est acétyle et la chaîne est en position 4 de Ar et en position 2 de la tétraline.

EXEMPLE 44.

Dans les mêmes conditions que celles de l'Exemple 43, à partir du 2-acétyl-4-(2,3-époxypropoxy)benzofuranne, on obtient le chlorhydrate de N-(1,2,3,4-tétrahydronapht-2-yl)-2-hydroxy-3-(2-acétylbenzofuran-4-yloxy)propanamine, correspondant à la formule (iF), L" = H, E" et G", ensemble, forment un groupe -CH=C-(COCH3)-O- et R est H, chaîne en position 2 de la tétraline.

EXEMPLE 45.

En opérant comme décrit dans l'Exemple 27, à partir de 27 g de 4-(4,5,6,7-tétrahydrobenzofuran-2-yl)-1-époxypropoxybenzène, obtenu selon US 3,894,058, et de 14,85 g de 2-aminotétraline dans 150 ml d'éthanol, on obtient le chlorhydrate de N-(1,2,3,4-tétrahydronapht-2-yl)-2-hydroxy-3-[4-(4,5,6,7-tétrahydrobenzofuran-2-yl)] phénoxypropanamine, correspondant à la formule (i), R = H, Ar = radical 31 où Z est H, chaîne en position 2 de la tétraline.

EXEMPLE 46.

En opérant comme décrit dans l'Exemple 27, à partir de 20,8 g de 4-(2,3-époxypropoxy)-2,1,3-benzothiadiazole, obtenu selon DE 2 404 858, et 14,82 g de 2-aminotétraline dans 125 ml d'éthanol absolu, on obtient le chlorhydrate de N-(1,2,3,4-tétrahydronapht-2-yl)-2-hydroxy-3-(2,1,3-benzothiadiazol-4-yloxy)propanamine, correspondant à la formule (i), R = H, Ar = radical 32, chaîne en position 2 de la tétraline.

EXEMPLE 47.

En opérant comme décrit dans l'Exemple 27, à partir de 20,6 g de 7-(2,3-époxypropoxy)phtalide, obtenu à partir du 7-hydroxyphtalide et de l'épichlorhydrine selon BE 815.745, et de 14,81 g de 2-aminotétraline dans 125 ml d'éthanol absolu, on obtient le chlorhydrate de N-(1,2,3,4-tétrahydronapht-2-yl)-2-hydroxy-3-phtalid-7-yloxypropanamine, correspondant à la formule (iF), L" = H, E" et G" forment, ensemble, un groupe -CO-O-$CH_2$- et R est H, chaîne en position 2 de la tétraline.

EXEMPLE 48.

En opérant comme décrit dans l'Exemple 27, à partir de 28,8 g de 2,3-époxypropyléther du khelline-quinol et de 14,83 g de 2-aminotétraline dans 150 ml d'éthanol absolu, on obtient le chlorhydrate du 3-(1,2,3,4-tétrahydronapht-2-ylamino)-2-hydroxypropyléther de khelline-quinol, correspondant à la formule (i), R = H, Ar = radical 34, chaîne en position 2 de la tétraline.

EXEMPLE 49.

En opérant comme décrit dans l'Exemple 27, à partir de 18,8 g de 4-(2,3-époxypropoxy)-1-oxoindane, (JP 49-048649) et de 14,81 g de 2-aminotétraline dans 120 ml d'éthanol absolu, on obtient le chlorhydrate de N-(1,2,3,4-tétrahydronapht-2-yl)-2-hydroxy-3-(1-oxoindan-4-yloxy)propanamine, correspondant à la formule (i), R = H, Ar = radical 35, chaîne en position 2 de la tétraline.

EXEMPLE 50.

En opérant comme décrit dans l'Exemple 27, à partir de 20,3 g de 4-(2,3-époxypropoxy)-2-oxoindoline (JP 49-094666) et de 14,81 g de 2-aminotétraline dans 100 ml d'éthanol, on obtient le chlorhydrate de N-(1,2,3,4-tétrahydronapht-2-yl)-2-hydroxy-3-(2-oxoindolin-4-yloxy)propanamine, correspondant à la formule (i), R = H, Ar = radical 36, chaîne en position 2 de la tétraline.

EXEMPLE 51.

En opérant dans les mêmes conditions que celles de l'Exemple 35, à partir du 1-hydroxy-9-oxofluorène, on obtient le chlorhydrate de N-(1,2,3,4-tétrahydronapht-2-yl)-2-hydroxy-3-(9-oxofluoren-1-yloxy)propanamine, correspondant à la formule (i), R = H, Ar = radical 37, chaîne en position 2 de la tétraline.

EXEMPLE 52.

On agite pendant 24 heures à la température ambiante un mélange de 8,3 g de 4-hdyroxy-2-méthoxyméthylindole, de 8,9 g d'épichlorhydrine et d'une solution de 1,92 g d'hydroxyde de sodium dans 35 ml d'eau et 35 ml de dioxanne, puis on extrait avec du chlorure de méthylène, on sèche et l'on évapore le solvant. On reprend le résidu (10,2 g) avec 80 ml d'éthanol absolu et on ajoute au mélange 6,49 g de 2-aminotétraline. En opérant ensuite comme décrit dans l'Exemple 27, on obtient le chlorhydrate de N-(1,2,3,4-tétrahydronapht-2-yl)-2-hydroxy-3-(2-méthoxyméthylindol-4-yloxy)propanamine, correspondant à la formule (i), R = H, Ar = radical 38, chaîne en position 2 de la tétraline.

EXEMPLE 53.

En opérant comme décrit dans l'Exemple 27, à partir de 27,4 g de 2,2,5,7,8-pentaméthyl-6-(2,3-époxypropoxy)chromanne, obtenu selon NL 69/011816, et de 14,85 g de 2-aminotétraline, on obtient le chlorhydrate de N-(1,2,3,4-tétrahydronapht-2-yl)-2-hydroxy-3-(2,2,5,7,8-pentaméthylchroman-6-yloxy)propanamine, correspondant à la formule (i), R = H, Ar = radical 41, chaîne en position 2 de la tétraline.

EXEMPLE 54.

En opérant comme décrit dans l'Exemple 27, à partir de 20,2 g de 5(2,3-époxypropoxy)-1-oxotétraline, obtenue selon BE 739 195, et de 14,81 g de 2-aminotétraline dans 130 ml d'éthanol absolu, on obtient le chlorhydrate de N-(1,2,3,4-tétrahydronapht-2-yl)-2-hydroxy-3-(1-oxo-1,2,3,4-tétrahydronapht-5-yloxy)propanamine, correspondant à la formule (iA), où E et G forment, ensemble, un groupe $-CH_2-CH_2-CH_2-CO-$ et R est H, chaîne en position 2 de la tétraline.

EXEMPLE 55.

En opérant comme décrit dans l'Exemple 27, à partir de 18,8 g de 7-(2,3-époxypropoxy)indène, obtenu du 7-hydroxyindène et de l'épichlorhydrine selon DE 1 955 229, et de 14,85 g de 2-aminotétraline dans 120 ml d'éthanol absolu, on obtient le chlorhydrate de N-(1,2,3,4-tétrahydronapht-2-yl)-2-hydroxy-3-inden-7-yloxypropanamine (correspondant à la formule (i), R = H, Ar = radical 43, chaîne en position 2 de la tétraline.

EXEMPLE 56.

En opérant comme décrit dans l'Exemple 27, à partir de 20,4 g de 8-(2,3-époxypropoxy)-2H-chromène et

de 14,85 g de 2-aminotétraline dans 120 ml d'éthanol absolu, on obtient le chlorhydrate de N-(1,2,3,4-tétrahydronapht-2-yl)-2-hydroxy-3-(2H-chromen-8-yloxy)propanamine, correspondant à la formule (i), R = H, Ar = radical 44, chaîne en position 2 de la tétraline.

EXEMPLE 57.

On agite pendant 24 heures à la température ambiante un mélange de 1,4 g de 1,4-dihydroxyindane, 1 g d'épichlorhydrine et 0,4 g d'hydroxyde de sodium dans 15 ml d'eau, puis on extrait avec de l'éther éthylique. On sèche la phase organique, on l'évapore à sec et on reprend le résidu (1,2 g) avec 20 ml d'éthanol absolu. On ajoute à la solution 0,87 g de 2-aminotétraline dans 10 ml d'éthanol et l'on opère ensuite comme décrit dans l'Exemple 27. On obtient ainsi le chlorhydrate de N-(1,2,3,4-tétrahydronapht-2-yl)-2-hydroxy-3-(1-hydroxyndan-4-yloxy)propanamine, correspondant à la formule (i), R = H, Ar = radical 45, chaîne en position 2 de la tétraline.

EXEMPLE 58.

En opérant comme décrit dans l'Exemple 27, à partir de 19 g de 4-(2,3-époxypropoxy)indane et de 14,81 g de 2-aminotétraline dans 150 ml d'éthanol absolu, on obtient le chlorhydrate de N-(1,2,3,4-tétrahydronapht-2-yl)-2-hydroxy-3-indan-4-yloxypropanamine, correspondant à la formule (iA), où E et G forment, ensemble, un groupe $-CH_2-CH_2-CH_2-$ et R est H, chaîne en position 2 de la tétraline.

EXEMPLE 59.

En opérant comme décrit dans l'Exemple 27, à partir de 20,7 g de 4-(2,3-époxypropoxy)-1,2-benzisothiazole, obtenu comme décrit dans BE 863 622, et de 14,82 g de 2-aminotétraline dans 150 ml d'éthanol absolu, on obtient le chlorhydrate de N-(1,2,3,4-tétrahydronapht-2-yl)-2-hydroxy-3-(1,2-benzisothiazol-4-yloxy)propanamine, correspondant à la formule (i), R = H, Ar = radical 58, chaîne en position 2 de la tétraline.

EXEMPLE 60.

En opérant comme décrit dans l'Exemple 27, à partir de 20,5 g de 4-(2,3-époxypropoxy)-3-méthyl-1,2-benzisoxazole (DE 2 711 382) et de 14,82 g de 2-aminotétraline dans 150 ml d'éthanol absolu, on obtient le chlorhydrate de N-(1,2,3,4-tétrahydronapht-2-yl)-2-hydroxy-3-(3-méthyl-1,2-benzisoxazol-4-yloxy)propanamine, correspondant à la formule (i), R = H, Ar = radical 59, chaîne en position 2 de la tétraline.

EXEMPLE 61.

On ajoute une solution de 1,175 g d'hydroxyde de sodium dans 16 ml d'eau à une solution de 4 g de 4-hydroxy-2-benzimidazolinone dans 24 ml de méthanol, puis on traite ce mélange avec 4,16 g d'épichlorhydrine. On agite le mélange 17 heures à la température ambiante, puis on évapore le solvant et on extrait le résidu avec de l'acétate d'éthyle. On lave à l'eau, on sèche sur du sulfate de magnésium et on évapore à sec. On reprend le résidu avec 30 ml d'éthanol et on ajoute à la solution 4,35 g de 2-aminotétraline dissoute dans 20 ml d'éthanol. En opérant comme décrit dans l'Exemple 27, on obtient ainsi le chlorhydrate de N-(1,2,3,4-tétrahydronapht-2-yl)-2-hydroxy-3-(2-oxobenzimidazolin-4-yloxy)propanamine, correspondant à la formule (i), R = H, Ar = radical 61, où Z et Z' sont H, chaîne en position 2 de la tétraline.

EXEMPLE 62.

En opérant comme décrit dans l'Exemple 27, à partir de 18,1 g de 3-(2,3-époxypropoxy)-2-cyanothiophène, obtenu selon DE 2 720 613 et de 14,8 g de 2-aminotétraline dans 100 ml d'éthanol, on obtient le chlorhydrate de N-(1,2,3,4-tétrahydronapht-2-yl)-2-hydroxy-3-(2-cyanothién-3-yloxy)propanamine, correspondant à la formule (i), R = H, Ar = radical 62 où Z est H, CN est en position 2 et la chaîne est en position 5 de Ar et en position 2 de la tétraline.

EXEMPLE 63.

En opérant comme décrit dans l'Exemple 27, à partir de 2,94 g de 4-(4-éthoxycarboyl-1,2,3-thiadiazol-5-yl)-1-(2,3-époxypropoxy)benzène et de 1,49 g de 2-aminotétraline dans 20 ml d'éthanol absolu, on obtient le

chlorhydrate de N-(1,2,3,4-tétrahydronapht-2-yl)-2-hydroxy-3-/4-(4-éthoxycarbonyl-1,2,3-thiadiazol- 5-yl)phénoxy/propanamine, correspondant à la formule (i), R = H, Ar = radical 63, où Z est 4-$COOC_2H_5$, chaîne en position 2 de la tétraline.

EXEMPLE 64.

En opérant comme décrit dans l'Exemple 27, à partir de 2,81 g de 2-[3-(2,3-époxypropoxy)phényl]-4-trifluorométhylimidazole, obtenu selon DE 2 608 448, et de 1,5 g de 2-aminotétraline dans 20 ml d'éthanol absolu, on obtient le chlorhydrate de N-(1,2,3,4-tétrahydronapht-2-yl)-2-hydroxy-3-[3-(4-trifluorométhylimidazol-2-yl)phénoxy]propanamine, correspondant à la formule (i), R = H, Ar = radical 64, où Z est trifluorométhyle, liaison imidazole-benzène : 2-4, chaîne en position 2 de la tétraline.

EXEMPLE 65.

A une solution de 9,5 g de 3-pyridinol in 100 ml de diméthylsulfoxyde on ajoute un solution de 2,3 g de sodium dans 40 ml de méthanol. On élimine le méthanol par distillation sous pression réduite, puis, à la solution résultante, on ajoute 10,2 g d'épichlorhydrine à 25°C. On agite le mélange 4 heures à 25°C, on verse la solution dans un mélange eau-glace (500 ml) et l'on extrait d'abord avec 100, puis avec 75, puis avec 50, enfin avec 25 ml de chloroforme. On lave les extraits réunis avec 50 ml d'eau, on sèche, on évapore le solvant et on obtient la 3-(2,3-époxypropoxy)pyridine sous forme d'huile. On dissout 3 g de l'huile obtenu dans 20 ml d'éthanol et on ajoute à la solution ainsi obtenue une solution de 3 g de 2-aminotétraline dans 20 ml d'éthanol. En opérant ensuite comme décrit dans l'Exemple 27, on obtient le chlorhydrate de N-(1,2,3,4-tétrahydronapht-2-yl)-2-hydroxy-3-pyrid-3-yloxypropanamine, correspondant à la formule (i), R = H, Ar = radical 65, où Z est H, chaîne en position 2 de la tétraline.

EXEMPLE 66.

En opérant comme décrit dans l'Exemple 27, à partir de 2,77 g de 1-acétyl-5-(2,3-époxypropoxy)-1,2,3,4-tétrahydroquinoléine et de 1,49 g de 2-aminotétraline dans 30 ml d'éthanol, on obtient le chlorhydrate de N-(1,2,3,4-tétrahydronapht-2-yl)-2-hydroxy-3-(1-acétyl-1,2,3,4-tétrahydroquinoléin- 5-yloxy)propanamine, correspondant à la formule (i), R = H, Ar = radical 66, où Z est méthyle, chaîne en position 2 de la tétraline.

EXEMPLE 67.

En opérant comme décrit dans l'Exemple 27, à partir de 2,33 g d'acétyl-4-(2,3-époxypropoxy)indazole, obtenu selon BE 853.949, et de 1,49 g de 2-aminotétraline dans 30 ml d'éthanol, on obtient le chlorhydrate de N-(1,2,3,4-tétrahydronapht-2-yl)-2-hydroxy-3-(1-acétylindazol-4-yl)propanamine, correspondant à la formule (i), R = H, Ar = radical 67, où Z est H et Z' est acétyle, chaîne en position 2 de la tétraline.

EXEMPLE 68.

En opérant comme décrit dans l'Exemple 27, à partir de 1,91 g de 4-(2,3-époxypropoxy)indazole, obtenu selon BE 853.949, et de 1,49 g de 2-aminotétraline dans 30 ml d'éthanol absolu, on obtient le chlohydrate de N-(1,2,3,4-tétrahydronapht-2-yl)-2-hydroxy-3-indazol-4-ylpropanamine, correspondant à la formule (i), R = H, Ar = radical 67, où Z et Z' sont H, chaîne en position 2 de la tétraline.

EXEMPLE 69.

En opérant comme décrit dans l'Exemple 27, à partir de 2,84 g de 6-(2,3-époxypropoxy)-1-hydroxyxanthén-9-one, obtenu selon US 3 912 733, et de 1,49 g de 2-aminotétraline dans 40 ml d'éthanol absolu, on obtient le chlorydrate de N-(1,2,3,4-tétrahydronapht-2-yl)-2-hydroxy-3-(1-hydroxy-9-oxoxanth-6-yloxy)propanamine, correspondant à la formule (i), R = H, Ar = radical 1, où Z est H, chaîne en position 2 de la tétraline.

EXEMPLES 70 A 77.

En opérant comme décrit dans l'Exemple 32, par réaction, respectivement, du 4-hydroxy-2-méthylbenzofuranne, du 4-hydroxybenzofuranne, du 5-hydroxybenzofuranne, du 5-hydroxy-2-méthylbenzofuranne, du 6-hydroxy-3-méthylbenzofuranne et du 7-hydroxybenzofuranne avec l'épichlorhydrine en présence de pipéridine et en traitant l'époxyde brute obtenu, dissous dans l'éthanol absolu, avec une solution de 2-aminotétraline dans l'éthanol absolu, on obtient, respectivement

- le chlorhydrate de N-(1,2,3,4-tétrahydronapht-2-yl)-2-hydroxy-3-(2-méthylbenzofuran-4-yloxy)propanamine (Ex. 70);
- le chlorhydrate de N-(1,2,3,4-tétrahydronapht-2-yl)-2-hydroxy-3-benzofuran-4-yloxypropanamine (Ex. 71);
- le chlorhydrate de N-(1,2,3,4-tétrahydronapht-2-yl)-2-hydroxy-3-benzofuran-5-yloxypropanamine (Ex. 72);
- le chlorhydrate de N-(1,2,3,4-tétrahydronapht-2-yl)-2-hydroxy-3-(2-méthylbenzofuran-5-yloxy)propanamine (Ex. 73);
- le chlorhydrate de N-(1,2,3,4-tétrahydronapht-2-yl)-2-hydroxy-3-(3-méthylbenzofuran-6-yloxy)propanamine (Ex. 74); et
- le chlorhydrate de N-(1,2,3,4-tétrahydronapht-2-yl)-2-hydroxy-3-benzofuran-7-yloxypropanamine (Ex. 75),

correspondant à des composés de formule (i), R = H, Ar = radical 27, où Z et Z' sont H ou méthyle, chaîne en position 2 de la tétraline.

De la même façon, à partir, respectivement, du 4-hydroxybenzothiophène et du 5-hydroxybenzothiophène, on obtient

- le chlorhydrate de N-(1,2,3,4-tétrahydronapht-2-yl)-2-hydroxy-3-benzothien-4-yloxypropanamine (Ex. 76); et
- le chlorhydrate de N-(1,2,3,4-tétrahydronapht-2-yl)-2-hydroxy-3-benzothién-5-yloxypropanamine (Ex. 77),

correspondant à des composés de formule (i), R = H, Ar = radical 28, où Z est H, chaîne en position 2 de la tétraline.

EXEMPLE 78.

En opérant comme décrit dans l'Exemple 33, a partir de 7-hydroxy-3-méthylthiophène et épichlorhydrine en présence d'hydroxyde de sodium et en traitant l'époxyde brut obtenu, dissous dans l'éthanol absolu, avec une solution de 2-aminotétraline dans l'éthanol absolu, on obtient le chlorhydrate de N-(1,2,3,4-tétrahydronapht-2-yl)-2-hydroxy-3-(3-méthylbenzothién-7-yloxy)propanamine, correspondant à la formule (i), R = H, Ar = radical 28, où Z est méthyle et la chaîne est dans la position 7 de Ar et en position 2 de la tétraline.

EXEMPLES 79 A 81.

En opérant comme décrit dans l'Exemple 32, à partir du 5-hydroxy-1,4-benzodioxanne et de l'épichlorhydrine en présence de pipéridine et en traitant une solution alcoolique de l'époxyde obtenu avec une solution alcoolique de 2-aminotétraline, on obtient le chlorhydrate de N-(1,2,3,4-tétrahydronapht-2-yl)-2-hydroxy-3-(1,4-benzodioxan-5-yloxy)propanamine (Ex. 79), correspondant à un composé de formule (iF), où E" et G", ensemble, forment un groupe $-O-CH_2-CH_2-O-$ et L" et R sont H, chaîne en position 2 de la tétraline.

De la même façon, à partir, respectivement, de la 6-hydroxy-3,4-dihydro-2H-1,5-benzodioxépine et de la 7-hydroxy-2,3,4,5-tétrahydro-1,6-benzodioxocine, on obtient, respectivement

- le chlorhydrate de N-(1,2,3,4-tétrahydronapht-2-yl)-2-hydroxy-3-(3,4-dihydro-2H-1,5-benzodioxépin-6-yloxy)propanamine (Ex. 80); et
- le chlorhydrate de N-(1,2,3,4-tétrahydronapht-2-yl)-2-hydroxy-3-(2,3,4,5-tétrahydro-1,6-benzodioxocin-7-yloxy)propanamine (Ex. 81), correspondant à des produits de formule (i), ou R = H, Ar = radical 48, où n est, respectivement, 3 et 4, chaîne en position 2 de la tétraline.

EXEMPLES 82 ET 83.

En opérant comme décrit dans l'Exemple 33, à partir, respectivement, de la 8-hydroxyquinoléine et de la 5-hydroxyquinoléine et de l'épichlorhydrine en présence d'hydroxyde de sodium et en traitant ensuite une solution alcoolique de l'époxyde ainsi obtenu avec une solution alcoolique de 2-aminotétraline, on obtient, respectivement,

- le chlorhydrate de N-(1,2,3,4-tétrahydronapht-2-yl)-2-hydroxy-3-quinoléin-8-yloxypropanamine, correspondant à la formule (i), R = H, Ar = radical 50, chaîne en position 2 de la tétraline (Ex. 82), et
- le chlorhydrate de N-(1,2,3,4-tétrahydronapht-2-yl)-2-hydroxy-3-quinoléin-5-yloxypropanamine, correspondant à la formule (i), R = H, Ar = radical 49, chaîne en position 2 de la tétraline (Ex. 83).

EXEMPLE 84.

En opérant comme décrit dans l'Exemple 27, à partir de 2,91 g de 8-acétylméthoxys-5-(2,3-époxypropoxy)-3,4-dihydrocarbostyrile, obtenu par réaction du 5,8-dihydroxy-3,4-dihydrocarbostyrile avec bromoacétone en présence de carbonate de potassium dans un mélange eau/acétone 4/1, au reflux pendant 5 heures, et 1,48 g de 2-aminotétraline dans 30 ml d'éthanol absolu, on obtient le chlorhydrate de N-(1,2,3,4-tétrahydronapht-2-yl)-2-hydroxy-3-(8-acétylméthoxy-3,4-carbostyril-5-yloxy)propanamine, correspondant à la formule (iF), R = H, L" = acétonyloxy, et E" et G", ensemble, forment un groupe -CH -CH -CO-NH-, chaîne en position 2 de la tétraline.

EXEMPLES 85 A 88.

En opérant comme décrit dans l'Exemple 27, à partir de 2,04 g de 2-propargyloxy-1-(2,3-époxypropoxy)benzène et de 2-aminotétraline dans 30 ml d'éthanol absolu, on obtient le chlorhydrate de N-(1,2,3,4-tétrahydronapht-2-yl)-2-hydroxy-3-(2-propargyloxyphényl)propanamine, p.f. 129-130°C (Ex. 85).
De la même façon, a partir de 2,04 g de 2-propargyloxy-1-(2,3-époxypropoxy)benzène et, respectivement de 1,48 g de 1-aminotétraline, 1,64 g de 2-amino-7-hydroxytétraline et 1,64 g de 2-amino-6-hydroxytétraline, on obtient, respectivement:

- le chlorhydrate de N-(1,2,3,4-tétrahydronapht-1-yl)-2-hydroxy-3-(2-propargyloxyphényl)propanamine, p.f. 114-116°C (Ex. 86);
- le chlorhydrate de N-(7-hydroxy-1,2,3,4-tétrahydronapht-2-yl)-2-hydroxy-3-(2-propargyloxyphényl)propanamine, p.f. 158-160°C (Ex. 87); et
- le chlorhydrate de N-(6-hydroxy-1,2,3,4-tétrahydronapht-2-yl)-2-hydroxy-3-(2-propargyloxyphényl)propanamine, p.f. 179-182°C (Ex. 88).

EXEMPLES 89 ET 90.

En opérant comme décrit dans l'Exemple 27, à partir de 5 g de 1-(2-acétamido-3-nitrophénoxy)-2,3-époxypropane et 2,96 g de 2-aminotétraline dans 80 ml d'éthanol absolu, on obtient le chlorhydrate de N-(1,2,3,4-tétrahydronapht-2-yl)-2-hydroxy-3-(2-acétamido-3-nitrophénoxy)- propanamine, correspondant à la formule (iA), R = H, E = acétamido, G = nitro, chaîne en position 2 de la tétraline (Ex. 89).
On refroidit jusqu'à congélation un mélange de 4 g de chlorhydrate de N-(1,2,3,4-tétrahydronapht-2-yl)-2-hydroxy-3-(2-acétamido-3-nitrophénoxy)propanamine et 19 ml d'acide formique à 97% dans un recipient pour l'hydrogénation, puis on déplace l'air par de l'azote et on ajoute rapidement 0,25 g de Pd/C à 10%. Après hydrogénation à la température ambiante et à 2,5 bar jusqu'à la complète absorption de l'hydrogène, on porte le mélange à la pression ambiante et on le chauffe 2 heures au bain-maire bouillant. On filtre le catalyseur, on évapore à sec et on reprend le résidu par de l'isopropanol saturé d'acide chlorhydrique gazeux. On obtient ainsi le dichlorhydrate de N-(1,2,3,4-tétrahydronapht-2-yl)-2-hydroxy3-benzimidazol-4-yloxypropanamine, correspondant à la formule (iF) où L" et R sont l'hydrogène et E" et G", ensemble, forment un groupe -N=CH-NH-, ou également à la formule (i), R = H, Ar = radical LXVII, chaîne en position 2 de la tétraline (Ex. 90).

EXEMPLES 91 ET 92.

On chauffe 5 heures au bain-maire bouillant un mélange de 1,1 g de 4-hydroxy-9-oxoxanthène, 4 g d'épichlorhydrine et 0,01 g de pipéridine, puis on élimine l'épichlorhydrine en excès par distillation, on dissout le résidu dans 50 ml d'acétate d'éthyle et on lave la solution avec 20 ml d'hydroxyde de sodium 2N. On évapore à sec et on dissout le 1-chloro-2-hydroxy-3-(9-oxoxanth-4-yloxy)propane brut ainsi obtenu (0,6 g) dans 20 ml d'éthanol absolu. On traite la solution ainsi obtenu avec une solution de 0,3 g de 2-aminotétraline dans 10 ml d'éthanol et on chauffe le mélange 4 heures au reflux. On évapore à sec et on reprend le résidu par une solution saturée d'acide chlorhydrique gazeux dans l'isopropanol. On obtient ainsi le chlorhydrate de N-(1,2,3,4-tétrahydronapht-2-yl)-2-hydroxy-3-(9-oxoxanth-4-yloxy)propanamine, correspondant à la formule (i), R = H, Ar = radical 29, où Z est CO, chaîne en position 2 de la tétraline (Ex. 91).
De la même façon, à partir du 4-hydroxyxanthène, on obtient le chlorhydrate de N-(1,2,3,4-tétrahydronapht-2-yl)-2-hydroxy-3-xanth-4-yloxypropanamine, correspondant à la formule (i), R = H, Ar = radical 29, où Z est méthylène, chaîne en position 2 de la tétraline (Ex. 92).

EXEMPLES 93 A 96.

En opérant comme décrit dans l'Exemple 91, à partir, respectivement du 4-hydroxy-1-méthylcarbostyrile, du 4-hydroxy-1-éthylcarbostyrile, du 4-hydroxy-1-allylcarbostyrile et du 4-hydroxy-1-phénylcarbostyrile et par réaction de la chlorhydrine brute ainsi obtenue avec la 2-aminotétraline, on obtient, respectivement

- le chlorhydrate de N-(1,2,3,4-tétrahydronapht-2-yl)-2-hydroxy-3-(1-méthyl-2-oxo-1,2-dihydroquinoléin-4-yloxy)propanamine (Ex. 93);
- le chlorhydrate de N-(1,2,3,4-tétrahydronapht-2-yl)-2-hydroxy-3-(1-éthyl-2-oxo-1,2-dihydroquinoléin-4-yloxy)propanamine (Ex. 94);
- le chlorhydrate de N-(1,2,3,4-tétrahydronapht-2-yl)-2-hydroxy-3-(1-allyl-2-oxo-1,2-dihydroquinoléin-4-yloxy)propanamine (Ex. 95); et
- le chlorhydrate de N-(1,2,3,4-tétrahydronapht-2-yl)-2-hydroxy-3-(1-phényl-2-oxo-1,2-dihydroquinoléin-4-yloxy)propanamine (Ex. 96); correspondant à des produits de formule (i), R = H, Ar = radical 30, où Z est, respectivement, méthyle, éthyle, allyle, phényle; chaîne en position 2 de la tétraline.

EXEMPLE 97.

En opérant comme décrit dans l'Exemple 27, à partir de 4,37 g de 1-(1-phényltétrazol-5-yloxy)-2,3-époxypropane (BE 866 278) et de 1,48 g de 2-aminotétraline dans 60 ml d'éthanol absolu, on obtient le chlorhydrate de N-(1,2,3,4-tétrahydronapht-2-yl)-2-hydroxy-3-(1-phényltétrazol-5-yloxy)propanamine, correspondant à la formule (i), R = H, Ar = radical 60, où Z et Z' sont hydrogène, chaîne en position 2 de la tétraline.

EXEMPLE 98.

On chauffe 5 heures au reflux un mélange de 1,95 g de 1-(2-naphtyloxy)-2,3-époxypropane, 2,65 g de 2-aminotétraline et 35 ml d'éthanol, puis on évapore l'éthanol et l'on obtient la N-(1,2,3,4-tétrahydronapht-2-yl)-2-hydroxy-3-(2-naphtyloxy)propanamine base sous forme d'un solide qui est cristallisé de l'acétate d'éthyle (5 ml). On filtre, on lave à l'acétate d'éthyle et puis à l'éther éthylique. Après essication, on obtient 1,8 g de produit; p.f. 109-115°C.

EXEMPLE 99.

En opérant comme décrit dans l'Exemple 98, par réaction de 4,5 g de 1-(2-naphtyloxy)-2,3-époxypropane avec 2,95 g de 1-aminotétraline, on obtient la N-(1,2,3,4-tétrahydronapht-1-yl)-2-hydroxy-3-(2-naphtyloxy)propanamine base sous forme d'une huile qui est dissoute dans 40 ml d'un mélange isopropyl éther/isopropanol 1/1 et acidifiée avec HCl dans l'isopropanol. On obtient ainsi le chlorhydrate qui est cristallisé de 20 ml d'isopropanol; p.f. 135-137°C. Rendement: 2,2 g.

EXEMPLE 100.

En opérant comme décrit dans l'Exemple 98, par réaction de 3,8 g de (R)-1-(1-naphtyloxy)-2,3-époxypropane (/alpha/ = -31,08°; méthanol, c = 1,5 %, préparé à partir de l'1-naphtol selon la méthode décrite en Heterocycles, 1983,20,1975-1978) et de 2,8 g de S(+)-1-aminotétraline, obtenue du chlorhydrate de S(-)-1-aminotétraline (Il Farmaco Ed. Sci., 1971, 26, 475-486) par neutralization avec de l'hydroxyde de sodium à 15% et extraction avec l'éther éthylique, en présence de 5 mg de chlorhydrate de S(-)-1-aminotétraline, on obtient la N-[(S)-1,2,3,4-tétrahydronapht-1-yl)]-(R)-2-hydroxy-3-(1-naphtyloxy)propanamine sous forme d'une huile jaune qui, repris avec une petite quantité d'éther isopropy- lique, cristallise. Après recristallisation de 50 ml d'isopropanol, on obtient 3,6 g de produit; p.f. 107-109°C, /alpha/ = +6,6° (méthanol, c = 1%).

EXEMPLE 101.

En opérant comme décrit dans les Exemples 98 et 100, à partir de 4,5 g de (S)-1-(1-naphtyloxy)-2,3-époxypropane (/alpha/ = +30,4°; méthanol, c = 1,5 %, voir Exemple 100) et de 3,3 g de R(-)-1-aminotétraline base, obtenue du chlorhydrate (Il Farmaco Ed. Sci., 1971, 26, 475-486), on obtient la N-[(R)-1,2,3,4-tétrahydronapht-1-yl)]-(S)-2-hydroxy-3-(1-naphtyloxy)propanamine base; p.f. 107-109°C, /alpha/ = -6,5° (méthanol, c = 1%).

EXEMPLE 102.

En opérant comme décrit dans l'Exemple 99, à partir de 4,5 g de (R)-1-(1-naphtyloxy)-2,3-époxypropane et de 3,3 g de (R)-1-aminotétraline base, on obtient, après cristallisation de 50 ml d'éthanol, 4,4 g de chlorhydrate de N-[(R)-1,2,3,4-tétrahydronapht-1-yl)]-(R)-2-hydroxy-3-(1-naphtyloxy)propanamine; p.f. 176-178°C, /alpha/ = +21,9° (méthanol, c = 1%).

EXEMPLE 103.

En opérant comme décrit dans l'Exemple 102, à partir de 3,3 g de (S)-1-(1-naphtyloxy)-2,3-époxypropane et de 2,5 g de S(+)-1-aminotétraline en présence de 5 mg de chlorhydrate de S(-)-1-aminotétraline, on obtient, après cristallisation de 40 ml d'éthanol, 3,4 g de chlorhydrate de N-[(S)-1,2,3,4-tétrahydronapht-1-yl)]-(S)-2-hydroxy-3-(1-naphtyloxy)propanamine; p.f. 177-179°C, /alpha/ = -20,5° (méthanol, c = 1%).

EXEMPLES 104 - 107.

En opérant comme décrit dans les Exemples 98 et 99, on obtient les composés suivants, sous forme de base libre ou de chlorhydrate

- N-(1,2,3,4-tétrahydronapht-2-yl)-2-hydroxy-3-(5,6,7,8-tétrahydronapht-1-yloxy)propanamine; p.f. 90-93°C (cristallisé dans l'acétate d'éthyle) (Ex. 104);
- chlorhydrate de N-(7-hydroxy-1,2,3,4-tétrahydronapht-2-yl)-2-hydroxy-3-napht-2-yloxypropanamine; p.f. 184-187°C (éthanol) (Ex. 105);
- chlorhydrate de N-(7-hydroxy-1,2,3,4-tétrahydronapht-2-yl)-2-hydroxy-3-(5,6,7,8-tétrahydronapht- 1-yloxy)propanamine; p.f. 172-175°C (isopropanol) (Ex. 106);
- chlorhydrate de N-(1,2,3,4-tétrahydronapht-1-yl)-2-hydroxy-3-(5,6,7,8-tétrahydronapht-1-yloxy)propanamine; p.f. 172-176°C (isopropanol) (Ex. 107).

EXEMPLES 108 - 112.

De la même façon, on obtient les composés de formule (iA) suivants

- chlorhydrate de N-(1,2,3,4-tétrahydronapht-1-yl)-2-hydroxy-3-(2-cyanophénoxy)propanamine; p.f. 161-163°C (isopropanol) (Ex. 108);
- chlorhydrate de N-(1,2,3,4-tétrahydronapht-1-yl)-2-hydroxy-3-(3-cyanophénoxy)propanamine; p.f. 185-187°C (éthanol 95%) (Ex. 109);
- chlorhydrate de N-(1,2,3,4-tétrahydronapht-1-yl)-2-hydroxy-3-(3-trifluorométhylphénoxy)propanamine; p.f. 135-137°C (isopropanol) (Ex. 110);
- N-(1,2,3,4-tétrahydronapht-2-yl)-2-hydroxy-3-(3-trifluorométhylphénoxy)propanamine; p.f. 84-89°C (éther isopropylique) (Ex. 111);
- chlorhydrate de N-(7-hydroxy-1,2,3,4-tétrahydronapht-2-yl)-2-hydroxy-3-(3-trifluorométhylphénoxy)propanamine; p.f. 215-217°C (isopropanol) (Ex. 112).

EXEMPLES 113 - 119.

De la même façon, on prépare les composés de formule (iB) suivants

- chlorhydrate de N-(1,2,3,4-tétrahydronapht-1-yl)-2-hydroxy-3-(4-cyanophénoxy)propanamine; p.f. 156-160°C (acétone) (Ex. 113);
- chlorhydrate de N-(7-hydroxy-1,2,3,4-tétrahydronapht-2-yl)-2-hydroxy-3-[4-(2-méthoxyéthyl)phénoxy]propanamine; p.f. 153-156°C (isopropanol) (Ex. 114);
- N-(1,2,3,4-tétrahydronapht-2-yl)-2-hydroxy-3-[4-(2-méthoxyéthyl)phénoxy]propanamine; p.f. 95-97°C (éther isopropylique) (Ex. 115);
- chlorhydrate de N-(1,2,3,4-tétrahydronapht-1-yl)-2-hydroxy-3-[4-(2-méthoxyéthyl)phénoxy]propanamine; p.f. 136-139°C (isopropanol) (Ex. 116);
- chlorhydrate de N-(7-hydroxy-1,2,3,4-tétrahydronapht-2-yl)-2-hydroxy-3-(4-chlorophénoxy)propanamine; p.f. 168-170°C (isopropanol) (Ex. 117);
- N-(1,2,3,4-tétrahydronapht-2-yl)-2-hydroxy-3-(4-chlorophénoxy)propanamine; p.f. 117-119°C (acétate d'éthyle) (Ex. 118);
- chlorhydrate de N-(1,2,3,4-tétrahydronapht-1-yl)-2-hydroxy-3-(4-chlorophénoxy)propanamine; p.f. 131-135°C (isopropanol) (Ex. 119).

EXEMPLES 120 - 122.

En opérant comme décrit dans l'Exemple 1, à partir, respectivement, de la 1-aminotétraline, de la 2-aminotétraline et de la 2-amino-7-hydroxytétraline, et par réaction avec le 3-(2,3-époxy-1-propoxy)-4-morpholino-1,2,5-thiadiazole (préparé comme décrit dans le J. Org. Chem., 1979, 44, 1826), on obtient, respectivement

- le chlorhydrate de N-(1,2,3,4-tétrahydronapht-1-yl)-2-hydroxy-3-(4-morpholino-1,2,5-thiadiazol-4-yloxy)propanamine; p.f. 156-159°C (isopropanol) (Ex. 120);
- le chlorhydrate de N-(1,2,3,4-tétrahydronapht-2-yl)-2-hydroxy-3-(4-morpholino-1,2,5-thiadiazol-4-yloxy)propanamine; p.f. 171-174°C (isopropanol) (Ex. 121);
- la N-(7-hydroxy-1,2,3,4-tétrahydronapht-2-yl)-2-hydroxy-3-(4-morpholino-1,2,5-thiadiazol-4-yloxy)propanamine; p.f. 187-191°C (isopropanol) (Ex. 122).

EXEMPLE 123.

(a) On dissout 51,42 g de 3-chlorophénol dans une solution de 17,2 g d'hydroxyde de sodium dans 70 ml d'eau et on y ajoute 46,9 ml d'épichlorhydrine. Après une nuit sous agitation à température ambiante, on extrait le mélange réactionnel par de l'éther éthylique et on lave la phase organique à une solution de NaOH 1N et ensuite à l'eau. On sèche sur sulfate de sodium et, après concentration sous pression réduite on obtient 46 g de 1-(3-chlorophénoxy)-2,3,-époxypropane; p.e. 105-110°C à 0,5 mmHg.

(b) On chauffe au reflux pendant 5 heures 2,2 g de 1-aminotétraline et 2,77 g du produit ci-dessus dans 75 ml d'éthanol absolu. On évapore à sec et on chromatographie le résidu sur une colonne de gel de silice en éluant par un mélange acétate d'éthyle/cyclohexane 9/1 v/v. On évapore à sec les fractions réunies, on dissout le résidu ainsi obtenu dans de l'isopropanol (25 ml) et on acidifie par de l'acide chlorhydrique dans de l'isopropanol. Après filtration du précipité, on obtient 1,6 g de chlorhydrate de N-(1,2,3,4-tétrahydronapht-1-yl)-2-hydroxy-3-(3-chlorophénoxy)propanamine; p.f. 157-158°C.

EXEMPLE 124.

On chauffe au reflux pendant 5 heures une solution de 2 g de 2-amino-7-hydroxytétraline et 2,25 g de 1-(3-chlorophénoxy)-2,3-époxypropane dans 50 ml d'éthanol absolu. On évapore l'éthanol sous pression réduite et on chromatographie le résidu ainsi obtenu sur une colonne de gel de silice en éluant d'abord par de l'acétate d'éthyle et ensuite par un mélange acétate d'éthyle/méthanol 9/1 v/v jusqu'à la complète élution des fractions du produit. On réunit les fractions ainsi obtenues et on le concentre à sec. On dissout le résidu dans 20 ml d'isopropanol et on rend acide la solution par de l'acide chlorhydrique dans l'isopropanol. Après filtration du précipité, on le lave à l'éther éthylique et on obtient 2,2 g de chlorhydrate de N-(7-hydroxy-1,2,3,4-tétrahydronapht-2-yl)-2-hydroxy-3-(3-chlorophénoxy)propanamine; p.f. 193-195°C.

EXEMPLE 125.

On chauffe au reflux pendant 5 heures une solution de 1,1 g de 2-aminotétraline et 1,38 g de 1-(3-chlorophénoxy)-2,3-époxypropane dans 50 ml d'éthanol absolu. On évapore l'éthanol sous pression réduite et on reprend le résidu par 15 ml d'acétate d'éthyle pour cristalliser le produit. On le dissout dans 20 ml d'isopropanol et on rend acide la solution par de l'acide chlorhydrique dans l'isopropanol. On obtient un précipité qui, après cristallisation dans 50 ml d'éthanol à 95%, donne 1,0 g de chlorhydrate de N-(1,2,3,4-tétrahydronapht-2-yl)-2-hydroxy-3-(3-chlorophénoxy)propanamine; p.f. 172-174°C.

EXEMPLE 126.

On chauffe au reflux pendant 5 heures une solution de 2,95 g de 1-aminotétraline et 4,09 g de [2-(2-propynyloxy)phénoxy]-2,3-époxypropane (préparé selon la méthode décrite dans la demande de brevet hollandais 6516433) dans 100 ml d'éthanol. Après évaporation à sec, on soumet le résidu ainsi obtenu à une chromatographie sur une colonne de gel de silice en éluant par de l'acétate d'éthyle. On réunit les fractions et on les évapore à sec. On dissout le résidu dans 25 ml d'isopropanol et on y ajoute une solution d'acide chlorhydrique dans l'isopropanol. On filtre le précipité ainsi obtenu et, après cristallisation dans 12 ml d'isopropanol, on obtient 2,25 g de chlorhydrate de N-(1,2,3,4-tétrahydronapht-1-yl)-2-hydroxy-3-[2-(2-propynyloxy)phénoxy]propanamine; p.f. 114-116°C.

EXEMPLE 127.

En opérant comme décrit dans l'Exemple 1, à partir de 2,55 g de 2-amino-7-hydroxy-tétraline et de 3,18 g de [2-(2-propynyloxy)phénoxy]-2,3-époxypropane, on obtient un produit qui, après lavage à l'isopropanol et à l'éther éthylique, donne 2,6 g du chlorhydrate de N-(7-hydroxy-1,2,3,4-tétrahydronapht-2-yl)-2-hydroxy-3-[2-(2-propynyloxy)phénoxy]propanamine; p.f. 158-160°C.

EXEMPLE 128.

En opérant comme décrit dans l'Exemple 126, mais en remplaçant la 1-aminotétraline par la 2-aminotétraline, on obtient un précipité du chlorhydrate qui est lavé à l'isopropanol et à l'éther éthylique et qui donne le chlorhydrate de N-(1,2,3,4-tétrahydronapht-2-yl)-2-hydroxy-3-[2-(2-propynyloxy)phénoxy]propanamine; p.f. 129-130°C.

EXEMPLE 129.

En opérant comme décrit dans l'Exemple 1, à partir de 0,6 g de 2-amino-6-hydroxytétraline et de 0,75 g de [2-(2-propynyloxy)phénoxy]-2,3-époxypropane, on obtient 0,2 g de chlorhydrate de N-(6-hydroxy-1,2,3,4-tétrahydronapht-2-yl)-2-hydroxy-3-/2-(2-propynyloxy)phénoxy/propanamine; p.f. 179-182°C.

EXEMPLE 130.

On chauffe au reflux pendant 5 heures 2,1 g de 2-aminotétraline et 2,65 g de 1-(2-chlorophénoxy)-2,3-époxypropane (préparé selon la méthode décrite dans GB 767 991) dans 70 ml d'éthanol. On évapore à sec et on chromatographie le résidu ainsi obtenu sur une colonne de gel de silice en éluant par de l'acétate d'éthyle. Après évaporation à sec des fractions réunies, on cristallise le résidu dans 10 ml d'acétate d'éthyle et on obtient 1,5 g de N-(1,2,3,4-tétrahydronapht-2-yl)-2-hydroxy-3-(2-chlorophénoxy)propanamine; p.f. 98-101°C.

EXEMPLE 131.

On chauffe au reflux pendant 5 heures 2,95 g de 1-aminotétraline et 3,69 g de 1-(2-chlorophénoxy)-2,3-époxypropane dans 100 ml d'éthanol absolu. On évapore à sec et on chromatographie le résidu ainsi obtenu sur une colonne de gel de silice en éluant par de un mélange acétate d'éthyle/cyclohexane 7/3 v/v. On évapore à sec les fractions réunies et on reprend le résidu avec 10 ml d'isopropanol. On filtre, on cristallise le produit ainsi obtenu dans 10 ml d'isopropanol et on lave à l'héxane. On obtient 3,0 g de N-(1,2,3,4-tétrahydronapht-1-yl)-2-hydroxy-3-(2-chlorophénoxy)propanamine; p.f. 78-81°C.

EXEMPLE 132.

On chauffe au reflux pendant 5 heures 1,9 g de 2-amino-7-hydroxytétraline et 2,15 g de 1-(2-chlorophénoxy)-2,3-époxypropane dans 60 ml d'éthanol absolu. On évapore le solvant sous pression réduite et on soumet le résidu ainsi obtenu à une chromatographie sur une colonne de gel de silice en éluant d'abord par de l'acétate d'éthyle, jusqu'à la complète élution de la première tache et ensuite par un mélange acétate d'éthyle/méthanol 9/1 v/v. On réunit les fractions ainsi obtenues et on les évapore à sec. On dissout le résidu dans 20 ml d'isopropanol et on rend acide la solution par de l'acide chlorhydrique dans l'isopropanol. On filtre et on obtient 2,25 g de chlorhydrate de N-(7-hydroxy-1,2,3,4-tétrahydronapht-2-yl)-2-hydroxy-3-(2-chlorophénoxy)propanamine; p.f. 201-203°C.

EXEMPLE 133.

On chauffe à 80°C pendant 5 heures 2,5 g de 2-aminotétraline et 3,52 g de 1-(4-acétamidophénoxy)-2,3-époxypropane dans 40 ml de diméthylsulfoxide. On laisse refroidir le mélange réactionnel jusqu'à la température ambiante et on y ajoute 180 ml d'eau. On obtient un précipité qui, après cristallisation dans 120 ml d'éthanol absolu, donne 2,15 g de N-(1,2,3,4-tétrahydronapht-2-yl)-2-hydroxy-3-(4-acétamidophénoxy)propanamine; p.f. 133-136°C.

EXEMPLE 134.

On chauffe au reflux pendant 5 heures 5,92 g de 1-aminotétraline et 8,30 g de 1-(4-acétamidophénoxy)-2,3-époxypropane dans 200 ml d'éthanol absolu. On évapore à sec et on lave le résidu ainsi obtenu à l'isopropanol et à l'éther éthylique. On obtient 11,3 g de produit, caractérisé par p.f. 125-130°C. On dissout le produit ainsi obtenu dans de l'eau acide et on règle le pH à 10-11 par addition de NaOH. On filtre le produit précipité, l'on dissout dans une petite quantité de méthanol et on le soumet à une chromatographie sur une colonne de gel de silice en éluant par un mélange acétate d'éthyle/méthanol 9/1 v/v. On évapore à sec les fraction réunines et on lave à l'héxane le résidu ainsi obtenu. On obtient 2 g de N-(1,2,3,4-tétrahydronapht-1-yl)-2-hydroxy-3-(4-acétamidophénoxy)propanamine; p.f. 80-84°C.

EXEMPLE 135.

On chauffe au reflux pendant 5 heures 0,8 g de 1-aminotétraline et 1,3 g de 1-[(trans-6,7-dihydroxy-5,6,7,8-tétrahydronapht-1-yl)oxy]-2,3-époxypropane (préparé selon la méthode décrite dans J. Med. Chem., 1978, 21, No. 9, 913-922) dans 20 ml d'éthanol absolu. On évapore l'éthanol et on soumet le résidu à une chromatographie sur une colonne de gel de silice en éluant d'abord par de l'acétate d'éthyle et ensuite par un mélange acétate d'éthyle/méthanol 7/3 v/v. On évapore à sec les fractions réunies et on obtient 1,0 g de N-(1,2,3,4-tétrahydronapht-1-yl)-2-hydroxy-3-(trans-6,7-dihydroxy-5,6,7,8-tétrahydronapht-1-yloxy)propanamine sous forme de solide vitreux.
IR (KBr): 3362, 2930, 1454, 1253, 1065 cm-1

EXEMPLE 136.

(a) On refroidit à 0-5°C une solution de 5 g de R(+)-2,3-époxy-1-propanol, 7,54 g de 2-allylphénol et 14,7 de triphénylphosphine dans 80 ml de tétrahydrofuranne anhydre et on y ajoute goutte à goutte 11,7 g d'azodicarboxylate de diéthyle. On laisse le mélange réactionnel à température ambiante pendant 3 heures, on évapore à sec, on reprend le résidu ainsi obtenu par de l'éther éthylique et on le lave à une solution aqueuse d'hydroxyde de sodium à 10% et ensuite à l'eau. On sèche la solution organique sur du sulfate de sodium et on la concentre sous pression réduite. On soumet le résidu huileux ainsi obtenu à une chromatographie sur une colonne de gel de silice en éluant par un mélange hexane/acétate d'éthyle 7/3 v/v. On obtient 9,0 g de S(+)-1-(2-allylphénoxy)-2,3,-époxypropane qui est ensuite purifié par distillation; p.e. 100-105°C à 0,3 mmHg, /alpha/ = +14,3° (méthanol, c = 1 %).
(b) On chauffe au reflux pendant 5 heures un mélange de 1,7 g de S(-)-2-aminotétraline, 2,2 g du produit ci-dessus et 30 ml d'éthanol absolu. On évapore l'éthanol et on soumet le résidu ainsi obtenu à une chromatographie sur une colonne de gel de silice en éluant par de l'acétate d'éthyle. On réunit les fractions ainsi obtenues et on les évapore à sec. On obtient un résidu huileux qui, repris par un mélange de 10 ml d'acétate d'éthyle et de 10 ml d'hexane, cristallise. On filtre et on obtient 1,1 g de N-[(S)-1,2,3,4-tétrahydronapht-2-yl]-(S)-2-hydroxy-3-(2-allylphénoxy)propanamine; p.f. 70-72°C (éther isopropylique); /alpha/ = -51,0° (méthanol, c = 0,5 %).

EXEMPLE 137.

En opérant comme décrit dans l'Exemple 136, à partir de 2,2 g de R(+)-2-aminotétraline et de 2,85 g de S(+)-1-(2-allylphénoxy)-2,3-époxypropane, on obtient 2,3 g de N-[(R)-1,2,3,4-tétrahydronapht-2-yl]-(S)-2-hydroxy-3-(2-allylphénoxy)propanamine; p.f. 65-68°C; /alpha/ = + 32,0° (méthanol, c = 0,5 %).

EXEMPLE 138.

(a) En opérant comme décrit dans l'Exemple 136 (a), en remplaçant le R(+)-2,3-époxy-1-propanol par le S(-)-2,3-époxy-1-propanol, on obtient le R(-)-1-(2-allylphénoxy)-2,3,-époxypropane; /alpha/ = -11,8°C (méthanol, c = 1 %).
(b) On chauffe au reflux pendant 5 heures un mélange de 1,7 g de S(-)-2-aminotétraline, 2,2 g du produit ci-dessus et 30 ml d'éthanol absolu. On évapore l'éthanol et on chromatographie le résidu ainsi obtenu sur une colonne de gel de silice en éluant par de l'acétate d'éthyle. On réunit les fractions et on les évapore à sec. On reprend le résidu par de l'éther éthylique et on le filtre. On obtient 1,8 g de N-[(S)-1,2,3,4-tétrahydronapht-2-yl]-(R)-2-hydroxy-3-(2-allylphénoxy) propanamine; p.f. 70-72°C (acétate d'éthyle); /alpha/ = -31,3° (méthanol, c = 0,5 %).

EXEMPLE 139.

En opérant comme décrit dans l'Exemple 138, à partir de 2,2 g de R(+)-2-aminotétraline et de 2,85 g de R(-)-1-(2-allylphénoxy)-2,3-époxypropane, on obtient 3,0 g de N-[(R)-1,2,3,4-tétrahydronapht-2-yl]-(R)-2-hydroxy-3-(2-allylphénoxy)propanamine; p.f. 71-73°C (éther isopropylique); /alpha/ = + 53,6° (méthanol, c = 0,5 %).

EXEMPLES 140 - 143.

En opérant comme décrit dans les Exemples 136-139, en remplaçant la S(-)-2-aminotétraline par la S(-)-2-amino-7-hydroxytétraline et la R(+)-2-aminotétraline par la R(+)-2-amino-7-hydroxytétraline, on obtient les produits suivants:

- N-[(S)-7-hydroxy-1,2,3,4-tétrahydronapht-2-yl)]-(S)-2-hydroxy-3-(2-allylphénoxy)propanamine; p.f. 173-174°C (éther isopropylique); /alpha/ = -79,6° (méthanol, c = 0,5 %) (Ex. 140);
- N-[(R)-7-hydroxy-1,2,3,4-tétrahydronapht-2-yl)]-(S)-2-hydroxy-3-(2-allylphénoxy)propanamine; p.f. 167-169°C (éther isopropylique); /alpha/ = +31,4° (méthanol, c = 0,5 %) (Ex. 141);
- N-[(S)-7-hydroxy-1,2,3,4-tétrahydronapht-2-yl)]-(R)-2-hydroxy-3-(2-allylphénoxy)propanamine; p.f. 168-170°C (éther isopropylique); /alpha/ = -36,3° (méthanol, c = 0,5 %) (Ex. 142);
- N-[(R)-7-hydroxy-1,2,3,4-tétrahydronapht-2-yl)]-(R)-2-hydroxy-3-(2-allylphénoxy)propanamine; p.f. 173-175°C (éther isopropylique); /alpha/ = +75,6° (méthanol, c = 0,5 %) (Ex. 143).

EXEMPLES 144 - 147.

De la même façon, en opérant essentiellement comme décrit dans les Exemples 136-139, en remplaçant la S(-)-2-aminotétraline par la S(-)-1-aminotétraline et la R(+)-2-aminotétraline par la R(+)-1-aminotétraline, on obtient les produits suivants, sous forme de base libre ou de chlorhydrate

- chlorhydrate de N-[(S)-1,2,3,4-tétrahydronapht-1-yl)]-(S)-2-hydroxy-3-(2-allylphénoxy)propanamine; p.f. 127-129°C (isopropanol); /alpha/ = -23,4° (méthanol, c = 0,5 %) (Ex. 144);
- N-[(R)-1,2,3,4-tétrahydronapht-1-yl)]-(S)-2-hydroxy-3-(2-allylphénoxy)propanamine; p.f. 71-73°C (éther isopropylique); /alpha/ = -13,1° (méthanol, c = 0,5 %) (Ex. 145);
- N-[(S)-1,2,3,4-tétrahydronapht-1-yl)]-(R)-2-hydroxy-3-(2-allylphénoxy)propanamine; p.f. 72-74°C (éther isopropylique); /alpha/ = +12,6° (méthanol, c = 0,5 %) (Ex. 146);
- chlorhydrate de N-[(R)-1,2,3,4-tétrahydronapht-1-yl)]-(R)-2-hydroxy-3-(2-allylphénoxy)propanamine; p.f. 126-128°C (isopropanol); /alpha/ = +21,7° (méthanol, c = 0,5 %) (Ex. 147).

## Revendications

### Revendications pour les Etats contractants suivants : AT, BE, CH, DE, FR, GB, IT, LI, LU, NL, SE

**1.** Composé de formule (i)

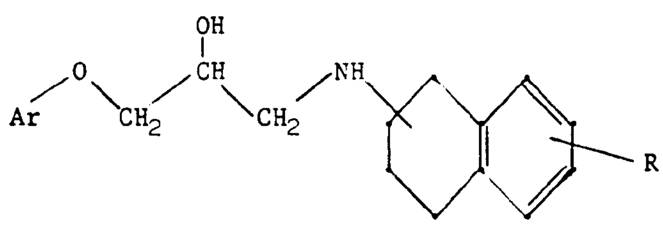

(i)

dans laquelle R représente l'hydrogène, un groupe hydroxy ou un groupe méthoxy et Ar représente un groupe aromatique ou hétéroaromatique éventuellement substitué, ou un de ses sels d'acides minéraux ou organiques, pharmaceutiquement acceptables ou non.

**2.** Composé selon la revendication 1, de formule (i) dans laquelle les acides minéraux ou organiques sont pharmaceutiquement acceptables.

3. Composé selon la revendication 2, de formule (i) dans laquelle Ar représente un groupe aromatique ou hétéroaromatique mono-, di-, ou tri- cyclique, éventuellement substitué, dont un atome de carbone du carbocycle aromatique ou de l'hétérocycle aromatique est directement lié à l'oxygène de l'aryloxypropanolaminotétraline.

4. Composé selon la revendication 3, de formule (i) dans laquelle Ar est le résidu éthérifiant en position 3 d'un 1-amino-2,3 propandiol N- substitué, ledit propandiol N-substitué ayant une activité bêta-bloquante adrénergique.

5. Composé selon la revendication 3 de formule (iA) ou (iB)

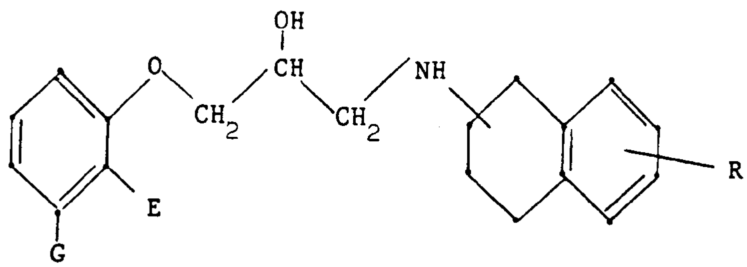

(iA)

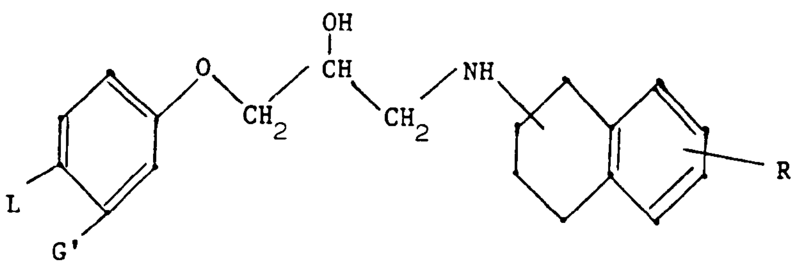

(iB)

dans lesquelles R est tel que défini dans la revendication 1, E représente l'hydrogène, un halogène, un groupe alkyle, alkényle, alkynyle, alcoxy, alkényloxy, alkynyloxy, tétrahydrofurfuryloxy-, phénoxy, cycloalkyle de 3 à 6 atomes de carbone, 1-cyclohexényle, 2-cyclohexényle, 2,5-méthano- cyclohexyle, alkylthio, alcanoyle, phénylalcanoyle, cyano, 3-chloroallyle, 2-indolyle, 2-benzoxazolyle, 2-benzothiazolyle, 2-quinolinyle, 2-quinoxalinyle, 2-thiényle, 4-thiazolyle, 4-morpholinyle, G est l'hydrogène, un halogène, un alkyle, un alkényle, un groupe trifluorométhyle, un groupe cyano ou un groupe acétamido, au moins un des substituants E et G étant l'hydrogène, ou bien E et G représentent, respectivement, un groupe acétamido et un groupe nitro ou, ensemble, forment un groupe $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, $-CH=CH-CH=CH-$, $-CO-CH_2-CH_2-CH_2-$, $-CH_2-CH_2-CH_2-CO-$ ou $-CH_2-CH(OH)-CH(OH)-CH_2-$; G' est l'hydrogène, L représente un halogène; un groupe alkyle, non substitué ou substitué par un groupe alcoxy, alcoxycarbonylamino, aminocarbonyle, cycloalkylalcoxy; un groupe alkényle, non substitué ou substitué par un groupe cyano; un groupe alcanoylamino ou haloalcanoylamino; un groupe uréido N'-mono ou disubstitué par des groupe alkyle ou cycloalkyle; un groupe alcoxy éventuellement substitué sur l'alkyle par un groupe alcoxy; un groupe cyano; ou un groupe hydroxyméthyle éthérifié par un groupe alcoxyalkyle; ou bien G' et L représentent, respectivement, un groupe nitro et un groupe acétamido; ou bien G' et L, ensemble forment un groupe $-CH=CH-CH=CH-$; ainsi que ses sels d'acides minéraux ou organiques, pharmaceutiquement acceptables.

6. Composé selon, la revendication 5, de formule (iA) ou (iB), dans lesquelles E représente l'hydrogène, un halogène, un groupe alkyle, alkényle, alkynyle, alcoxy, alkényloxy, alkynyloxy et G est l'hydrogène, ou bien E et G, ensemble, forment un groupe $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, $-CH=CH-CH=CH-$, $-CO-CH_2-CH_2-CH_2-$, $-CH_2-CH_2-CH_2-CO-$ ou $-CH_2-CH(OH)-CH(OH)-CH_2-$; G' représente l'hydrogène, L représente un atome de chlore, un groupe cyano ou un groupe 2-méthoxyéthyle ou bien G' et L, ensemble forment un groupe $-CH=CH-CH=CH-$.

7. Composé selon la revendication 6, de formule (iA), dans laquelle E représente un halogène, un groupe alkyle, alkényle, alcoxy, alkényloxy ou alkynyloxy, ou bien E et G, ensemble, forment un groupe -CH=CH-

CH=CH- ou $-CH_2-CH(OH)-CH(OH)-CH_2-$.

**8.** Composé selon la revendication 7 qui est la N-(1,2,3,4-tétrahydronapht-1-yl)-2-hydroxy-3-(1-naphtyloxy)propanamine sous forme optiquement active ou non, ou un de ses sels d'acides minéraux ou organiques pharmaceutiquement acceptables.

**9.** Composé selon la revendication 8 qui est la N-[(R)-1,2,3,4-tétrahydronapht-1-yl)]-(S)-2-hydroxy-3-(1-naphtyloxy)propanamine ou un de ses sels d'acides minéraux ou organiques pharmaceutiquement acceptables.

**10.** Composé selon la revendication 7 qui est la N-(7-hydroxy-1,2,3,4-tétrahydronapht-2-yl)-2-hydroxy-[3-(2-allylphénoxy)]propanamine sous forme optiquement active ou non ou un de ses sels d'acides minéraux ou organiques pharmaceutiquement acceptables.

**11.** Composé selon la revendication 10 qui est le chlorhydrate de N-(7-hydroxy-1,2,3,4-tétrahydronapht-2-yl)-2-hydroxy-[3-(2-allylphénoxy)]propanamine, sous forme otpiquement active ou non.

**12.** Composé selon la revendication 2, de formule (iF)

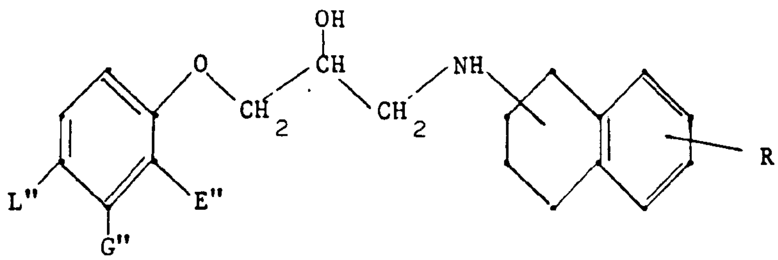

(iF)

dans laquelle R est tel que défini dans la revendication 1, L" est l'hydrogène et E" et G", ensemble, forment un groupe -CH=CH-NH-, ainsi que ses sels d'acides minéraux ou organiques, pharmaceutiquement acceptables.

**13.** Procédé pour la préparation d'un composé selon la revendication 1, caractérisé en ce qu'on traite un 1-aryloxypropane de formule

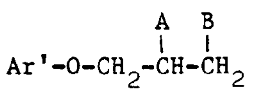

(ii)

dans laquelle Ar' a la signification donnée à la revendication 1 à Ar, mais peut contenir des groupes N-protecteurs fixés sur des groupes NH2 ou NH, A est un groupe hydroxy, B est un halogène choisi parmi le chlore, le brome ou l'iode ou bien A et B, ensemble, forment un groupe époxy, avec une aminotétraline de formule

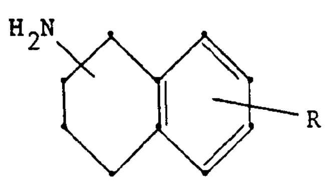

(iii)

dans laquelle R est tel que défini à la revendication 1, éventuellement en présence d'une amine tertiaire; on déprotège éventuellement le produit ainsi obtenu par élimination du ou des groupe(s) N-protecteur(s) ; lorsque le composé de départ a la formule (ii) où Ar' est un groupe 2-acétamido-3-nitrophényle ou 4-acétamido-3-nitrophényle, on déacétyle éventuellement le produit obtenu par chauffage dans l'acide chlorhydrique et on soumet éventuellement le produit obtenu à un hydrogénation en présence de palladium sur charbon à 10% dans de l'acide formique à 97%; et on transforme éventuellement le produit ainsi obtenu en un de ses sels.

**14.** Composition pharmaceutique contenant, en tant que principe actif, un composé de formule (i) selon l'une quelconque des revendications 2 à 12.

**15.** Composition pharmaceutique selon la revendication 14 sous forme d'unité de dosage, dans laquelle le principe actif est mélangé à un excipient pharmaceutique.

**16.** Composition pharmaceutique selon l'une des revendications 14 et 15, contenant de 0,1 à 500 mg de principe actif par unité de dosage.

**17.** Réactif pour la différentiation des récepteurs bêta-adrénergiques dans les essais biochimiques, caractérisé en ce qu'il comprend un composé selon la revendication 1 convenablement marqué.

**Revendications pour l'Etat contractant suivant : ES**

**1.** Procédé pour la préparation d'un composé de formule (i)

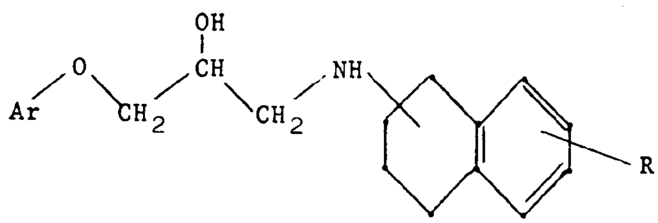

(i)

dans laquelle R représente l'hydrogène, un groupe hydroxy ou un groupe méthoxy et Ar représente un groupe aromatique ou hétéroaromatique éventuellement substitué, et de ses sels d'acides minéraux ou organiques, pharmaceutiquement acceptables ou non, caractérisé en ce qu'on traite un 1-aryloxypropane de formule

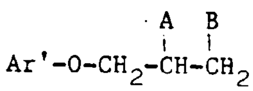

(ii)

dans laquelle Ar' a la signification donnée ci-dessus à Ar, mais peut contenir des groupes N-protecteurs fixés sur des groupes NH2 ou NH, A est un groupe hydroxy, B est un halogène choisi parmi le chlore, le brome ou l'iode ou bien A et B, ensemble, forment un groupe époxy, avec une aminotétraline de formule

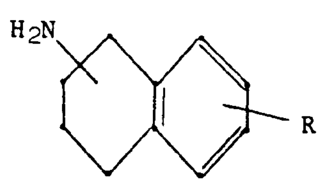

(iii)

dans laquelle R est tel que défini ci-dessus, éventuellement en présence d'une amine tertiaire; on déprotège éventuellement le produit ainsi obtenu par élimination du (des) groupe(s) N-protecteur(s) ; lorsque le composé de départ a la formule (ii) où Ar' est un groupe 2-acétamido-3-nitrophényle ou 4-acétamido-3-nitrophényle, on déacétyle éventuellement le produit obtenu par chauffage dans l'acide chlorhydrique et on soumet éventuellement le produit obtenu à un hydrogénation en présence de palladium sur charbon à 10% dans de l'acide formique à 97%; et on transforme éventuellement le produit ainsi obtenu en un de ses sels.

**2.** Procédé selon la revendication 1, caractérisé en ce que le 1-aryloxypropane de formule (ii) et l'aminotétraline de formule (iii) sont choisis pour la préparation d'un composé de formule (i) dans laquelle R est

tel que défini dans la revendication 1 et Ar représente un groupe aromatique ou hétéroaromatique mono-, di- ou tricyclique, éventuellement substitué, dont un atome de carbone du carbocycle aromatique ou de l'hétérocycle aromatique est directement lié à l'oxygène de l'aryloxypropanolaminotétraline; ou d'un de ses sels d'acides minéraux ou organiques.

3. Procédé selon la revendication 2, caractérisé en ce que le 1-aryloxypropane de formule (ii) et l'aminotétraline de formule (iii) sont choisis pour la préparation d'un composé de formule (iA) ou (iB)

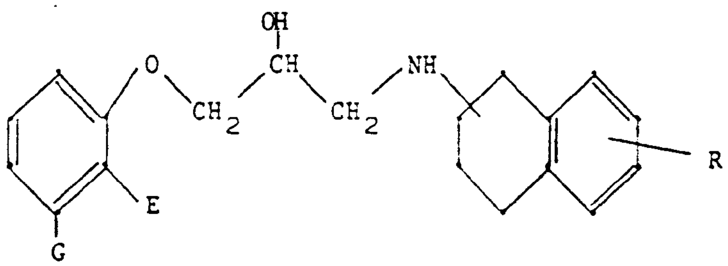

(iA)

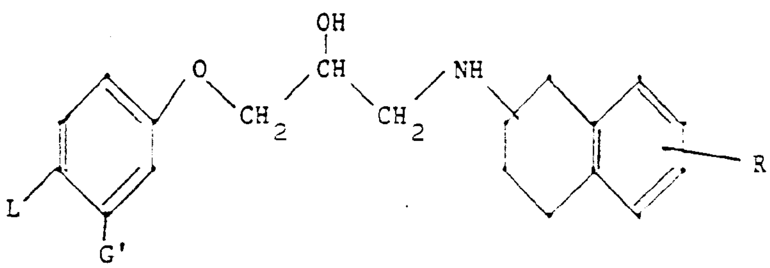

(iB)

dans lesquelles R est tel que défini dans la revendication 1, E représente l'hydrogène, un halogène, un groupe alkyle, alkényle, alkynyle, alcoxy, alkényloxy, alkynyloxy, tétrahydrofurfuryloxy, phénoxy, cycloalkyle de 3 à 6 atomes de carbone, 1-cyclohexényle, 2-cyclohexényle, 2,5-méthanocyclohexyle, alkylthio, alcanoyle, phénylalcanoyle, cyano, 3-chloroallyle, 2-indolyle, 2-benzoxazolyle, 2-benzothiazolyle, 2-quinolinyle, 2-quinoxalinyle, 2-thiényle, 4-thiazolyle, 4-morpholinyle, G est l'hydrogène, un halogène, un alkyle, un alkényle, un groupe trifluorométhyle, un groupe cyano ou un groupe acétamido, au moins un des substituants E et G étant l'hydrogène, ou bien E et G représentent, respectivement, un groupe acétamido et un groupe nitro ou, ensemble, forment un groupe $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, $-CH=CH-CH=CH-$, $-CO-CH_2-CH_2-CH_2$, $-CH_2-CH_2-CH_2-CO-$ ou $-CH_2-CH(OH)-CH(OH)-CH_2-$; G' est l'hydrogène, L représente un halogène; un groupe alkyle, non substitué ou substitué par un groupe alcoxy, alcoxycarbonylamino, aminocarbonyle, cycloalkylalcoxy; un groupe alkényle, non substitué ou substitué par un groupe cyano; un groupe alcanoylamino ou haloalcanoylamino; un groupe ureido N'-mono ou disubstitué par des groupe alkyle ou cycloalkyle; un groupe alcoxy éventuellement substitué sur l'alkyle par un groupe alcoxy; un groupe cyano; ou un groupe hydroxyméthyle éthérifié par un groupe alcoxyalkyle; ou bien G' et L représentent, respectivement, un groupe nitro et un groupe acétamido; ou bien G' et L, ensemble forment un groupe $-CH=CH-CH=CH-$; ou un de ses sels d'acides minéraux ou organiques.

4. Procédé selon la revendication 3, caractérisé en ce que le 1-aryloxypropane de formule (ii) et l'aminotétraline de formule (iii) sont choisis pour la préparation d'un composé de formule (iA) ou (iB) dans lesquelles E représente l'hydrogène, un halogène, un groupe alkyle, alkényle, alkynyle, alcoxy, alkényloxy, alkynyloxy et G est l'hydrogène, ou bien E et G, ensemble, forment un groupe $-CH_2-CH_2-CH_2$, $-CH_2-CH_2-CH_2-CH_2-$, $-CH=CH-CH_2-$, $-CH_2-CH=CH-$, $-CH=CH-CH_2-CH_2-$, $-CH=CH-CH=CH-$, $-CO-CH_2-CH_2-CH_2-$, $-CH_2-CH_2-CH_2-CO-$ ou $-CH_2-CH(OH)-CH(OH)-CH_2$, G' représente l'hydrogène, L représente un atome de chlore, un groupe cyano ou un groupe 2-méthoxyéthyle ou bien G' et L, ensemble forment un groupe $-CH=CH-CH=CH-$; ou un de ses sels d'acides minéraux ou organiques.

5. Procédé selon la revendication 4, caractérisé en ce que le 1-aryloxypropane de formule (ii) et l'aminotétraline de formule (iii) sont choisis pour la préparation d'un composé de formule (iA) dans laquelle E représente un halogène, un groupe alkyle, alkényle, alcoxy, alkényloxy ou alkynyloxy, ou bien E et G, ensemble, forment un groupe -CH=CH-CH=CH- ou $-CH_2-CH(OH)-CH(OH)-CH_2-$, ou un de ses sels d'acides minéraux ou organiques.

6. Procédé selon la revendication 5, caractérisé en ce que le 1-aryloxypropane de formule (ii) et l'aminotétraline de formule (iii) sont choisis pour la préparation de la N-(1,2,3,4-tétrahydronapht-1-yl)-2-hydroxy-3-(1-naphtyloxy-propanamine, sous forme optiquement active ou non, ou un de ses sels d'acides minéraux ou organiques.

7. Procédé selon la revendication 6, caractérisé en ce que le 1-aryloxypropane de formule (ii) et l'aminotétraline de formule (iii) sont choisis pour la préparation de la N-[(R)-1,2,3,4-tétrahydronapht-1-yl)]-(S)-2-hydroxy-3 (1-naphtyloxy)propanamine ou d'un de ses sels d'acides minéraux ou organiques.

8. Procédé selon la revendication 5, caractérisé en ce que le 1-aryloxypropane de formule (ii) et l'aminotétraline de formule (iii) sont choisis pour la préparation de la N-(7-hydroxy-1,2,3, 4-tétrahydronapht-2-yl)-2-hydroxy-[3-(2-allylphénoxy)] propanamine, sous forme optiquement active ou non, ou d'un de ses sels d'acides minéraux ou organiques.

9. Procédé selon la revendication 8, caractérisé en ce que le 1-aryloxypropane de formule (ii) et l'aminotétraline de formule (iii) sont choisis pour la préparation du chlorhydrate de N-(7-hydroxy-1,2,3,4-tétrahydronapht-2-yl)-2-hydroxy-[3-(2-allylphénoxy)]propanamine, sous forme optiquement active ou non.

10. Procédé selon la revendication 1, caractérisé en ce que le 1-aryloxypropane de formule (ii) et l'aminotétraline de formule (iii) sont choisis pour la préparation d'un composé de formule (iF)

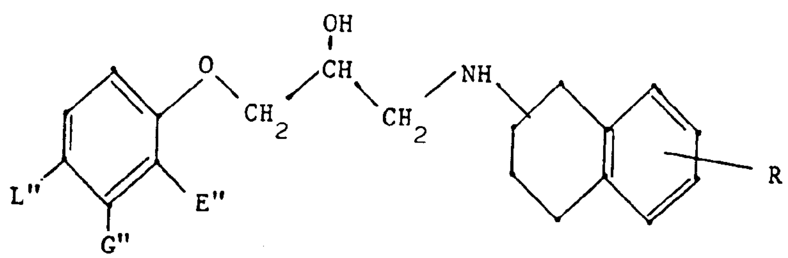

(iF)

dans laquelle R est tel que défini dans la revendication 1, L" est l'hydrogène et E" et G", ensemble, forment un groupe -CH=CH-NH-, ou d'un de ses sels d'acides minéraux ou organiques.

11. Procédé pour la préparation de compositions pharmaceutiques caractérisé en ce qu'il consiste à mélanger, à titre de principe actif, un composé de formule (i) préparé selon l'une quelconque des revendications 1 à 10 ou un de ses sels pharmaceutiquement acceptable, à un excipient pharmaceutique.

12. Procédé selon la revendication 11, pour la préparation de compositions pharmaceutiques sous forme d'unité de dosage.

13. Procédé selon l'une des revendications 11 ou 12, pour la préparation de compositions pharmaceutiques contenant de 0,1 à 500 mg de principe actif par unité de dosage.

14. Réactif pour la différentiation des récepteurs bêta-adrénergiques dans les essais biochimiques, caractérisé en ce qu'il comprend un composé de formule (i)

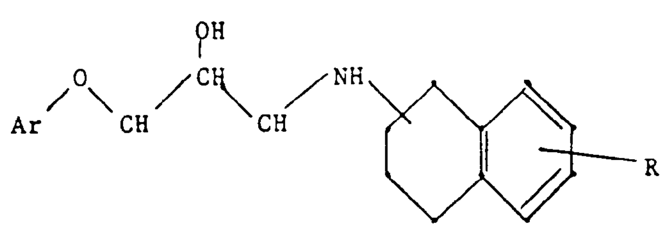

(i)

dans laquelle R et Ar sont tels que définis dans la revendication 1, convenablement marqué.

**Revendications pour l'Etat contractant suivant : GR**

**1.** Composé de formule (i)

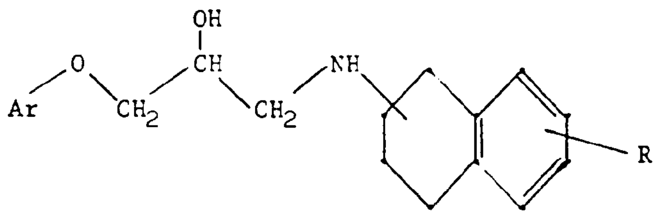

(i)

dans laquelle R représente l'hydrogène, un groupe hydroxy ou un groupe méthoxy et Ar représente un groupe aromatique ou hétéroaromatique éventuellement substitué, ou un de ses sels d'acides minéraux ou organiques, pharmaceutiquement acceptables ou non.

**2.** Composé selon la revendication 1, de formule (i) dans laquelle les acides minéraux ou organiques sont pharmaceutiquement acceptables.

**3.** Composé selon la revendication 2, de formule (i) dans laquelle Ar représente un groupe aromatique ou hétéroaromatique mono-, di-, ou tri- cyclique, éventuellement substitué, dont un atome de carbone du carbocycle aromatique ou de l'hétérocycle aromatique est directement lié à l'oxygène de l'aryloxypropanolaminotétraline.

**4.** Composé selon la revendication 3, de formule (i) dans laquelle Ar est le résidu éthérifiant en position 3 d'un 1-amino-2,3-propandiol N- substitué, ledit propandiol N-substitué ayant une activité bêta-bloquante adrénergique.

**5.** Composé selon la revendication 3 de formule (iA) ou (iB)

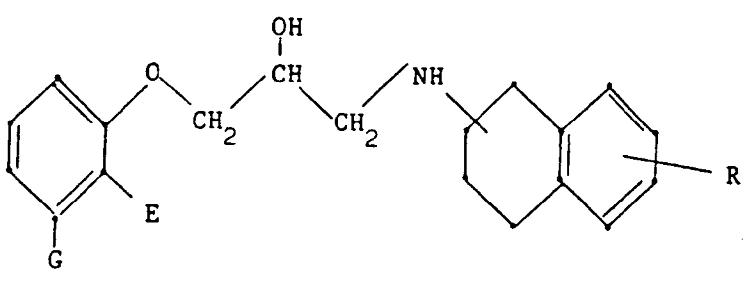

(iA)

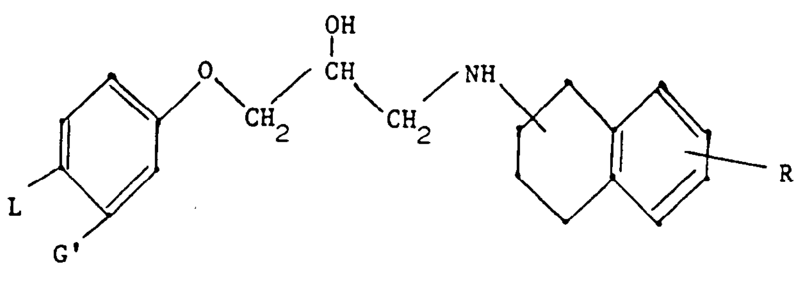

(iB)

dans lesquelles R est tel que défini dans la revendication 1, E représente l'hydrogène, un halogène, un groupe alkyle, alkényle, alkynyle, alcoxy, alkényloxy, alkynyloxy, tétrahydrofurfuryloxy, phénoxy, cycloalkyle de 3 à 6 atomes de carbone, 1-cyclohexényle, 2-cyclohexényle, 2,5-méthano- cyclohexyle, alkylthio, alcanoyle, phénylalcanoyle, cyano, 3-chloroallyle, 2-indolyle, 2-benzoxazolyle, 2-benzothiazolyle, 2-quinolinyle, 2-quinoxalinyle, 2-thiényle, 4-thiazolyle, 4-morpholinyle, G est l'hydrogène, un halogène, un

alkyle, un alkényle, un groupe trifluorométhyle, un groupe cyano ou un groupe acétamido, au moins un des substituants E et G étant l'hydrogène, ou bien E et G représentent, respectivement, un groupe acétamido et un groupe nitro ou, ensemble, forment un groupe $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, -CH=CH-CH=CH-, $-CO-CH_2\ CH_2-CH_2-$, $-CH_2-CH_2-CH_2-CO-$ ou $-CH_2-CH(OH)-CH(OH)-CH_2-$; G' est l'hydrogène, L représente un halogène; un groupe alkyle, non substitué ou substitué par un groupe alcoxy, alcoxycarbonylamino, aminocarbonyle, cycloalkylalcoxy; un groupe alkényle, non substitué ou substitué par un groupe cyano; un groupe alcanoylamino ou haloalcanoylamino; un groupe uréido N'-mono ou disubstitué par des groupe alkyle ou cycloalkyle; un groupe alcoxy éventuellement substitué sur l'alkyle par un groupe alcoxy; un groupe cyano; ou un groupe hydroxyméthyle éthérifié par un groupe alcoxyalkyle; ou bien G' et L représentent, respectivement, un groupe nitro et un groupe acétamido; ou bien G' et L, ensemble forment un groupe -CH=CH-CH=CH-; ainsi que ses sels d'acides minéraux ou organiques, pharmaceutiquement acceptables.

6. Composé selon la revendication 5, de formule (iA) ou (iB), dans lesquelles E représente l'hydrogène, un halogène, un groupe alkyle, alkényle, alkynyle, alcoxy, alkényloxy, alkynyloxy et G est l'hydrogène, ou bien E et G, ensemble, forment un groupe $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, -CH=CH-CH=CH-, $-CO-CH_2-CH_2-CH_2-$, $-CH_2-CH_2-CH_2-CO-$ ou $-CH_2-CH(OH)-CH(OH)-CH_2-$; G' représente l'hydrogène, L représente un atome de chlore, un groupe cyano ou un groupe 2-méthoxyéthyle ou bien G' et L, ensemble forment un groupe -CH=CH-CH=CH-.

7. Composé selon la revendication 6, de formule (iA), dans laquelle E représente un halogène, un groupe alkyle, alkényle, alcoxy, alkényloxy ou alkynyloxy, ou bien E et G, ensemble, forment un groupe -CH=CH-CH=CH- ou $-CH_2-CH(OH)-CH(OH)-CH_2-$.

8. Composé selon la revendication 7 qui est la N-(1,2,3,4-tétrahydronapht-1-yl)-2-hydroxy-3-(1-naphtyloxy)-propanamine sous forme optiquement active ou non, ou un de ses sels d'acides minéraux ou organiques pharmaceutiquement acceptables.

9. Composé selon la revendication 8 qui est la N-[(R)-1,2,3,4-tétrahydronapht-1-yl)]-(S)-2-hydroxy-3-(1-naphtyloxy)propanamine ou un de ses sels d'acides minéraux ou organiques pharmaceutiquement acceptables.

10. Composé selon la revendication 7 qui est la N-(7-hydroxy-1,2,3,4-tétrahydronapht-2-yl)-2-hydroxy-[3-(2-allylphénoxy)]propanamine sous forme optiquement active ou non ou un de ses sels d'acides minéraux ou organiques pharmaceutiquement acceptables.

11. Composé selon la revendication 10 qui est le chlorhydrate de N-(7-hydroxy-1,2,3,4-tétrahydronapht-2-yl)-2-hydroxy-[3-(2-allylphénoxy)]propanamine, sous forme otpiquement active ou non.

12. Composé selon la revendication 2, de formule (iF)

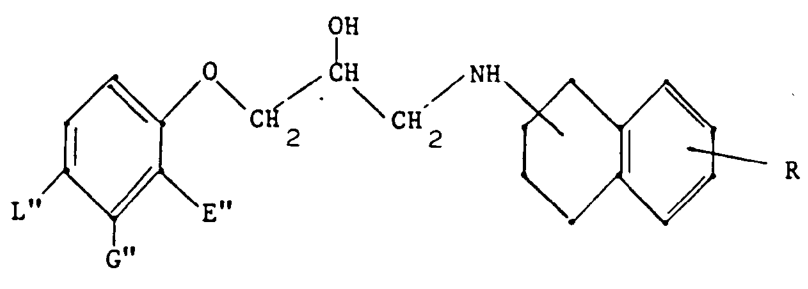

(iF)

dans laquelle R est tel que défini dans la revendication 1, L" est l'hydrogène et E" et G", ensemble, forment un groupe -CH=CH-NH-, ainsi que ses sels d'acides minéraux ou organiques, pharmaceutiquement acceptables.

13. Procédé pour la préparation d'un composé selon la revendication 1, caractérisé en ce qu'on traite un 1-aryloxypropane de formule

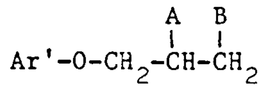

(ii)

dans laquelle Ar' a la signification donnée à la revendication 1 à Ar, mais peut contenir des groupes N-protecteurs fixés sur des groupes NH2 ou NH, A est un groupe hydroxy, B est un halogène choisi parmi le chlore, le brome ou l'iode ou bien A et B, ensemble, forment un groupe époxy, avec une aminotétraline de formule

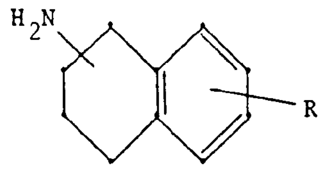

(iii)

dans laquelle R est tel que défini à la revendication 1, éventuellement en présence d'une amine tertiaire; on déprotège éventuellement le produit ainsi obtenu par élemination du ou des groupe(s) N-protecteur(s) ; lorsque le composé de départ a la formule (ii) où Ar' est un groupe 2-acétamido-3-nitrophényle ou 4-acétamido-3-nitrophényle, on déacétyle éventuellement le produit obtenu par chauffage dans l'acide chlorhydrique et on soumet éventuellement le produit obtenu à un hydrogénation en présence de palladium sur charbon à 10% dans de l'acide formique à 97%; et on transforme éventuellement le produit ainsi obtenu en un de ses sels.

**14.** Procédé pour la préparation de compositions pharmaceutiques caractérisé en ce qu'il consiste à mélanger, à titre de principe actif, un composé de formule (i) selon l'une quelconque des revendications 2 à 12 ou un de ses sels pharmaceutiquement acceptable, à un excipient pharmaceutique.

**15.** Procédé selon la revendication 14, pour la préparation de compositions pharmaceutiques sous forme d'unité de dosage.

**16.** Procédé selon l'une des revendications 14 ou 15, pour la préparation de compositions pharmaceutiques contenant de 0,1 à 500 mg de principe actif par unité de dosage.

**17.** Réactif pour la différentiation des récepteurs bêta-adrénergiques dans les essais biochimiques, caractérisé en ce qu'il comprend un composé selon la revendication 1 convenablement marqué.

## Patentansprüche

### Patentansprüche für folgende Vertragsstaaten : AT, BE, CH, DE, FR, GB, LI, LU, NL, SE

**1.** Verbindung der Formel (i)

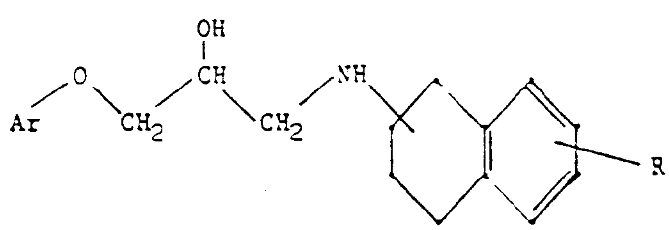

(i)

worin R Wasserstoff, eine Hydroxy- oder eine Methoxygruppe darstellt und Ar eine gegebenenfalls substituierte aromatische oder heteroaromatische Gruppe darstellt, oder eines ihrer Salze von gegebenenfalls pharmazeutisch akzeptablen Mineral- oder organischen Säuren.

2. Verbindung nach Anspruch 1 der Formel (i), worin die Mineral- oder organischen Säuren pharmazeutisch akzeptabel sind.

3. Verbindung nach Anspruch 2 der Formel (i), worin Ar eine gegebenenfalls substituierte aromatische oder heteroaromatische mono-, di- oder trizyklische Gruppe darstellt, bei der ein Kohlenstoffatom des aromatischen Carbozyklus oder des aromatischen Heterozyklus direkt an den Sauerstoff des Aryloxypropanolaminotetralins gebunden ist.

4. Verbindung nach Anspruch 3 der Formel (i), worin Ar der veresternde Rest in der 3-Stellung eines N-substituierten 1-Amino-2,3-propandiols ist, wobei das N-substituierte Propandiol adrenerge betablockierende Aktivität aufweist.

5. Verbindung nach Anspruch 3 der Formel (iA) oder (iB)

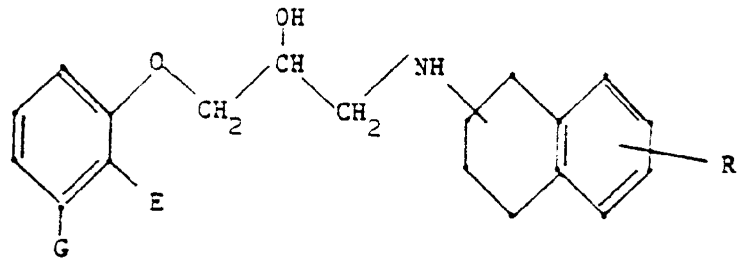

(iA)

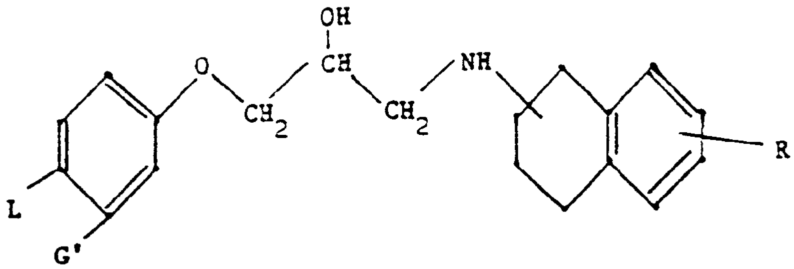

(iB)

worin R wie in Anspruch 1 definiert ist, E Wasserstoff, ein Halogen, eine Alkyl-, Alkenyl-, Alkinyl-, Alkoxy-, Alkenyloxy-, Alkinyloxy-, Tetrahydrofurfuryloxy-, Phenoxy- oder Cycloalkylgruppe mit 3 bis 6 Kohlenstoffatomen, 1-Cyclohexenyl, 2-Cyclohexenyl, 2,5-Methanocyclohexyl, Alkylthio, Alkanoyl, Phenylalkanoyl, Cyano, 3-Chlorallyl, 2-Indolyl, 2-Benzoxazolyl, 2-Benzothiazolyl, 2-Chinolinyl, 2-Chinoxalinyl, 2-Thienyl, 4-Thiazolyl oder 4-Morpholinyl darstellt, G Wasserstoff, ein Halogen, ein Alkyl, ein Alkenyl, eine Trifluormethylgruppe, eine Cyanogruppe oder eine Acetamidogruppe ist, wobei mindestens einer der Substituenten E und G Wasserstoff ist, oder aber E und G eine Acetamidogruppe bzw. eine Nitrogruppe darstellen oder zusammen eine Gruppe $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, $-CH=CH-CH=CH-$, $-CO-CH_2-CH_2-CH_2-$, $-CH_2-CH_2-CH_2-CO-$ ou $-CH_2-CH(OH)-CH(OH)-CH_2-$ bilden; G' Wasserstoff ist, L ein Halogen, eine gegebenenfalls durch eine Alkoxygruppe, Alkoxycarbonylamino, Aminocarbonyl, Cycloalkylalkoxy substituierte Alkylgruppe, eine gegebenenfalls durch eine Cyanogruppe substituierte Alkenylgruppe, eine Alkanoylamino- oder Haloalkanoylaminogruppe, eine durch eine Alkyl- oder Cycloalkylgruppe N'-mono- oder -disubstituierte Ureidogruppe, eine gegebenenfalls am Alkyl durch eine Alkoxygruppe substituierte Alkoxygruppe, eine Cyanogruppe oder eine mit einer Alkoxyalkylgruppe veretherte Hydroxymethylgruppe ist; oder aber G' und L eine Nitrogruppe bzw. eine Acetamidogruppe darstellen; oder aber G' und L zusammen eine Gruppe $-CH=CH-CH=CH-$ bilden; sowie deren Salze von pharmazeutisch akzeptablen Mineral- oder organischen Säuren.

6. Verbindung nach Anspruch 5 der Formel (iA) oder (iB), worin E Wasserstoff, ein Halogen, eine Alkyl-, Alkenyl-, Alkinyl-, Alkoxy-, Alkenyloxy- oder Alkinyloxygruppe darstellt und G Wasserstoff ist, oder aber E und G zusammen eine Gruppe $-CH_2-CH_2-CH_2-$, $-CH_2-CH_2-CH_2-CH_2-$, $-CH=CH-CH_2-$, $-CH_2-CH=CH-$, -

$CH=CH-CH_2-CH_2$-, $-CH=CH-CH=CH-$, $-CO-CH_2-CH_2-CH_2$-, $-CH_2-CH_2-CH_2-CO-$ oder $-CH_2-CH(OH)-CH(OH)-CH_2$- darstellt; G' Wasserstoff darstellt, L ein Chloratom, eine Cyanogruppe oder eine 2-Methoxyethylgruppe darstellt oder aber G' und L zusammen eine Gruppe $-CH=CH-CH=CH-$ bilden.

7. Verbindung nach Anspruch 6 der Formel (iA), worin E ein Halogen, eine Alkyl-, Alkenyl-, Alkoxy-, Alkenyloxy- oder Alkinyloxygruppe darstellt, oder aber E und G zusammen eine Gruppe $-CH=CH-CH=CH$- oder $-CH_2-CH(OH)-CH(OH)-CH_2$- bilden.

8. Verbindung nach Anspruch 7, u.zw. N-(1,2,3,4-Tetrahydronaphth-1-yl)-2-hydroxy-3-(1-naphthyloxy)-propanamin, gegebenenfalls in optisch aktiver Form, oder eines ihrer Salze von pharmazeutisch akzeptablen Mineral- oder organischen Säuren.

9. Verbindung nach Anspruch 8, u.zw. N-[(R)-1,2,3,4-tetrahydronaphth-1-yl)]-(S)-2-hydroxy-3-(1-naphthyloxy)-propanamin oder eines ihrer Salze von pharmazeutisch akzeptablen Mineral- oder organischen Säuren.

10. Verbindung nach Anspruch 7, u.zw. N-(7-Hydroxy-1,2,3,4-tetrahydronaphth-2-yl)-2-hydroxy-[3-(2-allylphenoxy)]-propanamin, gegebenenfalls in optisch aktiver Form, oder eines ihrer Salze von pharmazeutisch akzeptablen Mineral- oder organischen Säuren.

11. Verbindung nach Anspruch 10, u.zw. N-(7-Hydroxy-1,2,3,4-tetrahydronaphth-2-yl)-2-hydroxy-[3-(2-allylphenoxy)]-propanamin-hydrochlorid, gegebenenfalls in optisch aktiver Form.

12. Verbindung nach Anspruch 2 der Formel (iF)

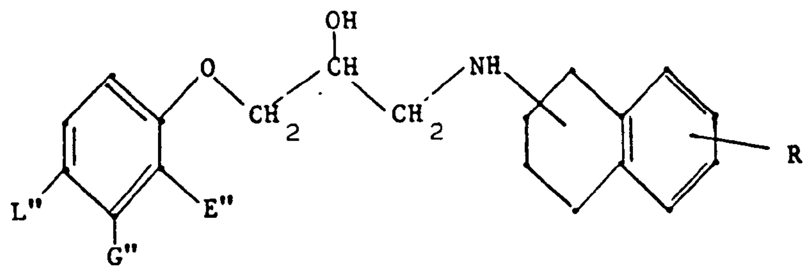

(iF)

worin R wie in Anspruch 1 definiert ist, L" Wasserstoff ist und E" und G" zusammen eine Gruppe $-CH=CH-NH$- bilden, sowie deren Salze von pharmazeutisch akzeptablen Mineral- oder organischen Säuren.

13. Verfahren zur Herstellung einer Verbindung nach Anspruch 1, dadurch gekennzeichnet, daß man ein 1-Aryloxypropan der Formel

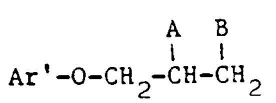

(ii)

worin Ar' die in Anspruch 1 für Ar angegebene Bedeutung hat, aber an $NH_2$- oder NH-Gruppen hängende N-Schutzgruppen enthalten kann, A eine Hydroxygruppe ist, B ein Halogen, ausgewählt aus Chlor, Brom oder Jod, ist, oder aber A und B zusammen eine Epoxygruppe bilden, mit einem Aminotetralin der Formel

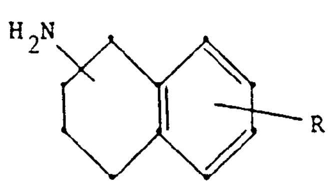

(iii)

worin R wie in Anspruch 1 definiert ist, gegebenenfalls in Gegenwart eines tertiären Amins behandelt; gegebenenfalls das so erhaltene Produkt durch Entfernen der N-Schutzgruppe(n) entschützt; wenn die Ausgangsverbindung die Formel (ii) hat, worin Ar' eine Gruppe 2-Acetamido-3-nitrophenyl oder 4-Acetamido -3-nitrophenyl ist, man gegebenenfalls das erhaltenen Produkt durch Erhitzen in Salzsäure deacetyliert und gegebenenfalls das erhaltene Produkt einer Hydrierung in Gegenwart von 10 % Palladium auf Kohle in 97 %iger Ameisensäure unterzieht, und man gegebenenfalls das so erhaltene Produkt in eines seiner Salze überführt.

**14.** Pharmazeutische Zusammensetzung, die als Wirkstoff eine Verbindung der Formel (i) nach einem der Ansprüche 2 bis 12 enthält.

**15.** Pharmazeutische Zusammensetzung nach Anspruch 14 in Dosiseinheitsform, in der der Wirkstoff mit einem pahrmazeutischen Exzipienten vermischt ist.

**16.** Pharmazeutische Zusammensetzung nach einem der Ansprüche 14 und 15, die 0,1 bis 500 mg Wirkstoff pro Dosiseinheit enthält.

**17.** Reagens zur Differenzierung von betaadrenergen Rezeptoren in biochemischen Versuchen, dadurch gekennzeichnet, daß es eine zweckmäßig markierte Verbindung nach Anspruch 1 enthält.

**Patentansprüche für folgenden Vertragsstaat : ES**

**1.** Verfahren zur Herstellung einer Verbindung der Formel (i)

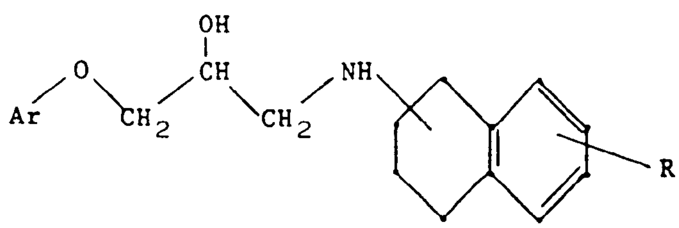

(i)

worin R Wasserstoff, eine Hydroxy- oder eine Methoxygruppe darstellt und Ar eine gegebenenfalls substituierte aromatische oder heteroaromatische Gruppe darstellt, oder eines ihrer Salze von gegebenenfalls pharmazeutisch akzeptablen Mineral- oder organischen Säuren, dadurch gekennzeichnet, daß man ein 1-Aryloxypropan der Formel

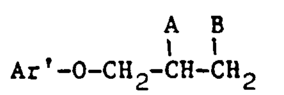

(ii)

worin Ar' die oben für Ar angegebene Bedeutung hat, aber an $NH_2$- oder NH-Gruppen hängende N-Schutzgruppen enthalten kann, A eine Hydroxygruppe ist, B ein Halogen, ausgewählt aus Chlor, Brom oder Jod, ist, oder aber A und B zusammen eine Epoxygruppe bilden, mit einem Aminotetralin der Formel

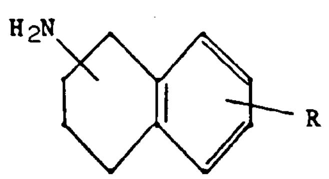

(iii)

worin R wie oben definiert ist, gegebenenfalls in Gegenwart eines tertiären Amins behandelt; gegebenenfalls das so erhaltene Produkt durch Entfernen der N-Schutzgruppe(n) entschützt; wenn die Ausgangsverbindung die Formel (ii) hat, worin Ar' eine Gruppe 2-Acetamido-3-nitrophenyl oder 4-Acetamido-3-nitrophenyl ist, man gegebenenfalls das erhaltenen Produkt durch Erhitzen in Salzsäure deacetyliert

und gegebenenfalls das erhaltene Produkt einer Hydrierung in Gegenwart von 10 % Palladium auf Kohle in 97 %iger Ameisensäure unterzieht, und man gegebenenfalls das so erhaltene Produkt in eines seiner Salze überführt.

2. Verfahren nach Anspruch 1, dadurch gekennzeichnet, daß das 1-Aryloxypropan der Formel (ii) und das Aminotetralin der Formel (iii) zur Herstellung einer Verbindung der Formel (i), in welcher R wie in Anspruch 1 definiert ist und Ar eine gegebenenfalls substituierte aromatische oder heteroaromatische mono-, di- oder trizyklische Gruppe darstellt, bei der ein Kohlenstoffatom des aromatischen Carbozyklus oder des aromatischen Heterozyklus direkt an den Sauerstoff des Aryloxypropanolaminotetralins gebunden ist; oder eines ihrer Salze von Mineral- oder organischen Säuren ausgewählt sind.

3. Verfahren nach Anspruch 2, dadurch gekennzeichnet, daß das 1-Aryloxypropan der Formel (ii) und das Aminotetralin der Formel (iii) zur Herstellung einer Verbindung der Formel (iA) oder (iB)

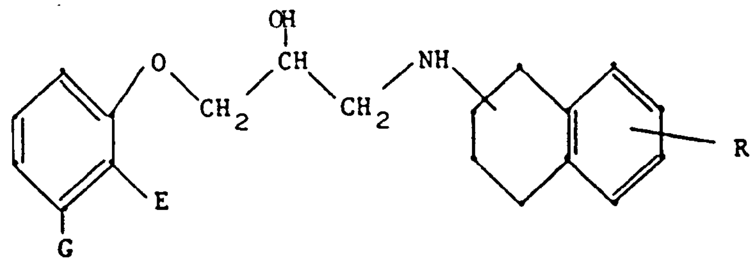

(iA)

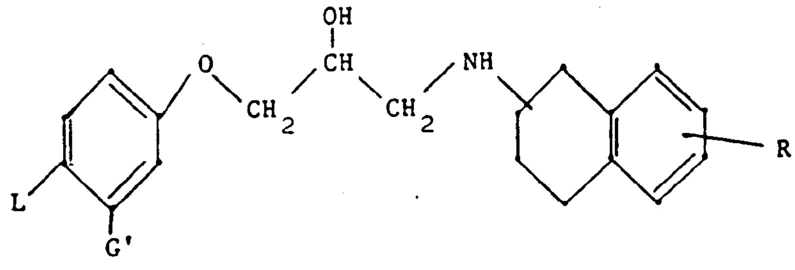

(iB)

worin R wie in Anspruch 1 definiert ist, E Wasserstoff, ein Halogen, eine Alkyl-, Alkenyl-, Alkinyl-, Alkoxy-, Alkenyloxy-, Alkinyloxy-, Tetrahydrofurfuryloxy-, Phenoxy- oder Cycloalkylgruppe mit 3 bis 6 Kohlenstoffatomen, 1-Cyclohexenyl, 2-Cyclohexenyl, 2,5-Methanocyclohexyl, Alkylthio, Alkanoyl, Phenylalkanoyl, Cyano, 3-Chlorallyl, 2-Indolyl, 2-Benzoxazolyl, 2-Benzothiazolyl, 2-Chinolinyl, 2-Chinoxalinyl, 2-Thienyl, 4-Thiazolyl oder 4-Morpholinyl darstellt, G Wasserstoff, ein Halogen, ein Alkyl, ein Alkenyl, eine Trifluormethylgruppe, eine Cyanogruppe oder eine Acetamidogruppe ist, wobei mindestens einer der Substituenten E und G Wasserstoff ist, oder aber E und G eine Acetamidogruppe bzw. eine Nitrogruppe darstellen oder zusammen eine Gruppe $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, $-CH=CH-CH=CH-$, $-CO-CH_2-CH_2-CH_2-$, $-CH_2-CH_2-CH_2-CO-$ oder $-CH_2-CH(OH)-CH(OH)-CH_2-$ bilden; G' Wasserstoff ist, L ein Halogen, eine gegebenenfalls durch eine Alkoxygruppe, Alkoxycarbonylamino, Aminocarbonyl, Cycloalkylalkoxy substituierte Alkylgruppe, eine gegebenenfalls durch eine Cyanogruppe substituierte Alkenylgruppe, eine Alkanoylamino- oder Haloalkanoylaminogruppe, eine durch eine Alkyl- oder Cycloalkylgruppe N'-mono- oder -disubstituierte Ureidogruppe, eine gegebenenfalls am Alkyl durch eine Alkoxygruppe substituierte Alkoxygruppe, eine Cyanogruppe oder eine mit einer Alkoxyalkylgruppe veretherte Hydroxymethylgruppe ist; oder aber G' und L eine Nitrogruppe bzw. eine Acetamidogruppe darstellen; oder aber G' und L zusammen eine Gruppe -CH=CH-CH=CH- bilden; sowie von deren Salzen von pharmazeutisch akzeptablen Mineral- oder organischen Säuren ausgewählt sind.

4. Verfahren nach Anspruch 3, dadurch gekennzeichnet, daß das 1-Aryloxypropan der Formel (ii) und das Aminotetralin der Formel (iii) zur Herstellung einer Verbindung der Formel (iA) oder (iB), worin E Wasserstoff, ein Halogen, eine Alkyl-, Alkenyl-, Alkinyl-, Alkoxy-, Alkenyloxy- oder Alkinyloxygruppe darstellt und G Wasserstoff ist, oder aber E und G zusammen eine Gruppe $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, $-CH=CH-CH=CH-$, $-CO-CH_2-CH_2-CH_2-$, $-CH_2-CH_2-$

$CH_2$-CO- oder -$CH_2$-CH(OH)-CH(OH)-$CH_2$- darstellen; G' Wasserstoff darstellt, L ein Chloratom, eine Cyanogruppe oder eine 2-Methoxyethylgruppe darstellt oder aber G' und L zusammen eine Gruppe -CH=CH-CH=CH- bilden, oder eines ihrer Mineral- oder organischer Salze ausgewählt sind.

5. Verfahren nach Anspruch 4, dadurch gekennzeichnet, daß das 1-Aryloxypropan der Formel (ii) und das Aminotetralin der Formel (iii) zur Herstellung einer Verbindung der Formel (iA) oder (iB), worin E ein Halogen, eine Alkyl-, Alkenyl-, Alkoxy-, Alkenyloxy- oder Alkinyloxygruppe darstellt, oder aber E und G zusammen eine Gruppe -CH=CH-CH=CH- oder -$CH_2$-CH(OH)-CH(OH)-$CH_2$- bilden, oder eines ihrer Salze von Mineral- oder organischen Säuren ausgewählt sind.

6. Verfahren nach Anspruch 5, dadurch gekennzeichnet, daß das 1-Aryloxypropan der Formel (ii) und das Aminotetralin der Formel (iii) zur Herstellung von N-(1,2,3,4-Tetrahydronaphth-1-yl)-2-hydroxy-3-(1-naphthyloxy)-propanamin, gegebenenfalls in optisch aktiver Form, oder eines seiner Salze von Mineral- oder organischen Säuren ausgewählt sind.

7. Verfahren nach Anspruch 6, dadurch gekennzeichnet, daß das das 1-Aryloxypropan der Formel (ii) und das Aminotetralin der Formel (iii) zur Herstellung von N-[(R)-1,2,3,4-tetrahydronaphth-1-yl)]-(S)-2-hydroxy-3-(1-naphthyloxy)-propanamin oder eines seiner Salze von Mineral- oder organischen Säuren ausgewählt sind.

8. Verfahren nach Anspruch 5, dadurch gekennzeichnet, daß das 1-Aryloxypropan der Formel (ii) und das Aminotetralin der Formel (iii) zur Herstellung von N-(7-Hydroxy-1,2,3,4-tetrahydronaphth-2-yl)-2-hydroxy-[3-(2-allylphenoxy)]-propanamin, gegebenenfalls in optisch aktiver Form, oder eines seiner Salze von Mineral- oder organischen Säuren ausgewählt sind.

9. Verfahren nach Anspruch 8, dadurch gekennzeichnet, daß das 1-Aryloxypropan der Formel (ii) und das Aminotetralin der Formel (iii) zur Herstellung von N-(7-Hydroxy-1,2,3,4-tetrahydronaphth -2-yl)-2-hydroxy-[3-(2-allylphenoxy)]-propanamin-hydrochlorid, gegebenenfalls in optisch aktiver Form, ausgewählt sind.

10. Verfahren nach Anspruch 1, dadurch gekennzeichnet, daß das das 1-Aryloxypropan der Formel (ii) und das Aminotetralin der Formel (iii) zur Herstellung einer Verbindung der Formel (iF)

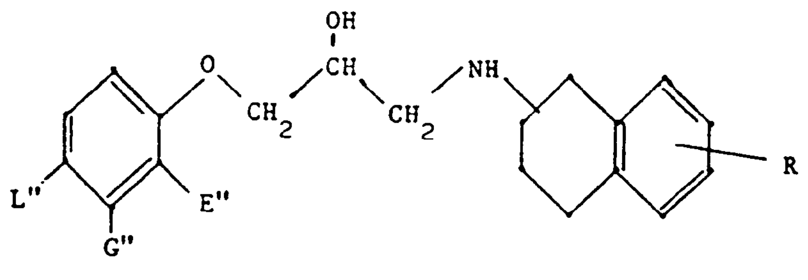

(iF)

worin R wie in Anspruch 1 definiert ist, L" Wasserstoff ist und E" und G" zusammen eine Gruppe -CH=CH-NH- bilden, oder eines ihrer Salze von Mineral- oder organischen Säuren ausgewählt sind.

11. Verfahren zur Herstellung von pharmazeutischen Zusammensetzungen, dadurch gekennzeichnet, daß es darin besteht, daß als Wirkstoff eine Verbindung der Formel (i), hergestellt nach einem der Ansprüche 1 bis 10, oder eines ihrer pharmazeutisch akzeptablen Salze mit einem pharmazeutischen Exzipienten vermischt wird.

12. Verfahren nach Anspruch 11 zur Herstellung von pharmazeutischen Zusammensetzungen in Dosiseinheitsform.

13. Verfahren nach einem der Ansprüche 11 oder 12 zur Herstellung von pharmazeutischen Zusammensetzungen, die 0,1 bis 500 mg Wirkstoff pro Dosiseinheit enthalten.

14. Reagens zur Differenzierung von betaadrenergen Rezeptoren in biochemischen Versuchen, dadurch gekennzeichnet, daß es eine zweckmäßig markierte Verbindung der Formel (i)

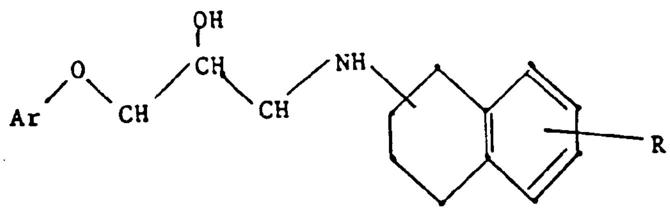

(i)

worin R und Ar wie in Anspruch 1 definiert sind, enthält.

**Patentansprüche für folgenden Vertragsstaat : GR**

1. Verbindung der Formel (i)

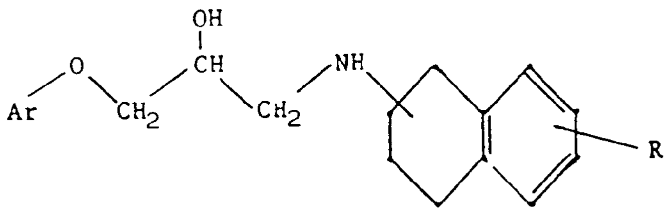

(i)

worin R Wasserstoff, eine Hydroxy- oder eine Methoxygruppedarstellt und Ar eine gegebenenfalls substituierte aromatische oder heteroaromatische Gruppe darstellt, oder eines ihrer Salze von gegebenenfalls pharmazeutisch akzeptablen Mineral- oder organischen Säuren.

2. Verbindung nach Anspruch 1 der Formel (i), worin die Mineral- oder organischen Säuren pharmazeutisch akzeptabel sind.

3. Verbindung nach Anspruch 2 der Formel (i), worin Ar eine gegebenenfalls substituierte aromatische oder heteroaromatische mono-, di- oder trizyklische Gruppe darstellt, bei der ein Kohlenstoffatom des aromatischen Carbozyklus oder des aromatischen Heterozyklus direkt an den Sauerstoff des Aryloxypropanolaminotetralins gebunden ist.

4. Verbindung nach Anspruch 3 der Formel (i), worin Ar der veresternde Rest in der 3-Stellung eines N-substituierten 1-Amino-2,3-propandiols ist, wobei das N-substituierte Propandiol adrenerge betablockierende Aktivität aufweist.

5. Verbindung nach Anspruch 3 der Formel (iA) oder (iB)

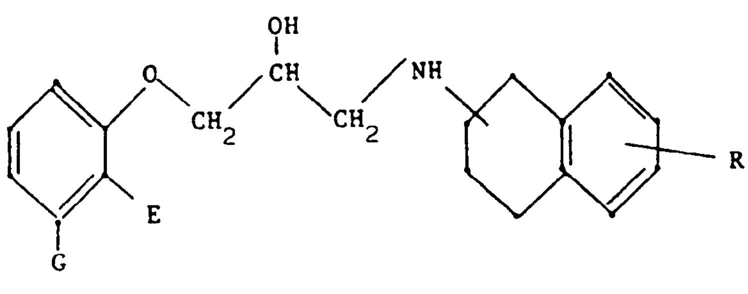

(iA)

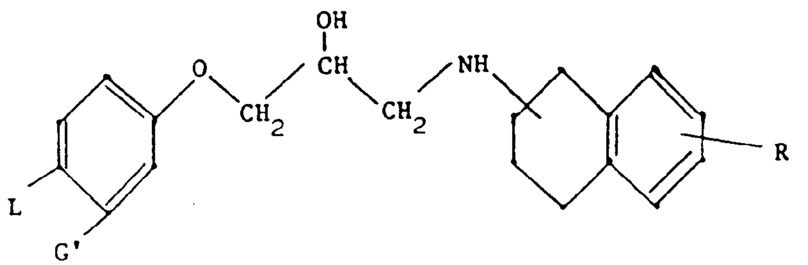

(iB)

worin R wie in Anspruch 1 definiert ist, E Wasserstoff, ein Halogen, eine Alkyl-, Alkenyl-, Alkinyl-, Alkoxy-, Alkenyloxy-, Alkinyloxy-, Tetrahydrofurfuryloxy-, Phenoxy- oder Cycloalkylgruppe mit 3 bis 6 Kohlenstoffatomen, 1-Cyclohexenyl, 2-Cyclohexenyl, 2,5-Methanocyclohexyl, Alkylthio, Alkanoyl, Phenylalkanoyl, Cyano, 3-Chlorallyl, 2-Indolyl, 2-Benzoxazolyl, 2-Benzothiazolyl , 2-Chinolinyl, 2-Chinoxalinyl, 2-Thienyl, 4-Thiazolyl oder 4-Morpholinyl darstellt, G Wasserstoff, ein Halogen, ein Alkyl, ein Alkenyl, eine Trifluormethylgruppe, eine Cyanogruppe oder eine Acetamidogruppe ist, wobei mindestens einer der Substituenten E und G Wasserstoff ist, oder aber E und G eine Acetamidogruppe bzw. eine Nitrogruppe darstellen oder zusammen eine Gruppe $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, -CH=CH-CH=CH-, $-CO-CH_2-CH_2-CH_2-$, $-CH_2-CH_2-CH_2-CO-$ oder $-CH_2-CH(OH)-CH(OH)-CH_2-$ bilden; G' Wasserstoff ist, L ein Halogen, eine gegebenenfalls durch eine Alkoxygruppe, Alkoxycarbonylamino, Aminocarbonyl, Cycloalkylalkoxy substituierte Alkylgruppe, eine gegebenenfalls durch eine Cyanogruppe substituierte Alkenylgruppe, eine Alkanoylamino- oder Haloalkanoylaminogruppe, eine durch eine Alkyl- oder Cycloalkylgruppe N'-mono- oder -disubstituierte Ureidogruppe, eine gegebenenfalls am Alkyl durch eine Alkoxygruppe substituierte Alkoxygruppe, eine Cyanogruppe oder eine mit einer Alkoxyalkylgruppe veretherte Hydroxymethylgruppe ist; oder aber G' und L eine Nitrogruppe bzw. eine Acetamidogruppe darstellen; oder aber G' und L zusammen eine Gruppe -CH=CH-CH=CH- bilden; sowie deren Salze von pharmazeutisch akzeptablen Mineral- oder organischen Säuren.

6. Verbindung nach Anspruch 5 der Formel (iA) oder (iB), worin E Wasserstoff, ein Halogen, eine Alkyl-, Alkenyl-, Alkinyl-, Alkoxy-, Alkenyloxy- oder Alkinyloxygruppe darstellt und G Wasserstoff ist, oder aber E und G zusammen eine Gruppe $-CH_2-CH_2-CH_2-$, $-CH_2-CH_2-CH_2-CH_2-$, $-CH=CH-CH_2-$, $-CH_2-CH=CH-$, $-CH-CH-CH_2-CH_2-$, -CH-CH-CH=CH-, $-CO-CH_2-CH_2-CH_2-$, $-CH_2-CH_2-CH_2-CO-$ oder $-CH_2-CH(OH)-CH(OH)-CH_2-$ darstellt; G' Wasserstoff darstellt, L ein Chloratom, eine Cyanogruppe oder eine 2-Methoxyethylgruppe darstellt oder aber G' und L zusammen eine Gruppe -CH=CH-CH=CH- bilden.

7. Verbindung nach Anspruch 6 der Formel (iA), worin E ein Halogen, eine Alkyl-, Alkenyl-, Alkoxy-, Alkenyloxy- oder Alkinyloxygruppe darstellt, oder aber E und G zusammen eine Gruppe -CH=CH-CH=CH- oder $-CH_2-CH(OH)-CH(OH)-CH_2-$ bilden.

8. Verbindung nach Anspruch 7, u.zw. N-(1,2,3,4-Tetrahydronaphth-1-yl)-2-hydroxy-3-(1-naphthyloxy)-propanamin, gegebenenfalls in optisch aktiver Form, oder eines ihrer Salze von pharmazeutisch akzeptablen Mineral- oder organischen Säuren.

9. Verbindung nach Anspruch 8, u.zw. N-[(R)-1,2,3,4-tetrahydronaphth-1-yl)]-(S)-2-hydroxy-3-(1-naphthyloxy)-propanamin oder eines ihrer Salze von pharmazeutisch akzeptablen Mineral- oder organischen Säuren.

10. Verbindung nach Anspruch 7, u.zw. N-(7-Hydroxy-1,2,3,4-tetrahydronaphth-2-yl)-2-hydroxy-[3-(2-allylphenoxy)]-propanamin, gegebenenfalls in optisch aktiver Form, oder eines ihrer Salze von pharmazeutisch akzeptablen Mineral- oder organischen Säuren.

11. Verbindung nach Anspruch 10, u.zw. N-(7-Hydroxy-1,2,3,4-tetrahydronaphth-2-yl)-2-hydroxy-[3-(2-allylphenoxy)]-propanamin-hydrochlorid, gegebenenfalls in optisch aktiver Form.

12. Verbindung nach Anspruch 2 der Formel (iF)

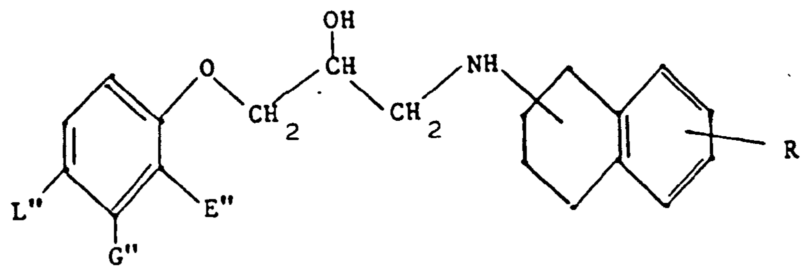

(1F)

worin R wie in Anspruch 1 definiert ist, L" Wasserstoff ist und E" und G" zusammen eine Gruppe -CH=CH-NH- bilden, sowie deren Salze von pharmazeutisch akzeptablen Mineral- oder organischen Säuren.

**13.** Verfahren zur Herstellung einer Verbindung nach Anspruch 1, dadurch gekennzeichnet, daß man ein 1-Aryloxypropan der Formel

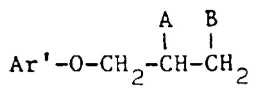

(ii)

worin Ar' die in Anspruch 1 für Ar angegebene Bedeutung hat, aber an $NH_2$- oder NH-Gruppen hängende N-Schutzgruppen enthalten kann, A eine Hydroxygruppe ist, B ein Halogen, ausgewählt aus Chlor, Brom oder Jod, ist, oder aber A und B zusammen eine Epoxygruppe bilden, mit einem Aminotetralin der Formel

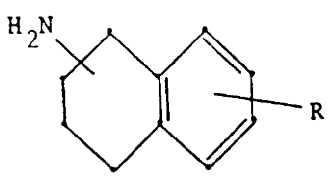

(iii)

worin R wie in Anspruch 1 definiert ist, gegebenenfalls in Gegenwart eines tertiären Amins behandelt; gegebenenfalls das so erhaltene Produkt durch Entfernen der N-Schutzgruppe(n) entschützt; wenn die Ausgangsverbindung die Formel (ii) hat, worin Ar' eine Gruppe 2-Acetamido-3-nitrophenyl oder 4-Acetamido -3-nitrophenyl ist, man gegebenenfalls das erhaltenen Produkt durch Erhitzen in Salzsäure deacetyliert und gegebenenfalls das erhaltene Produkt einer Hydrierung in Gegenwart von 10 % Palladium auf Kohle in 97 %iger Ameisensäure unterzieht, und man gegebenenfalls das so erhaltene Produkt in eines seiner Salze überführt.

**14.** Verfahren zur Herstellung von pharmazeutischen Zusammensetzungen, dadurch gekennzeichnet, daß es darin besteht, daß als Wirkstoff eine Verbindung der Formel (i), hergestellt nach einem der Ansprüche 1 bis 12, oder eines ihrer pharmazeutisch akzeptablen Salze mit einem pharmazeutischen Exzipienten vermischt wird.

**15.** Verfahren nach Anspruch 14 zur Herstellung von pharmazeutischen Zusammensetzungen in Dosiseinheitsform.

**16.** Verfahren nach einem der Ansprüche 14 oder 15 zur Herstellung von pharmazeutischen Zusammensetzungen, die 0,1 bis 500 mg Wirkstoff pro Dosiseinheit enthalten.

**17.** Reagens zur Differenzierung von betaadrenergen Rezeptoren in biochemischen Versuchen, dadurch gekennzeichnet, daß es eine zweckmäßig markierte Verbindung nach Anspruch 1 enthält.

## Claims

### Claims for the following Contracting States : AT, BE, CH, DE, FR, GB, LI, LU, NL, SE

1. Compound of formula (i)

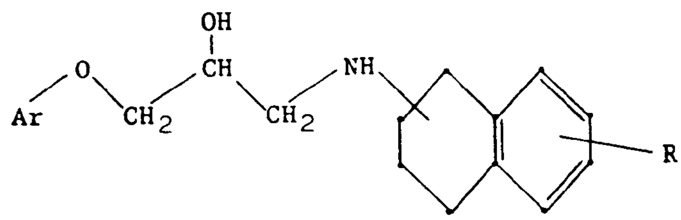

(i)

wherein R is hydrogen, a hydroxy group or methoxy group, and Ar represents an optionally substituted aromatic or heteroaromatic group, or one of its mineral or organic acid salts whether pharmaceutically acceptable or not.

2. Compound according to claim 1, of formula (i), wherein the mineral or organic acids are pharmaceutically acceptable.

3. Compound according to claim 2, of formula (i), wherein Ar represents an optionally substituted mono-, di- or tri-cyclic aromatic or heteroaromatic group, of which a carbon atom of the aromatic carbocycle or of the aromatic heterocycle is directly linked to the oxygen of the aryloxypropanolaminotetralin.

4. Compound according to claim 3, of formula (i) wherien Ar is the etherifying residue in the 3-position of an N-substituted 1-amino-2,3- propandiol, having a beta-blocking adrenergic activity.

5. Compound according to claim 3 of formula (iA) or (iB)

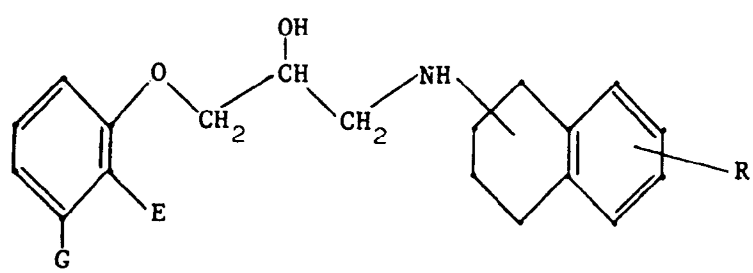

(iA)

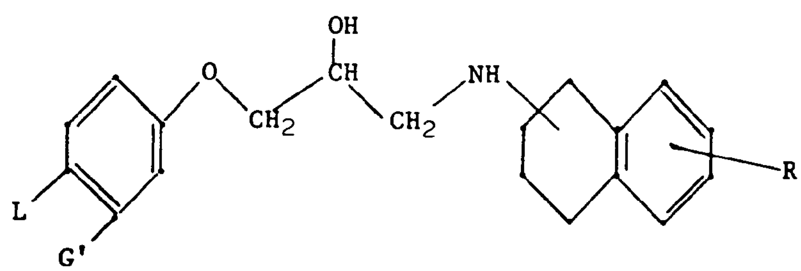

(iB)

wherein R is such as defined in claim 1, E represents hydrogen, a halogen, an alkyl, alkenyl, alkynyl, alkoxy, alkenyloxy, alkynyloxy, tetrahydrofurfuryloxy, phenoxy, cycloalkyl group of from 3 to 6 carbon atoms, 1-cyclohexenyl, 2-cyclohexenyl, 2,5-methano-cyclohexyl, alkylthio, alkanoyl, phenylalkanoyl, cyano, 3-chloroallyl, 2-indolyl, 2-benzoxazolyl, 2-benzothiazolyl, 2-quinolinyl, 2-quinoxalinyl, 2-thienyl, 4-thiazolyl,

4-morpholinyl; G is hydrogen, a halogen, an alkyl, an alkenyl, a trifluoromethyl group, a cyano group or an acetamido group, with at least one of the substituents E and G being hydrogen; or E and G represent, respectively, an acetamido group and a nitro group, or E and G taken together form a group $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, $-CH=CH-CH=CH-$, $-CO-CH_2-CH_2-CH_2-$, $-CH_2-CH_2-CH_2-CO-$ or $-CH_2-CH(OH)-CH(OH)-CH_2-$; G' is hydrogen; L is halogen, an alkyl group non-substituted or substituted with an alkoxy, alkoxycarbonylamino, aminocarbonyl or cycloalkylalkoxy group, an alkenyl group, non-substituted or substituted by a cyano group; an alkanoylamino or haloalkanoylamino group; a ureido N'-mono or di-substituted with alkyl or cycloalkyl groups; an alkoxy group optionally substituted on the alkyl by an alkoxy group; a cyano group; or a hydroxymethyl group etherified with an alkoxyalkyl group; or G' and L represent, respectively, a nitro group and an acetamido group, or G' and L, taken together for a group -CH=CH-CH=CH-; as well as its pharmaceutically acceptable mineral or organic acid salts.

6. Compound according to claim 5, of formula (iA) or (iB), wherein E is hydrogen, halogen, an alkyl, alkenyl, alkynyl, alkoxy, alkenyloxy, alkynyloxy and G is hydrogen, or E and G, taken together, form a group $-CH_2-CH_2-CH_2-$; $-CH_2-CH_2-CH_2-CH_2-$; $-CH=CH-CH_2-$; $-CH_2-CH=CH-$, $-CH=CH-CH_2-CH_2-$, -CH=CH-CH=CH-, $-CO-CH_2-CH_2-CH_2-$; $-CH_2-CH_2-CH_2-CO-$ or $-CH_2-CH(OH)-CH(OH)-CH_2-$; G' represents hydrogen, L represents an atom of chloride, a cyano group or a 2-methoxyethyl group or G' and L, taken together form a group -CH=CH-CH=CH-.

7. Compound according to claim 6, of formula (iA), wherein E is halogen, an alkyl, alkenyl, alkoxy, alkenyloxy or alkynyloxy group, or E and G, taken together, form a group -CH=CH-CH=CH- or $-CH_2-CH(OH)-CH(OH)-CH_2-$.

8. Compound according to claim 7 which is N(1,2,3,4-tetrahydronaphth-1-yl)-2-hydroxy-3-(1-naphtyloxy)-propanamine in optically active or inactive form, or one of its pharmaceutically acceptable mineral or organic acid salts.

9. Compound according to claim 7 which is N-[(R)-1,2,3,4-tetrahydronaphth-1-yl)]-(S)-2-hydroxy-3-(1-naphthyloxy)propanamine or one of its pharmaceutically acceptable mineral or organic acid salts.

10. Compound according to claim 7 which is N-(7-hydroxy-1,2,3,4-tetrahydronaphth-2-yl)-2-hydroxy-[3-(2-allylphenoxy)]propanamine in optically active or inactive form or one of its pharmaceutically acceptable mineral or organic acid salts.

11. Compound according to claim 10 which is N-(7-hydroxy-1,2,3,4-tetrahydronaphth-2-yl)-2-hydroxy-[3-(2-allylphenoxy)]propanamine hydrochloride, in optically active or inactive form.

12. Compound according to claim 2, of formula (iF)

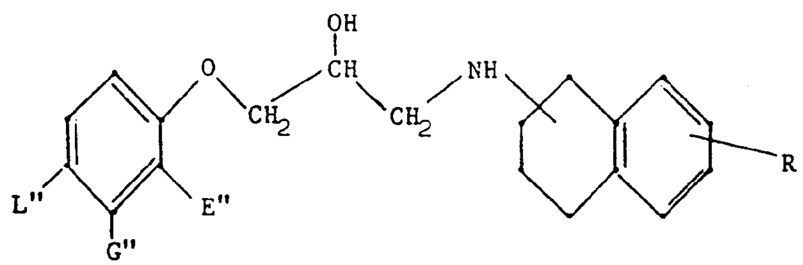

(iF)

wherein R is as defined in claim 1, L" is hydrogen and E" and G", taken together form a -CH=CH-NH- group, as well as its pharmaceutically acceptable mineral or organic acid salts.

13. Process for preparing a compound according to claim 1, characterized in that it comprises treating a 1-aryloxypropane of formula (ii)

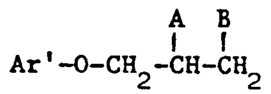

(ii)

wherein Ar' has the same meaning as that given in claims 1 to Ar, although it may contain N-protecting groups fixed on $NH_2$ or NH groups, A is a hydroxy group and B is a halogen selected from chlorine, bromine or iodine, or A and B, taken together form an epoxy group, with an aminotetralin of formula (iii)

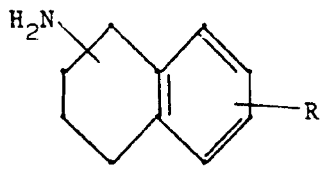

(iii)

wherein R is as defined in claim 1, optionally in the presence of a tertiary amine; optionally deprotecting the obtained product by removing the N-protecting group or groups; when the starting compound of formula (ii) in which Ar' is a 2-acetamido-3-nitrophenyl or 4-acetamido-3-nitrophenyl group, optionally deacetylating the obtained product by heating it in hydrochloric acid and optionally hydrogenating the obtained product in 97% formic acid in the presence of 10% Pd/C; and optionally converting the obtained product in one of its salts.

**14.** Pharmaceutical composition containing as the active ingredient, a compound of formula (i) according to any one of claims 2 to 12.

**15.** Pharmaceutical composition according to claim 14, in dosage unit form, wherein the active ingredient is mixed with a pharmaceutically acceptable carrier.

**16.** Pharmaceutical composition according to one of claims 14 and 15, containing from 0.1 to 500 mg of active ingredient per dosage unit.

**17.** Reagent for differentiating between the beta-adrenergic receptors in biochemical assays, characterized in that it comprises a suitably labelled compound according to claim 1.

**Claims for the following Contracting State : ES**

**1.** Process for preparing a compound of formula (i)

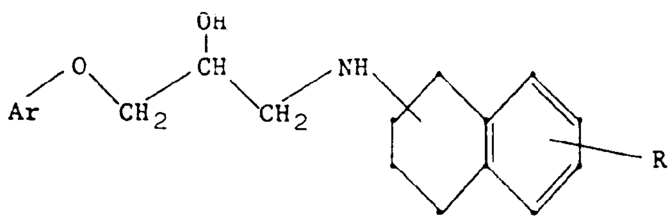

(i)

wherein R is hydrogen, a hydroxy group or methoxy group, and Ar represents an optionally substituted aromatic or heteroaromatic group, and of its mineral or organic acid salts whether pharmaceutically acceptable or not, characterized in that it comprises treating a 1-aryloxypropane of formula

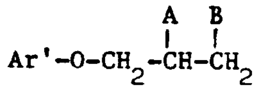

(ii)

wherein Ar' has the same meaning as that given hereinabove to Ar, although it may contain N-protecting groups fixed on $NH_2$ or NH groups, A is a hydroxy group and B is a halogen selected from chlorine, bromine or iodine, or A and B, taken together form an epoxy group, with an aminotetralin of formula

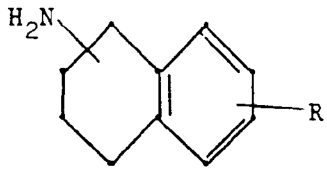

(iii)

wherein R is as defined above, optionally in the presence of a tertiary amine; optionally deprotecting the obtained product by removing the N-protecting group or groups; when the starting compound of formula (ii) in which Ar' is a 2-acetamido-3-nitrophenyl or 4-acetamido-3-nitrophenyl group, optionally deacetylating the obtained product by heating it in hydrochloric acid and optionally hydrogenating the obtained product in 97% formic acid in the presence of 10% Pd/C; and optionally converting the obtained product in one of its salts.

2. Process according to claim 1, characterized in that the 1-aryloxypropane of formula (ii) and the aminotetralin of formula (iii) are selected for preparing a compound of formula (i) wherein Ar represents an optionally substituted mono-, di- or tri-cyclic aromatic or heteroaromatic group, of which a carbon atom of the aromatic carbocycle or of the aromatic heterocycle is directly linked to the oxygen of the aryloxypropanolaminotetralin; or one of its mineral or organic acid salts.

3. Process according to claim 2, characterized in that the 1-aryloxypropane of formula (ii) and the aminotetralin of formula (iii) are selected for preparing a compound of formula (iA) or (iB)

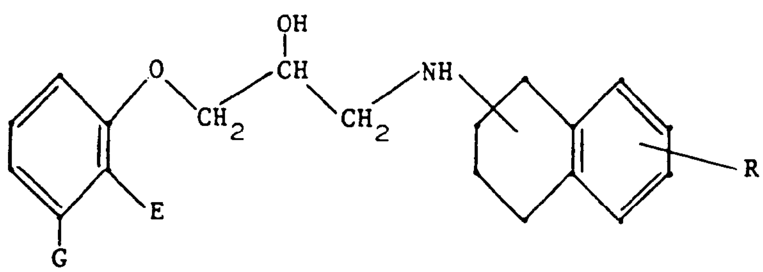

(iA)

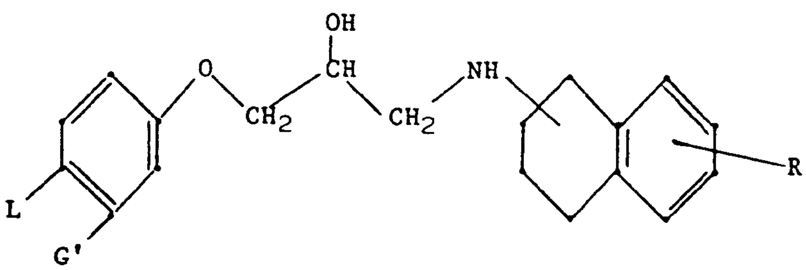

(iB)

wherein R is such as defined in claim 1, E represents hydrogen, a halogen, an alkyl, alkenyl, alkynyl, alkoxy, alkenyloxy, alkynyloxy, tetrahydrofurfuryloxy, phenoxy, cycloalkyl group of from 3 to 6 carbon atoms, 1-cyclohexenyl, 2-cyclohexenyl, 2,5-methano-cyclohexyl, alkylthio, alkanoyl, phenylalkanoyl, cyano, 3-chloroallyl, 2-indolyl, 2-benzoxazolyl, 2-benzothiazolyl, 2-quinolinyl, 2-quinoxalinyl, 2-thienyl, 4-thiazolyl, 4-morpholinyl; G is hydrogen, a halogen, an alkyl, an alkenyl, a trifluoromethyl group, a cyano group or an acetamido group, with at least one of the substituents E and G being hydrogen; or E and G represent, respectively, an acetamido group and a nitro group, or E and G taken together form a group $-CH_2-CH_2-CH_2-$, $-CH_2-CH_2-CH_2-CH_2-$, $-CH=CH-CH_2-$, $-CH_2-CH=CH-$, $-CH=CH-CH_2-CH_2-$, $-CH=CH-CH=CH-$, $-CO-CH_2-CH_2-CH_2-$, $-CH_2-CH_2-CH_2-CO-$ or $-CH_2-CH(OH)-CH(OH)-CH_2-$; G' is hydrogen; L is halogen, an alkyl group non-substituted or substituted with an alkoxy, alkoxycarbonylamino, aminocarbonyl or cycloalkylalkoxy group, an alkenyl group, non-substituted or substituted by a cyano; an alkanoylamino or haloalkanoylamino group; a ureido N'-mono or di-substituted with alkyl or cycloalkyl groups; an alkoxy group optionally substituted on the alkyl by an alkoxy group; a cyano group; or a hydroxymethyl group etherified with an alkoxyalkyl group; or G' and L represent, respectively, a nitro group and an acetamido group, or G' and L, taken together for a group $-CH=CH-CH=CH-$; or one of its pharmaceutically acceptable mineral or organic acid salts.

4. Process according to claim 3, characterized in that the 1-aryloxypropane of formula (ii) and the amininotetralin of formula (iii) are selected for preparing a compound of formula (iA) or (iB), wherein E is hydrogen, halogen, an alkyl, alkenyl, alkynyl, alkoxy, alkenyloxy, alkynyloxy and G is hydrogen, or E and G, taken together, form a group $-CH_2-CH_2-CH_2-$; $-CH_2-CH_2-CH_2-CH_2-$; $-CH=CH-CH_2-$; $-CH_2-CH=CH-$, $-CH=CH-CH_2-CH_2-$, $-CH=CH-CH=CH-$, $-CO-CH_2-CH_2-CH_2-$; $-CH_2-CH_2-CH_2-CO-$ or $-CH_2-CH(OH)-CH(OH)-CH_2-$; G' represents hydrogen, L represents an atom of chloride, a cyano group or a 2-methoxyethyl group or G' and L, taken together form a group $-CH=CH-CH=CH-$; or one of its mineral or organic acid salts.

5. Process according to claim 4, characterized in that the 1-aryloxypropane of formula (ii) and the aminitetralin of formula (iii) are selected for preparing a compound of formula (iA) wherein E is halogen, an alkyl, alkenyl, alkoxy, alkenyloxy or alkynyloxy group, or E and G taken together, for a group $-CH=CH-CH=CH-$ or $-CH_2-CH(OH)-CH(OH)-CH_2$, or one of its mineral or organic acid salts.

6. Process according to claim 5, characterized in that the 1-aryloxypropane of formula (ii) and the aminotetralin of formula (iii) are selected for preparing of N-(1,2,3,-4-tetrahydronaphth-1-yl)-2-hydroxy-3-(1-naphthyloxy)-propanamine in optically active or inactive form, or one of its mineral or organic acid salts.

7. Process according to claim 6, characterized in that the 1-aryloxypropane of formula (ii) and the aminotetralin of formula (iii) are selected for preparing N-[(R)-1,2,3,4-tetrahydronaphth-1-yl)]-(S)-2-hydroxy-3-(1-naphthyloxy)propanamine or one of its mineral or organic acid salts.

8. Process according to claim 5, characterized in that the 1-aryloxypropane of formula (ii) and the aminotetralin of formula (iii) are selected for preparing N-(7-hydroxy-1,2,3,4-tetrahydronaphth-2-yl)-2-hydroxy-[3-(2-allylphenoxy)]propanamine in optically active or inactive form or one of its mineral or organic acid salts.

9. Process according to claim 8, characterized in that the 1-aryloxypropane of formula (ii) and the aminotetraline of formula (iii) are selected for preparing N-(7-hydroxy-1,2,3,4-tetrahydronaphth-2-yl)-2-hydroxy-[3-(2-allylphenoxy)]propanamine hydrochloride, in optically active or inactive form.

10. Process according to claim 1, characterized in that the 1-aryloxypropane of formula (ii) and the aminotetraline of formula (iii) are selected for preparing a compound of formula (iF)

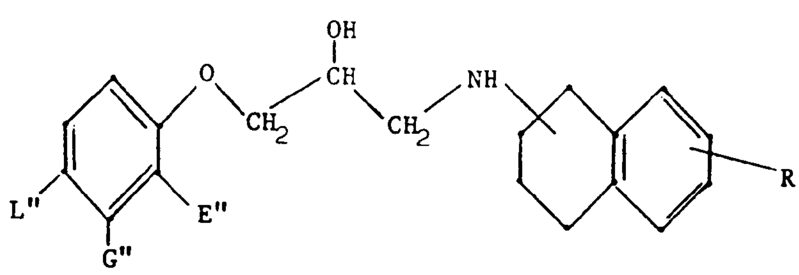

(iF)

wherein R is as defined in claim 1, L" is hydrogen and E" and G", taken together form a -CH=CH-NH-

group, or one of its organic or mineral salts.

**11.** Process for preparing pharmaceutical compositions, characterized in that it consists in mixing as active ingredient, a compound of formula (i) prepared according to any one of claims 1 to 10 or one of its pharmaceutically acceptable salts, with a pharmaceutical carrier.

**12.** Process according to claim 11, for preparing pharmaceutical compositions in dosage unit form.

**13.** Process according to one of claims 11 or 12, for preparing pharmaceutical compositions containing from 0.1 to 500 mg of active ingredient per dosage unit.

**14.** Reagent for differentiating between the beta-adrenergic receptors in biochemical assays, characterized in that it comprises a suitably labelled compound of formula (i)

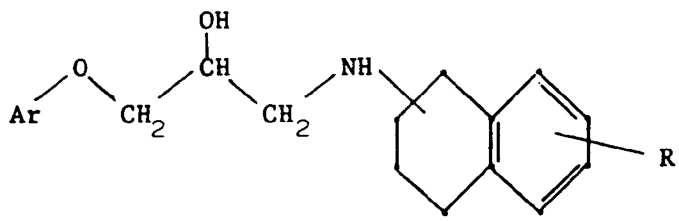

(i)

in which R and Ar are as defined in claim 1.

**Claims for the following Contracting State : GR**

**1.** Compound of formula (i)

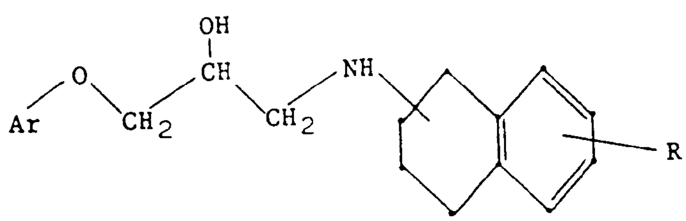

(i)

wherein R is hydrogen, a hydroxy group or methoxy group, and Ar represents an optionally substituted aromatic or heteroaromatic group, or one of its mineral or organic acid salts whether pharmaceutically acceptable or not.

**2.** Compound according to claim 1, of formula (i), wherein the mineral or organic acids are pharmaceutically acceptable.

**3.** Compound according to claim 2, of formula (i), wherein Ar represents an optionally substituted mono-, di- or tri-cyclic aromatic or heteroaromatic group, of which a carbon atom of the aromatic carbocycle or of the aromatic heterocycle is directly linked to the oxygen of the aryloxypropanolaminotetralin.

**4.** Compound according to claim 3, of formula (i) wherien Ar is the etherifying residue in the 3-position of an N-substituted 1-amino-2,3- propandiol, having a beta-blocking adrenergic activity.

**5.** Compound according to claim 3 of formula (iA) or (iB)

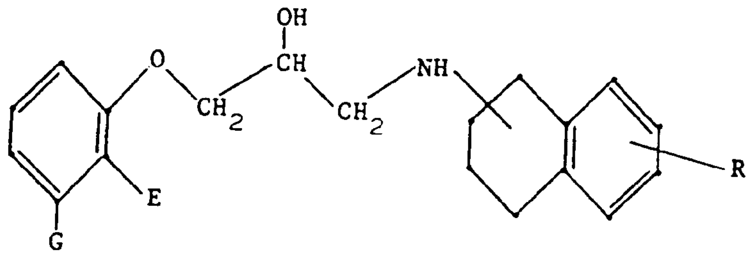

(iA)

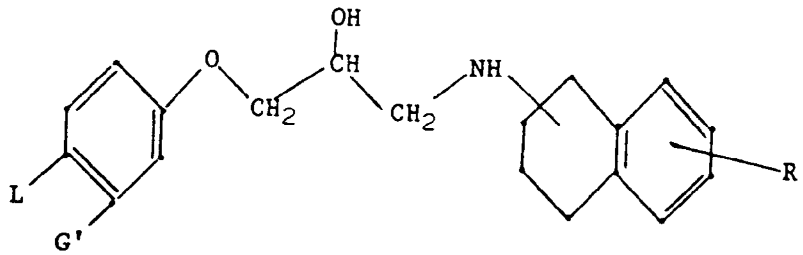

(iB)

wherein R is such as defined in claim 1, E represents hydrogen, a halogen, an alkyl, alkenyl, alkynyl, alkoxy, alkenyloxy, alkynyloxy, tetrahydrofurfuryloxy, phenoxy, cycloalkyl group of from 3 to 6 carbon atoms, 1-cyclohexenyl, 2-cyclohexenyl, 2,5-methano-cyclohexyl, alkylthio, alkanoyl, phenylalkanoyl, cyano, 3-chloroallyl, 2-indolyl, 2-benzoxazolyl, 2-benzothiazolyl, 2-quinolinyl, 2-quinoxalinyl, 2-thienyl, 4-thiazolyl, 4-morpholinyl; G is hydrogen, a halogen, an alkyl, an alkenyl, a trifluoromethyl group, a cyano group or an acetamido group, with at least one of the substituents E and G being hydrogen; or E and G represent, respectively, an acetamido group and a nitro group, or E and G taken together form a group $-CH_2-CH_2-CH_2-$, $-CH_2-CH_2-CH_2-CH_2-$,-CH=CH-$CH_2$-, -$CH_2$-CH=CH-, -CH=CH-$CH_2$-$CH_2$-, -CH=CH-CH=CH-, -CO-$CH_2$-$CH_2$-$CH_2$-, -$CH_2$-$CH_2$-$CH_2$-CO- or -$CH_2$-CH(OH)-CH(OH)-$CH_2$-; G' is hydrogen; L is halogen, an alkyl group non-substituted or substituted with an alkoxy, alkoxycarbonylamino, aminocarbonyl or cycloalkylalkoxy group, an alkenyl group, non-substituted or substituted by a cyano group; an alkanoylamino or haloalkanoylamino group; a ureido N'-mono or di-substituted with alkyl or cycloalkyl groups; an alkoxy group optionally substituted on the alkyl by an alkoxy group; a cyano group; or a hydroxymethyl group etherified with an alkoxyalkyl group; or G' and L represent, respectively, a nitro group and an acetamido group, or G' and L, taken together for a group -CH=CH-CH=CH-; as well as its pharmaceutically acceptable mineral or organic acid salts.

6. Compound according to claim 5, of formula (iA) or (iB), wherein E is hydrogen, halogen, an alkyl, alkenyl, alkynyl, alkoxy, alkenyloxy, alkynyloxy and G is hydrogen, or E and G, taken together, form a group $-CH_2-CH_2-CH_2-$; $-CH_2-CH_2-CH_2-CH_2-$; -CH=CH-$CH_2$-; -$CH_2$-CH=CH-, -CH=CH-$CH_2$-$CH_2$-, -CH=CH-CH=CH-, -CO-$CH_2$-$CH_2$-$CH_2$-; -$CH_2$-$CH_2$-$CH_2$-CO- or -$CH_2$-CH(OH)-CH(OH)-$CH_2$-; G' represents hydrogen, L represents an atom of chloride, a cyano group or a 2-methoxyethyl group or G' and L, taken together form a group -CH=CH-CH=CH-.

7. Compound according to claim 6, of formula (iA), wherein E is halogen, an alkyl, alkenyl, alkoxy, alkenyloxy or alkynyloxy group, or E and G, taken together, form a group -CH=CH-CH=CH- or -$CH_2$-CH(OH)-CH(OH)-$CH_2$-.

8. Compound according to claim 7 which is N(1,2,3,4-tetrahydronaphth-1-yl)-2-hydroxy-3-(1-naphtyloxy)-propanamine in optically active or inactive form, or one of its pharmaceutically acceptable mineral or organic acid salts.

9. Compound according to claim 8 which is N-[(R)-1,2,3,4-tetrahydronaphth-1-yl)]-(S)-2-hydroxy-3-(1-naphthyloxy)propanamine or one of its pharmaceutically acceptable mineral or organic acid salts.

10. Compound according to claim 7 which is N-(7-hydroxy-1,2,3,4-tetrahydronaphth-2-yl)-2-hydroxy-[3-(2-allylphenoxy)]propanamine in optically active or inactive form or one of its pharmaceutically acceptable mineral or organic acid salts.

**11.** Compound according to claim 10 which is N-(7-hydroxy-1,2,3,4-tetrahydronaphth-2-yl)-2-hydroxy-[3-(2-allylphenoxy)]propanamine hydrochloride, in optically active or inactive form.

**12.** Compound according to claim 2, of formula (iF)

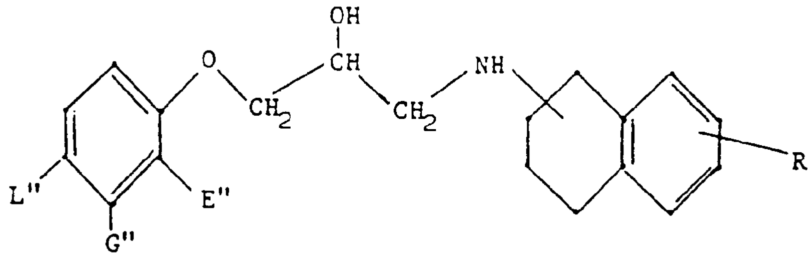

(iF)

wherein R is as defined in claim 1, L" is hydrogen and E" and G", taken together form a -CH=CH-NH- group, as well as its pharmaceutically acceptable mineral or organic acid salts.

**13.** Process for preparing a compound according to claim 1, characterized in that it comprises treating a 1-aryloxypropane of formula (ii)

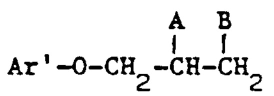

(ii)

wherein Ar' has the same meaning as that given in claims 1 to Ar, although it may contain N-protecting groups fixed on $NH_2$ or NH groups, A is a hydroxy group and B is a halogen selected from chlorine, bromine or iodine, or A and B, taken together form an epoxy group, with an aminotetralin of formula (iii)

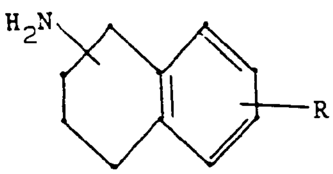

(iii)

wherein R is as defined in claim 1, optionally in the presence of a tertiary amine; optionally deprotecting the obtained product by removing the N-protecting group or groups; when the starting compound of formula (ii) in which Ar' is a 2-acetamido-3-nitrophenyl or 4-acetamido-3-nitrophenyl group, optionally deacetylating the obtained product by heating it in hydrochloric acid and optionally hydrogenating the obtained product in 97% formic acid in the presence of 10% Pd/C; and optionally converting the obtained product in one of its salts.

**14.** Process for preparing pharmaceutical compositions, characterized in that it consists in mixing, as active ingredient, a compound of formula (i) according to any one of claims 2 to 12 with a pharmaceutically acceptable carrier.

**15.** Process according claim 14, for preparing pharmaceutical compositions in dosage unit form.

**16.** Process according to claim 14 or 15, for preparing pharmaceutical compositions, containing from 0.1 to 500 mg of active ingredient per dosage unit.

**17.** Reagent for differentiating between the beta-adrenergic receptors in biochemical assays, characterized in that it comprises a suitably labelled compound according to claim 1.